# EUROPEAN PATENT SPECIFICATION

(11) **EP 2 252 321 B1**
(45) Date of publication and mention of the grant of the patent: **29.04.2015**
(21) Application number: 09704660.1
(22) Date of filing: 28.01.2009
(51) Int. Cl.: A61K 38/18, A61K 31/70, A61P 9/10

(54) **COMPOSITION COMPRISING TWO OR MORE ISOFORMS OF HEPATOCYTE GROWTH FACTOR FOR USE IN TREATING INCOMPLETE REVASCULARISATION OF THE MYOCARDIUM AFTER CORONARY ARTERY BYPASS GRAFTING**
ZUSAMMENSETZUNG ENTHALTEND ZWEI ODER MEHR ISOFORMEN VON HEPATOZYTEN-WACHSTUMSFAKTOR ZUR BEHANDLUNG VON UNVOLLSTÄNDIGER NEOVASKULARISATION DES HERZMUSKELS NACH EINER KORONARARTERIEN-BYPASSOPERATION
COMPOSITION COMPRENANT DE DEUX OU PLUSIEURS ISOFORMES DU FACTEUR DE CROISSANCE DES HÉPATOCYTES POUR LE TRAITEMENT DE RÉVASCULARISATION INCOMPLET DU MYOCARDE APRÈS LE PONTAGE AORTO-CORONARIEN

(30) Priority: 25.01.2008 US 23756 P
(43) Date of publication of application: 24.11.2010
(73) Proprietor: Viromed Co., Ltd., Seoul 151-747 (KR)
(72) Inventor: KIM, Jong-Mook, Seoul 138-752 (KR); KIM, Sujeong, Seoul 151-782 (KR); HAHN, Woong, Goyang-si Gyeonggi-do 410-720 (KR)
(74) Representative: Rowe, Daniel
(86) International application number: PCT/KR2009/000406
(87) International publication number: WO 2009/093880

(56) References cited:
- JP-A- 11 246 433
- US-A1- 2002 172 663
- US-A1- 2005 079 581
- US-A1- 2007 059 288
- HAHN ET AL: "Development of Novel Angiogenic DNA Medicine Using a Genomic/cDNA Hybrid of Hepatocyte Growth Factor Gene", MOLECULAR THERAPY, ACADEMIC PRESS, SAN DIEGO, CA, US, vol. 13, 1 January 2006 (2006-01-01), page S337, XP005675982, ISSN: 1525-0016
- CHO K R ET AL: "Therapeutic angiogenesis using naked DNA expressing two isoforms of the hepatocyte growth factor in a porcine acute myocardial infarction model", EUROPEAN JOURNAL OF CARDIO-THORACIC SURGERY, SPRINGER VERLAG, BERLIN, DE, vol. 34, no. 4, 1 October 2008 (2008-10-01), pages 857-863, XP025505468, ISSN: 1010-7940, DOI: 10.1016/J.EJCTS.2008.05.045 [retrieved on 2008-07-11]
- M. SAEED ET AL: "MR Assessment of Myocardial Perfusion, Viability, and Function after Intramyocardial Transfer of VM202, a New Plasmid Human Hepatocyte Growth Factor in Ischemic Swine Myocardium", RADIOLOGY, vol. 249, no. 1, 1 October 2008 (2008-10-01), pages 107-118, XP55033528, ISSN: 0033-8419, DOI: 10.1148/radiol.2483071579
- CARLSSON MARCUS ET AL: "Quantitative MR measurements of regional and global left ventricular function and strain after intramyocardial transfer of VM202 into infarcted swine myocardium", AMERICAN JOURNAL OF PHYSIOLOGY - HEART AND CIRCULATORY PHYSIOLOGY, vol. 295, no. 2, August 2008 (2008-08), pages H522-H532, XP002680566, ISSN: 0363-6135
- WEI WANG ET AL: "Induction of collateral artery growth and improvement of post-infarct heart function by hepatocyte growth factor gene transfer1", ACTA PHARMACOLOGICA SINICA, vol. 27, no. 5, 1 May 2006 (2006-05-01), pages 555-560, XP55033382, ISSN: 1671-4083, DOI: 10.1111/j.1745-7254.2006.00306.x
- V. JAYASANKAR ET AL: "Gene Transfer of Hepatocyte Growth Factor Attenuates Postinfarction Heart Failure", CIRCULATION, vol. 108, no. 90101, 9 September 2003 (2003-09-09), pages 230II--236, XP55033383, ISSN: 0009-7322, DOI: 10.1161/01.cir.0000087444.53354.66
- SAKAGUCHI G ET AL: "Control-Released Hepatocyte Growth Factor Prevents the Progression of Heart Failure in Stroke-Prone Spontaneously Hypertensive Rats", THE ANNALS OF THORACIC SURGERY, ELSEVIER, UNITED STATES, vol. 79, no. 5, 1 May 2005 (2005-05-01), pages 1627-1634, XP027732392, ISSN: 0003-4975 [retrieved on 2005-05-01]
- MORISHITA P ET AL: "THERAPEUTIC ANGIOGENESIS USING HEPATOCYTE GROWTH FACTOR (HGF)", CURRENT GENE THERAPY, BENTHAM SCIENCE PUBLISHERS LTD, NL, vol. 4, no. 2, 1 June 2004 (2004-06-01), pages 199-206, XP009047457, ISSN: 1566-5232
- NAKAGAMI H ET AL: "Hepatocyte growth factor as potential cardiovascular therapy", EXPERT REVIEW OF CARDIOVASCULAR THERAPY, FUTURE DRUGS, LONDON, GB, vol. 3, no. 3, 1 January 2005 (2005-01-01) , pages 513-519, XP009150848, ISSN: 1477-9072

## Description

### FIELD OF THE INVENTION

The present disclosure relates to methods for treating or preventing cardiac conditions in a subject comprising administering to the subject two or more isoforms of hepatocyte growth factor (HGF). The present disclosure further relates to methods for promoting endothelial cell growth in a blood vessel comprising administering to the blood vessel two or more isoforms of hepatocyte growth factor (HGF). In one embodiment, the two or more isoforms of HGF are administered as one or more polynucleotides encoding the isoforms.

### BACKGROUND OF THE INVENTION

HGF is a heparin binding glycoprotein also known as scatter factor or hepatopoietin-A. Originally identified as a potent hepatotrophic growth factor (Nakamura et al., Nature 342:440 (1989)), HGF is a mesenchymally derived heparin binding protein that has multiple biological effects such as mitogenesis, motogenesis and morphogenesis of various types of cells. A gene encoding HGF is located at chromosome 7q21.1 and comprises 18 exons and 17 introns, having the nucleotide sequence of SEQ ID NO: 1 (Seki T., et al., Gene 102:213-219 (1991)). A transcript of about 6 kb is transcribed from the HGF gene, and then a full length polypeptide HGF precursor (flHGF) consisting of 728 amino acids is synthesized therefrom, comprising the following domains: N-terminal hairpin loop-kringle1-kringle2-kringle3-kringle4-inactivated serine protease domain. Simultaneously, several other HGF polypeptide isoforms are synthesized by alternative splicing of the HGF gene. Known isoforms include deleted variant HGF (full length HGF except for a five residue deletion in kringle1), NK1 (N-terminal hairpin loop-kringle1), NK2 (N-terminal hairpin loop-kringle1-kringle2), and NK4 (N-terminal hairpin loop-kringle1-kringle2-kringle3-kringle4). In addition, allelic variants of each isoform exist. The biologically inactive precursors may be converted into active forms of disulfide-linked heterodimer by protease in serum. In the heterodimers, the alpha chain having a high molecular weight forms four kringle domains and an N-terminal hairpin loop like a preactivated peptide region of plasminogen. The kringle domains of a triple disulfide-bonded loop structure consisting of about 80 amino acids may play an important role in protein-protein interaction. The low molecular weight beta chain forms an inactive serine protease-like domain. dHGF consisting of 723 amino acids is a polypeptide with deletion of five amino acids in the 1 st kringle domain of the alpha chain, i. e., F, L, P, S and S, due to alternative splicing between exon 4 and exon 5.

*In vivo,* two isoforms of HGF (flHGF having 728 amino acids and dHGF having 723 amino acids) are generated via alternative splicing between exon 4 and exon 5. Although both of flHGF and dHGF share several biological functions, they are different in terms of immunological and several biological properties.

HGF has been shown to stimulate angiogenesis by regulating the growth of endothelial cells and migration of vascular smooth muscle cells. Because of its angiogenic activity, HGF is regarded as one of the promising candidates in therapeutic angiogenesis. "Therapeutic angiogenesis" means an intervention that utilizes angiogenic factors, either as recombinant proteins or as genes, for the treatment of ischemic diseases, such as coronary artery disease (CAD) or peripheral artery disease (PAD). HGF is also known to stimulate not only the growth but also the migration of endothelial cells (Bussolino et al., J Cell Biol. 119:629 (1992); Nakamura et al., J Hypertens 14:1067 (1996)), and has been tested for its role as a re-endothelialization stimulating agent (Yasuda et al., Circulation 101:2546 (2000); Hayashi et al., Gene Ther 7:1664 (2000)).

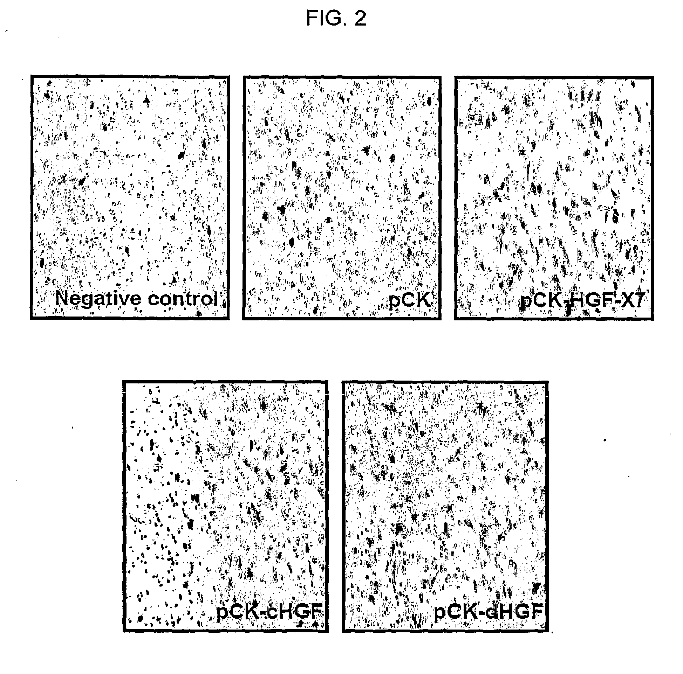

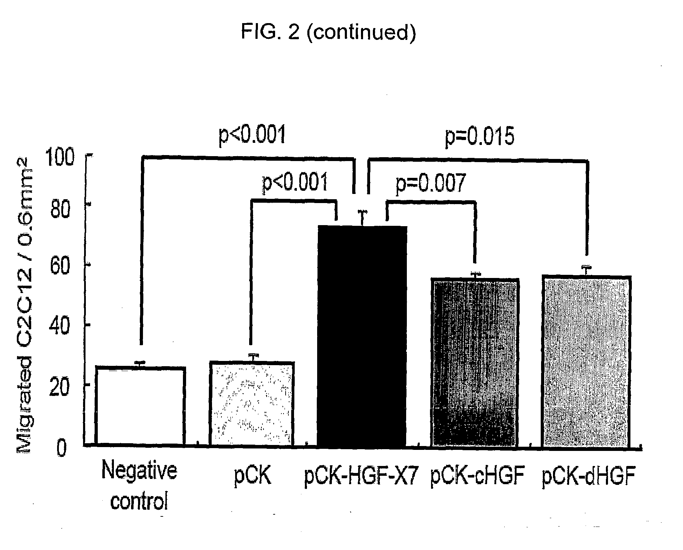

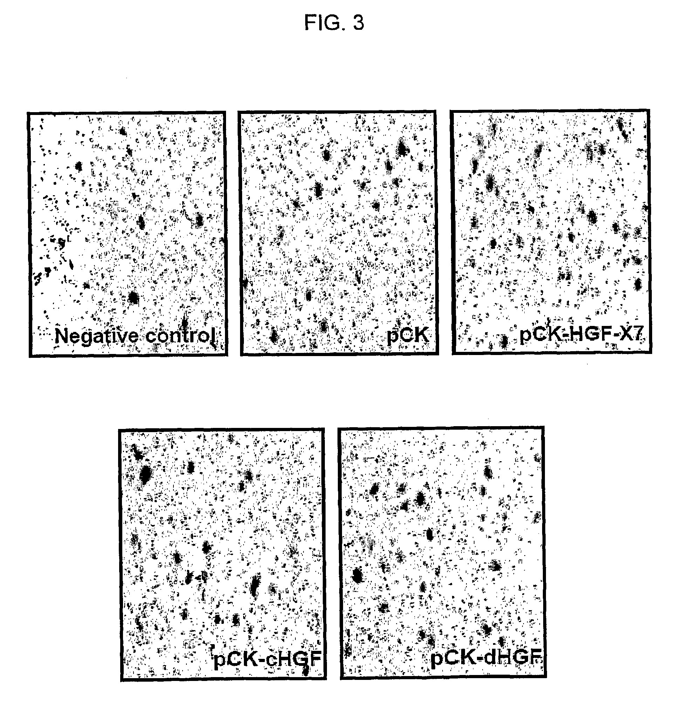

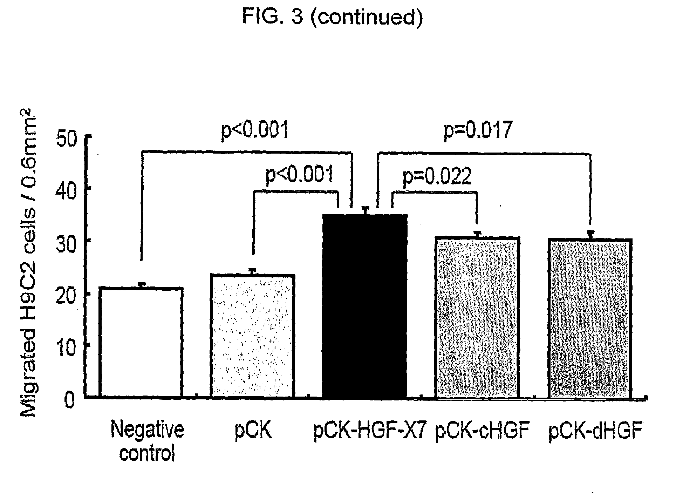

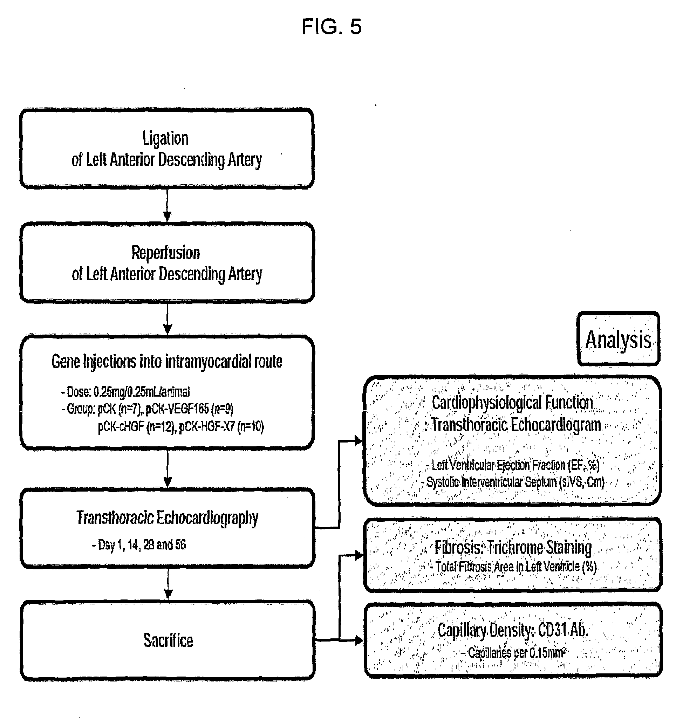

HGF has been used as an agent for therapeutic angiogenesis. Morishita and colleagues have used the HGF gene for treatment of PAD and CAD. They observed some therapeutic response for PAD after administering the HGF gene, but it was unclear whether HGF gene transfer was effective for treating CAD. To date, HGF gene transfer has been tested in various animal models for CAD (Miyagawa et al., Circulation 105:2556 (2002); Azuma et al., Gene Ther. 13:1206 (2006); Aoki et al., Gene Ther. 7:417 (2000); Funatsu et al., J. Thoracic Cardiovasc. Surg. 124:1099 (2002)). However, it is still controversial whether HGF gene transfer has beneficial effects on CAD. For example, Miyagawa and his colleagues showed that human HGF transfer could not increase the left ventricle ejection fraction (LVEF) of the infarcted heart 8 weeks after treatment in a rat myocardial infarction model (Miyagawa et al., Circulation 105:2556 (2002), Figure 2). Moreover HGF gene transfer had little effect on the percent fractional shortening and LV anterior wall thickness 8 weeks after treatment in the same model (Miyagawa et al., Circulation 105:2556 (2002), Figures 3 and 5).

HGF has also been used as an agent to inhibit restenosis. Coronary angioplasty procedures, e.g., balloon or stent, are widely used methods for the treatment of obscured blood vessels. However, intimal thickening, *e.g,,* coronary artery restenosis poses a significant problem when using angioplasty. One of the causes of restenosis is the hyper-proliferation and migration of vascular smooth muscle cells with accompanying synthesis of extracellular matrix, resulting from a response to vessel injury. There is evidence that rapid endothelial resurfacing could suppress smooth muscle cell proliferation, and thereby inhibit restenosis *(e.g.,* Bauters et al., Prog Cardiovasc Dis. 40:107 (1997)). As one method for the prevention of restenosis, local delivery of endothelial growth factors such as vascular endothelial growth factor (VEGF) or hepatocyte growth factor (HGF) to the injured blood vessel was attempted and showed effects on the attenuation of restenosis *(*Asahara et al., Circulation 94:3291 (1996); Yasuda et al., Circulation 101:2546 (2000); Hayashi et al., Gene Ther 7:1664 (2000); Walter et al., Circulation 110:36 (2004)).

All of the studies on HGF gene therapy described above have been done with flHGF cDNA encoding 728 amino acids, but not with dHGF cDNA encoding 723 amino acids (Miyagawa *et al.*; *Azuma et al.*; Aoki *et al.*; Funatsu *et al.;* Yasuda *et al.*; and Hayashi *et al*.).

Hahn, W., et al., Molecular Therapy, 2006, Vol 13 (Supp.l), P876 describes experiments to assess the therapeutic potential of the HGF hybrid HGF-X7, in a rabbit ischemic limb disease model and a rat ischemic heart disease model.

US 2005/0079581 discloses a hybrid HGF gene and its use for treating or preventing ischemic or liver diseases.

Jayasankar, V., et al., Circulation, 2003, Vol 108 (Supp. II), II-230 - II-236 discloses that the gene transfer of HGF to the myocardium of rats attenuates post-infarction heart failure following ligation of the left anterior descending coronary artery.

Sakaguchi, G., et al., Annals of Thoracic Surgery, 2005, Vol 79, 1627-1634 discloses that control-released HGF prevents the progression of heart failure in stroke-prone spontaneously hypertensive rats.

The present invention provides the first demonstration that the transfer of nucleotide sequences expressing multiple isoforms of HGF *(e.g.,* flHGF and dHGF) can effectively treat CAD in animals and humans, as compared with cDNA for flHGF which was tested in most of the previous reports. The present invention also provides the first demonstration that the transfer of nucleotide sequences expressing multiple isoforms of HGF can accelerate the re-endothelialization process of a blood vessel.

### SUMMARY OF THE INVENTION

In a first aspect the invention provides a composition for use in treating incomplete revascularisation of the myocardium in a subject who has had a coronary artery bypass graft but there has been incomplete revascularisation of the myocardium, the composition comprising two or more isoforms of HGF or one or more polynucleotides encoding the isoforms as active ingredients, wherein said two or more isoforms of HGF are selected from the group consisting of full length HGF (flHGF), deleted variant HGF (dHGF), NK1, NK2 and NK4.

In a second aspect the invention provides the use of a composition comprising two or more isoforms of HGF or one or more polynucleotides encoding the isoforms for the manufacture of a medicament for treating incomplete revascularisation of the myocardium in a subject who has had a coronary artery bypass graft, but there has been incomplete revascularisation of the myocardium, wherein said two or more isoforms are selected from the group consisting of full length HGF (flHGF), deleted variant HGF (dHGF), NK1, NK2 and NK4.

Further disclosed herein is the use of a composition comprising two or more isoforms of HGF or one or more polynucleotides encoding the isoforms for the manufacture of a medicament for treating or preventing a cardiac condition in a subject.

Further disclosed herein is the use of a composition comprising two or more isoforms of HGF or one or more polynucleotides encoding the isoforms for the manufacture of a medicament for promoting endothelial cell growth in a blood vessel.

Further disclosed herein are methods of treating or preventing a cardiac condition by administering two or more isoforms of HGF.

Further disclosed herein are methods of increasing the perfusion of ischemic cardiac tissue or increasing vascular density in the myocardium in a subject, comprising administering to the subject a composition comprising two or more isoforms of HGF.

Further disclosed herein are methods of treating a cardiac condition in a subject, comprising administering to the subject a composition comprising two or more isoforms of HGF.

Further disclosed herein are methods of enhancing endothelial repair or providing treatment at the site of vascular injury or a diseased vessel in a subject comprising administering to the subject a composition comprising two or more isoforms of HGF.

Further disclosed herein are methods for promoting endothelial cell growth in a blood vessel, comprising administering to the blood vessel a composition comprising two or more isoforms of HGF. In one instance, the blood vessel is injured. In a further instance, re-endothelialization of the blood vessel is promoted.

In one embodiment, the two or more isoforms of HGF include full length HGF (referred to herein as flHGF) and deleted variant HGF (referred to herein as dHGF). In another embodiment, the two or more isoforms of HGF also include NK1.

In a further embodiment, the two or more isoforms of HGF are administered in the form of polynucleotides encoding the isoforms.

In one embodiment of the invention, the composition is administered by injection.

In another embodiment of the invention the composition is administered by using a delivery device. In one embodiment, the delivery device is a stent. In a further embodiment, the stent is selected from the group consisting of a non-polymer-based stainless steel stent, a polymer-based stainless steel stent, a non-polymer-based cobalt chromium stent, and a polymer-based cobalt chromium stent.

In one embodiment of the invention, the two or more isoforms of HGF are administered directly to ischemic cardiac tissue in a subject.

Further disclosed herein are compositions comprising two or more isoforms of HGF.

In one instance, the compositions comprise polynucleotides encoding the two or more isoforms of HGF.

Further disclosed herein is a composition for increasing the perfusion of ischemic cardiac tissue in a subject, comprising two or more isoforms of HGF.

Further disclosed herein is a composition for promoting endothelial cell growth in a blood vessel in a subject, comprising two or more isoforms of HGF.

In one embodiment, administration of the composition to the blood vessel of the subject promotes endothelialization of the blood vessel. In another embodiment, administration of the composition to the blood vessel of a subject promotes and/or accelerates re-endothelialization of the blood vessel.

In a further embodiment, the subject is in need of treatment of restenosis.

### BRIEF DESCRIPTION OF DRAWINGS

The above and other objects and features of the present invention will become apparent from the following description of the invention, when taken in conjunction with the accompanying drawings.
Figure 1 shows the effects of HGF isoforms on HUVEC migration.
Figure 2 shows the effects of HGF isoforms on C2C12 cell migration.
Figure 3 shows the effects of HGF isoforms on H9C2 cell migration.
Figure 4 shows the effects of HGF isoforms on HUVEC proliferation.
Figure 5 shows a schematic diagram of the experimental procedure to assess the pharmacological efficacy of HGF in a rat ischemic heart disease model.
Figure 6 shows the effect of HGF on the function of the left ventricular ejection fraction.
Figure 7 shows the effect of HGF on the function of the systolic-interventricular septum.
Figure 8 shows the effect of HGF injection into the ischemic myocardium on capillary density.
Figure 9 shows the effect of HGF injection into the ischemic myocardium on myocardial fibrosis.
Figure 10 shows coronary artery territory on the 20 segment model of MIBI-SPECT.
Figure 11 shows selection of the myocardial territory for the pCK-HGF-X7 injection. pCK-HGF-X7 is administered by intramyocardial injections into both sides of the coronary artery within the myocardial territory having a perfusion decrease by the assessment of MIBI-SPECT.
Figure 12 shows the effect of pCK-HGF-X7 on myocardial perfusion under MIBI-SPECT.
Figure 13 shows myocardial perfusion (κ) assessed by myocardial contrast stress echocardiogram.
Figure 14 shows the acceleration of re-endothelialization by HGF plasmid eluting stent on OCT.
Figure 15 shows the acceleration of re-endothelialization by HGF plasmid eluting stent on SEM.

### DETAILED DESCRIPTION OF THE INVENTION

In a first aspect the invention provides a composition for use in treating incomplete revascularisation of the myocardium in a subject who has had a coronary artery bypass graft but there has been incomplete revascularisation of the myocardium, the composition comprising two or more isoforms of HGF or one or more polynucleotides encoding the isoforms as active ingredients, wherein said two or more isoforms of HGF are selected from the group consisting of full length HGF (flHGF), deleted variant HGF (dHGF), NK1, NK2 and NK4.

In a second aspect the invention provides the use of a composition comprising two or more isoforms of HGF or one or more polynucleotides encoding the isoforms for the manufacture of a medicament for treating incomplete revascularisation of the myocardium in a subject who has had a coronary artery bypass graft, but there has been incomplete revascularisation of the myocardium, wherein said two or more isoforms are selected from the group consisting of full length HGF (flHGF), deleted variant HGF (dHGF), NK1, NK2 and NK4.

In one embodiment, the composition of the invention described herein comprises three or more isoforms of HGF, e.g., four or more isoforms of HGF. In another embodiment, the composition comprises two or more isoforms of HGF selected from the group consisting of flHGF, dHGF, NK1, and NK2. In yet another embodiment, the composition comprises two or more isoforms of HGF selected from the group consisting of flHGF, dHGF, and NK1. In a further embodiment, the two or more isoforms of HGF comprise flHGF and dHGF. In a further embodiment, the two or more isoforms of HGF consist of flHGF and dHGF. In another embodiment, the two or more isoforms of HGF comprise flHGF, dHGF, and NK1. In another embodiment, the two or more isoforms of HGF are administered in the form of polynucleotides encoding the isoforms.

In another embodiment, administration of two or more isoforms of HGF, *e.g.,* to a subject in need of treatment of restenosis, suppresses smooth muscle cell proliferation in the blood vessel. Further disclosed herein is the discovery that administration of two or more isoforms of HGF to a subject suffering from a cardiac condition such as CAD is effective to increase perfusion of ischemic cardiac tissue and therefore treat or prevent a cardiac condition. Further disclosed herein is the discovery that administration of 2 isoforms of HGF promotes endothelialization of a blood vessel, *e.g.,* for attenuating restenosis through rapid re-endothelialization of a blood vessel. Accordingly, further disclosed herein are methods of treating or preventing a cardiac condition, *e.g.*, CAD or coronary artery restenosis, by administering two or more isoforms of HGF. Further disclosed herein are methods for promoting endothelial cell growth in a blood vessel, e.g., where the blood vessel is injured.

Further disclosed herein is the use of a composition comprising two or more isoforms of HGF or one or more polynucleotides encoding the isoforms for the manufacture of a medicament for treating or preventing a cardiac condition in a subject.

Further disclosed herein is the a use of a composition comprising two or more isoforms of HGF or one or more polynucleotides encoding the isoforms for the manufacture of a medicament for promoting endothelial cell growth in a blood vessel.

Further disclosed herein are methods of increasing the perfusion of ischemic cardiac tissue or increasing vascular density in the myocardium in a subject, comprising administering to the subject a composition comprising two or more isoforms of HGF.

Further disclosed herein are methods of treating or preventing a cardiac condition in a subject, comprising administering to the subject a composition comprising two or more isoforms of HGF.

Further disclosed herein are methods of enhancing endothelial repair or providing treatment at the site of vascular injury or a diseased vessel in a subject comprising administering to the subject a composition comprising two or more isoforms of HGF.

Further disclosed herein is a method of promoting endothelial cell growth in a blood vessel, comprising administering to the blood vessel a composition comprising two or more isoforms of HGF, wherein endothelial cell growth in the blood vessel is promoted.

In these instances, the cardiac condition to be treated or prevented is any condition related to decreased blood flow in the heart, aorta, or coronary arteries or ischemic tissue in the heart. Examples of cardiac conditions include, without limitation, coronary artery occlusion (e.g., resulting from or associated with lipid/cholesterol deposition, macrophage/inflammatory cell recruitment, plaque rupture, thrombosis, platelet deposition, or neointimal proliferation); ischemic syndromes (*e.g.,* resulting from or associated with myocardial infarction, stable angina, unstable angina, coronary artery restenosis or reperfusion injury); cardiomyopathy (*e.g.,* resulting from or associated with an ischemic syndrome, a cardiotoxin, an infection, hypertension, a metabolic disease (such as uremia, beriberi, or glycogen storage disease), radiation, a neuromuscular disease, an infiltrative disease (such as sarcoidosis, hemochromatosis, amyloidosis, Fabry's disease, or Hurler's syndrome), trauma, or an idiopathic cause); arrhythmia or dysrrhythmia (*e.g.,* resulting from or associated with an ischemic syndrome, a cardiotoxin, adriamycin, an infection, hypertension, a metabolic disease, radiation, a neuromuscular disease, an infiltrative disease, trauma, or an idiopathic cause); infection (*e.g,* caused by a pathogenic agent such as a bacterium, a virus, a fungus, or a parasite); and an inflammatory condition (*e.g.,* associated with myocarditis, pericarditis, endocarditis, immune cardiac rejection, or an inflammatory conditions resulting from one of idiopathic, autoimmune, or a connective tissue disease).

In these instances the methods may be used to treat or prevent atherosclerosis (*e.g.,* in the aorta or coronary arteries), to prevent complications associated with atherosclerosis (*e.g.,* angina pectoris, myocardial infarction, arrhythmias, heart failure, kidney failure, liver cirrhosis, Legg-Calve-Perthes disease, ischemic stroke, peripheral artery occlusion, aneurysm, embolism), or to reduce the early symptoms and signs of atherosclerosis (*e.g.,* the inability of blood flow to the affected tissue to increase with demand, as in angina, exertion, or intermittent claudication).

In these instances, the methods may be used to treat or prevent cardiac conditions resulting from vascular surgical intervention including, without limitation, angioplasty (*e.g.,* percutaneous transluminal coronary angioplasty, carotid percutaneous transluminal angioplasty, coronary angioplasty with stent implantation), stenting, atherectomy, or grafting (*e.g.,* coronary bypass grafting). In certain instances, the methods may be carried out before, during and/or after the surgical intervention. In certain instances, the cardiac condition is coronary artery restenosis.

In these instances, the two or more isoforms of HGF are administered to a subject having coronary artery disease. In certain instances, the subject has partial or complete blockage of one or more coronary arteries. In certain instances, the subject has had, is having, or is at risk for myocardial infarction. In a further instance, the subject has been determined to have or is suspected to have ischemic heart tissue, *e.g.,* based on an angiogram, electrocardiogram, echocardiogram, or other procedure. In certain instances, the subject is a candidate for a coronary artery bypass graft (CABG). In certain instances, the subject has one or more coronary arteries that are partially or completely blocked but are not suitable for CABG. In certain instances, the subject has had a CABG but there has been incomplete revascularization of the myocardium.

In these instances, the two or more isoforms of HGF are administered to a blood vessel to promote endothelial cell growth. In certain instances, the blood vessel is obscured or injured. In certain instances, an obscured blood vessel may include an artery or vein where the lumen of the blood vessel has been narrowed and blood flow through the vessel is decreased. In certain instances, the administration of two or more isoforms of HGF promotes re-endothelialization of the blood vessel, *e.g.* following injury to the blood vessel wall, *e.g.,* during an angioplasty procedure. In certain instances, re-endothelialization may be promoted and/or accelerated, *e.g.,* an increased rate of endothelial cell growth compared to endothelial cell growth not in the presence of two or more isoforms of HGF.

The term "treat," "treating," or "treatment" of a condition, *e.g.,* a cardiac condition or an obscured or injured blood vessel, as used herein, refers to the administration to a subject of a factor in an amount sufficient to result in amelioration of one or more symptoms of the condition, or prevent advancement of the condition, or cause regression of the condition, *e.g.,* due to the promotion of angiogenesis or endothelial cell growth. For example, with respect to amelioration of a symptom of a cardiac condition, treatment results in a measurable decrease in the symptom of at least 5%, preferably at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100%. Physiological effects related to the treatment of a cardiac condition that can be detected and measured to determine treatment include, without limitation, an increase in cardiac efficiency (as measured by at least one clinical index of cardiac function such as cardiac output, pulmonary artery and central venous pressures, or ventricular ejection fraction), transmural blood flow in the myocardium at rest or under stress conditions, regeneration of myocardial tissue, formation, maturation, and/or growth of collateral blood vessels (*e.g.,* local neoangiogenesis, increase in capillary density, arteriogenesis, lymphangiogenesis, vasculogenesis), cardiomyogenesis (*e.g.,* striated, smooth or myoepithelial cells), vascularization of myocardial tissue, contractile function of the heart, left ventricular ejection fraction, or inter ventricular septum; or a decrease in myocardial fibrosis, intimal thickening (neointimal proliferation/hyperplasia), endothelial or smooth muscle cell proliferation, chest pain, or shortness of breath.

The terms "prevent," "preventing," and "prevention," as used herein, refer to a decrease in the occurrence of one or more symptoms of a condition (*e.g.,* altered cardiac function or decreased blood flow due to an obscured or injured blood vessel) in an animal. The prevention may be complete, *e.g.,* the total absence of symptoms in a subject. The prevention may also be partial, such that the occurrence of symptoms in a subject is less than that which would have occurred without the present invention.

In one embodiment, the HGF isoforms or polynucleotides encoding the HGF isoforms are administered to the vasculature or heart of a subject, *e.g.,* an injured blood vessel, a partially or totally blocked coronary artery, ischemic myocardial tissue, pericardial space, or coronary sinus.

The HGF isoforms or polynucleotides encoding the HGF isoforms may be delivered to the desired site using any means known in the art. Examples of delivery devices that may be used include, without limitation, catheters (*e.g.,* balloon catheter, infusion catheter, stiletto catheter), needles, needle-free injectors, stents, infusion cannula, mesh, cardiac harness, shunts, cardiac pacemakers, implantable defibrillators, sutures, staples, perivascular wraps, pliable sheets or membranes which can substantially conform to the contours of a wound site, channeling devices, grafts, and pumps. Specific examples of methods of delivery of the HGF isoforms include, without limitation, delivery through a balloon catheter placed in a vein draining into the coronary sinus (*e.g.,* the great cardiac vein, middle cardiac vein, posterior vein of the left ventricle, or anterior interventricular vein or any of their side branches); delivery through a catheter conducted into the lumen of one or more coronary arteries *(e.g.,* the right or left coronary artery) wherein the HGF isoforms are coated on a balloon that is inflated at the site or injected from the tip of the catheter; delivery via a needle during open heart surgery (*e.g.,* into the left or right atrium or left or right ventricle); delivery into the pericardial space using internal entry through the left atrium, right ventricle or left ventricle or using external entry through an open chest procedure, minimally invasive surgery, or percutaneous entry accomplished by injection, catheterization, creation of laser-created perfusion channels, cannulization, use of a particle gun, or use of a pump; delivery by anterograde perfusion from a catheter placed into a conduit delivering blood to the tissue or retrograde perfusion from a catheter placed into a conduit receiving blood from the tissue; or delivery via an intraluminal device or intravascular prosthesis for maintaining vascular patency (*e.g.,* stent, graft, stent-graft, vena cava filter). In one embodiment, the device is biodegradable so that it does not need to be removed after it is no longer needed. In certain embodiments, the 2 HGF isoforms are delivered using a stent. In a further embodiment, the stent is selected from the group consisting of a non-polymer-based stainless steel stent, a polymer-based stainless steel stent, a non-polymer-based cobalt chromium stent, and a polymer-based cobalt chromium stent.

In one embodiment, polynucleotides encoding the HGF isoforms are delivered in the form of cells comprising the polynucleotides and expressing HGF polypeptides. The cells may be autologous or non-autologous (*e.g.,* allogeneic or xenogeneic) cells. Any cell that is viable after transplantation may be used, including, for example, fibroblasts, cardiomyocytes, endothelial cells, or stem cells, (*e.g.,* embryonic stem cells, hematopoietic stem cells, mesenchymal stem cells). Cells comprising the polynucleotides may be introduced as an injectable liquid suspension preparation, *e.g.,* for injection into the site of the damaged myocardium or intravenously. Cells may be introduced into an infarct zone to reduce the degree of scar formation and to augment ventricular function. When the polynucleotides are to be introduced into cells *ex vivo,* the cells may be obtained from a subject by any technique known in the art, including, but not limited to, biopsies, scrapings, and surgical tissue removal. The isolated cells may be cultured for a sufficient amount of time to allow the polynucleotides to be introduced into the cells, *e.g.,* 2, 4, 6, 8, 10, 12, 18, 24, 36, 48 hours or more. Methods for culturing primary cells for short periods of time are well known in the art. For example, cells may be cultured in plates (*e.g.,* in microwell plates) either attached or in suspension. In one embodiment, the presence of the polynucleotides in the cells is determined prior to introducing the cells back into the subject. In another embodiment, cells containing the polynucleotides are selected (*e.g.,* based on the presence of a selectable marker in the polynucleotides) and only those cells containing the polynucleotides are reintroduced into the subject.

When HGF isoforms or polynucleotides encoding the HGF isoforms are delivered by injection, the injection may be an intracardiac injection, e.g., intraatrial (left and/or right) or intraventricular (left and/or right). The injection may also be an intramyocardial injection. The injection site may be at or near the ischemic/hypoxic region, at the border of the normal tissue and the ischemic/hypoxic region, or in normal tissue. The injection site may be at the site of one or more coronary arteries, *e.g.,* an artery that is occluded. Injections may be transepicardial or transendocardial. Delivery may consist of one injection or multiple injections at one or more sites. Delivery may be made by intrapericardial injection. Delivery to a vascular site may be by intravascular injection, *e.g.,* intravenous or intraarterial (intracoronary, intraaortic). Delivery may be into at least two coronary arteries, *e.g.,* at least one left and one right coronary artery, *e.g.,* at a site at least about 1 cm into the lumen of a coronary artery. Vascular injection may be, for example, at a site adjacent to ischemic or diseased tissue, at the site of a vascular injury, or at the site of stenosis.

The administration of the HGF isoforms may be repeated more than once, *e.g.,* after an interval of 0.5, 1, 2, 3, 4, 5, 6, 7 days or more, *e.g.,* after 1, 2, 3, 4, 5, 6, 7, 8, 9, or 10 weeks or more. In one embodiment, the cardiac perfusion state or vascular health of a subject is monitored after each administration of the HGF isoforms, *e.g.,* by angiogram, electrocardiogram, echocardiogram, or other procedure, and additional administrations are provided as necessary.

In one embodiment of the invention, the two or more isoforms of HGF are administered to a subject currently undergoing an ischemic event. In another embodiment, the two or more isoforms of HGF are administered as soon as possible after an ischemic event has occurred, *e.g.,* within 0.5, 1, 2, 3, 4, 5, 6, 12, 18, 24, 36, 48, or 72 hours of the ischemic event.

The two or more isoforms of HGF are administered at a therapeutically effective dose, *e.g.,* a dose that leads to a measurable improvement in the cardiac and/or vessel condition of a subject, *e.g.,* an increase in perfusion of ischemic cardiac tissue, an increase in capillary density in the ischemic cardiac tissue, a decrease in fibrosis in the ischemic cardiac tissue, a decrease in the extent of vascular injury, an increase in endothelialization, *etc.* The effective dose will vary from subject to subject depending on the extent of the condition and/or need for endothelialization, the chosen route of administration, the age, sex and body weight of the individual subject, the health status of the subject, and the severity of the subject's symptoms, and can be administered in a single dose or in divided dose. Therefore, the daily dose should not be construed as a limitation to the scope of the invention in any way. For example, when the two or more isoforms of HGF are administered as proteins, a therapeutically effective dose may be in the range of about 1 µg to about 100 mg, *e.g.,* about 10 µg to about 10 mg of each protein. When the two or more isoforms of HGF are administered as polynucleotides, a therapeutically effective dose may be in the range of about 1 µg to about 10 mg, *e.g.,* about 5 µg to about 5 mg, *e.g.,* about 10 µg to about 2 mg, 100 µg to about 1 mg. When the administration of the HGF isoforms is repeated more than once, the dose administered may be the same or different each time.

In one embodiment, additional therapeutic agents or procedures (*e.g.,* angioplasty) that are known to be effective for the treatment of a cardiac and/or vessel condition are administered to the subject. Examples of therapeutic agents include, without limitation, angiogenesis promoters (*e.g.,* vascular endothelial growth factor, nitric oxide releasing or generating agents, fibroblast growth factor, platelet derived growth factor, interleukin-6, monocyte chemotactic protein-1, granulocyte-macrophage colony stimulating factor, transforming growth factor-β), anti-thrombotic agents (*e.g.,* aspirin, heparin, PPACK, enoxaprin, hirudin), anticoagulants, antibiotics, antiplatelet agents, thrombolytics (*e.g.*, tissue plasminogen activator), antiproliferatives, antiinflammatories, agents that inhibit hyperplasia, agents that inhibit restenosis, smooth muscle cell inhibitors, growth factors, growth factor inhibitors, cell adhesion inhibitors, chemotherapeutic agents, and combinations thereof.

The following definitions are provided and should be helpful in understanding the scope and practice of the present disclosure

The term "isolated" designates a biological material (cell, nucleic acid or protein) that has been removed from its original environment (the environment in which it is naturally present). For example, a polynucleotide present in the natural state in a plant or an animal is not isolated, however the same polynucleotide separated from the adjacent nucleic acids in which it is naturally present, is considered "isolated."

"Nucleic acid," "nucleic acid molecule," "oligonucleotide," and "polynucleotide" are used interchangeably and refer to the phosphate ester polymeric form of ribonucleosides (adenosine, guanosine, uridine or cytidine; "RNA molecules") or deoxyribonucleosides (deoxyadenosine, deoxyguanosine, deoxythymidine, or deoxycytidine; "DNA molecules"), or any phosphoester analogs thereof, such as phosphorothioates and thioesters, in either single stranded form, or a double-stranded helix. Double stranded DNA-DNA, DNA-RNA and RNA-RNA helices are possible. The term nucleic acid molecule, and in particular DNA or RNA molecule, refers only to the primary and secondary structure of the molecule, and does not limit it to any particular tertiary forms. Thus, this term includes double-stranded DNA found, *inter alia,* in linear or circular DNA molecules (*e.g.,* restriction fragments), plasmids, supercoiled DNA and chromosomes. In discussing the structure of particular double-stranded DNA molecules, sequences may be described herein according to the normal convention of giving only the sequence in the 5' to 3' direction along the non-transcribed strand of DNA *(i.e.,* the strand having a sequence homologous to the mRNA). A "recombinant DNA molecule" is a DNA molecule that has undergone a molecular biological manipulation. DNA includes, but is not limited to, cDNA, genomic DNA, plasmid DNA, synthetic DNA, and semi-synthetic DNA.

The term "fragment," as applied to polynucleotide sequences, refers to a nucleotide sequence of reduced length relative to the reference nucleic acid and comprising, over the common portion, a nucleotide sequence identical to the reference nucleic acid. Such a nucleic acid fragment according to the invention may be, where appropriate, included in a larger polynucleotide of which it is a constituent. Such fragments comprise, or alternatively consist of, oligonucleotides ranging in length from at least 6, 8, 9, 10, 12, 15, 18, 20, 21, 22, 23, 24, 25, 30, 39, 40, 42, 45, 48, 50, 51, 54, 57, 60, 63, 66, 70, 75, 78, 80, 90, 100, 105, 120, 135, 150, 200, 300, 500, 720, 900, 1000, 1500, 2000, 3000, 4000, 5000, or more consecutive nucleotides of a nucleic acid according to the invention.

A "gene" refers to a polynucleotide comprising nucleotides that encode a functional molecule, including functional molecules produced by transcription only (*e.g.*, a bioactive RNA species) or by transcription and translation *(e.g.,* a polypeptide). The term "gene" encompasses cDNA and genomic DNA nucleic acids. "Gene" also refers to a nucleic acid fragment that expresses a specific RNA, protein or polypeptide, including regulatory sequences preceding (5' non-coding sequences) and following (3' non-coding sequences) the coding sequence. "Native gene" refers to a gene as found in nature with its own regulatory sequences. "Chimeric gene" refers to any gene that is not a native gene, comprising regulatory and/or coding sequences that are not found together in nature. Accordingly, a chimeric gene may comprise regulatory sequences and coding sequences that are derived from different sources, or regulatory sequences and coding sequences derived from the same source, but arranged in a manner different than that found in nature. A chimeric gene may comprise coding sequences derived from different sources and/or regulatory sequences derived from different sources. "Endogenous gene" refers to a native gene in its natural location in the genome of an organism. A "foreign" gene or "heterologous" gene refers to a gene not normally found in the host organism, but that is introduced into the host organism by gene transfer. Foreign genes can comprise native genes inserted into a non-native organism, or chimeric genes. A "transgene" is a gene that has been introduced into the cell by a gene transfer procedure.

"Heterologous DNA" refers to DNA not naturally located in the cell, or in a chromosomal site of the cell. The heterologous DNA may include a gene foreign to the cell.

The term "genome" includes chromosomal as well as mitochondrial, chloroplast and viral DNA or RNA.

A nucleic acid molecule is "hybridizable" to another nucleic acid molecule, such as a cDNA, genomic DNA, or RNA, when a single stranded form of the nucleic acid molecule can anneal to the other nucleic acid molecule under the appropriate conditions of temperature and solution ionic strength. Hybridization and washing conditions are well known and exemplified in Sambrook et al. in Molecular Cloning: A Laboratory Manual, Second Edition, Cold Spring Harbor Laboratory Press, Cold Spring Harbor (1989), particularly Chapter 11 and Table 11.1 therein. The conditions of temperature and ionic strength determine the "stringency" of the hybridization.

Stringency conditions can be adjusted to screen for moderately similar fragments, such as homologous sequences from distantly related organisms, to highly similar fragments, such as genes that duplicate functional enzymes from closely related organisms. For preliminary screening for homologous nucleic acids, low stringency hybridization conditions, corresponding to a melting temperature (Tm) of 55°, can be used, *e.g.,* 5X SSC, 0.1% SDS, 0.25% milk, and no formamide; or 30% formamide, 5X SSC, 0.5% SDS. Moderate stringency hybridization conditions correspond to a higher Tm, *e.g.,* 40% formamide, with 5X or 6X SSC. High stringency hybridization conditions correspond to the highest Tm, *e.g.,* 50% formamide, 5X or 6X SSC.

Hybridization requires that the two nucleic acids contain complementary sequences, although depending on the stringency of the hybridization, mismatches between bases are possible. The term "complementary" is used to describe the relationship between nucleotide bases that are capable of hybridizing to one another. For example, with respect to DNA, adenosine is complementary to thymine and cytosine is complementary to guanine.

Hybridization conditions may comprise a hybridization step at Tm of 55°C, and utilizing conditions as set forth above. In other instances, the Tm is 60°C, 63°C, or 65°C.

Post-hybridization washes also determine stringency conditions. One set of conditions uses a series of washes starting with 6X SSC, 0.5% SDS at room temperature for 15 minutes (min), then repeated with 2X SSC, 0.5% SDS at 45°C for 30 min, and then repeated twice with 0.2X SSC, 0.5% SDS at 50°C for 30 min. A preferred set of stringent conditions uses higher temperatures in which the washes are identical to those above except for the temperature of the final two 30 min washes in 0.2X SSC, 0.5% SDS is increased to 60°C. Another preferred set of highly stringent conditions uses two final washes in 0.1X SSC, 0.1% SDS at 65°C.

The appropriate stringency for hybridizing nucleic acids depends on the length of the nucleic acids and the degree of complementation, variables well known in the art. The greater the degree of similarity or homology between two nucleotide sequences, the greater the value of Tm for hybrids of nucleic acids having those sequences. The relative stability (corresponding to higher Tm) of nucleic acid hybridizations decreases in the following order: RNA:RNA, DNA:RNA, DNA:DNA. For hybrids of greater than 100 nucleotides in length, equations for calculating Tm have been derived (see Sambrook *et al.,* supra, 9.50-0.51). For hybridization with shorter nucleic acids, *i.e.,* oligonucleotides, the position of mismatches becomes more important, and the length of the oligonucleotide determines its specificity (see Sambrook *et al.,* supra, 11.7-11.8).

Hybridization conditions may comprise a hybridization step in less than 500 mM salt and at least 37° C, and a washing step in 2X SSPE at a temperature of at least 63°C. The hybridization conditions may also comprise less than 200 mM salt and at least 37° C for the hybridization step. The hybridization conditions may further comprise 2X SSPE and 63°C for both the hybridization and washing steps.

The length for a hybridizable nucleic acid may be at least about 10 nucleotides. Preferably a minimum length for a hybridizable nucleic acid is at least about 15 nucleotides; *e.g.,* at least about 20 nucleotides; *e.g.,* at least 30 nucleotides. Furthermore, the skilled artisan will recognize that the temperature and wash solution salt concentration may be adjusted as necessary according to factors such as length of the probe.

The term "probe" refers to a single-stranded nucleic acid molecule that can base pair with a complementary single stranded target nucleic acid to form a double-stranded molecule.

As used herein, the term "oligonucleotide" refers to a short nucleic acid that is hybridizable to a genomic DNA molecule, a cDNA molecule, a plasmid DNA or an mRNA molecule. Oligonucleotides can be labeled, *e.g.,* with ³²P-nucleotides or nucleotides to which a label, such as biotin, has been covalently conjugated. A labeled oligonucleotide can be used as a probe to detect the presence of a nucleic acid. Oligonucleotides (one or both of which may be labeled) can be used as PCR primers, either for cloning full length or a fragment of a nucleic acid, for DNA sequencing, or to detect the presence of a nucleic acid. An oligonucleotide can also be used to form a triple helix with a DNA molecule. Generally, oligonucleotides are prepared synthetically, preferably on a nucleic acid synthesizer. Accordingly, oligonucleotides can be prepared with non-naturally occurring phosphoester analog bonds, such as thioester bonds, etc.

A "primer" refers to an oligonucleotide that hybridizes to a target nucleic acid sequence to create a double stranded nucleic acid region that can serve as an initiation point for DNA synthesis under suitable conditions. Such primers may be used in a polymerase chain reaction or for DNA sequencing.

"Polymerase chain reaction" is abbreviated PCR and refers to an in vitro method for enzymatically amplifying specific nucleic acid sequences. PCR involves a repetitive series of temperature cycles with each cycle comprising three stages: denaturation of the template nucleic acid to separate the strands of the target molecule, annealing a single stranded PCR oligonucleotide primer to the template nucleic acid, and extension of the annealed primer(s) by DNA polymerase. PCR provides a means to detect the presence of the target molecule and, under quantitative or semi-quantitative conditions, to determine the relative amount of that target molecule within the starting pool of nucleic acids.

"Reverse transcription-polymerase chain reaction" is abbreviated RT-PCR and refers to an *in vitro* method for enzymatically producing a target cDNA molecule or molecules from an RNA molecule or molecules, followed by enzymatic amplification of a specific nucleic acid sequence or sequences within the target cDNA molecule or molecules as described above. RT-PCR also provides a means to detect the presence of the target molecule and, under quantitative or semi-quantitative conditions, to determine the relative amount of that target molecule within the starting pool of nucleic acids.

A DNA "coding sequence" refers to a double-stranded DNA sequence that encodes a polypeptide and can be transcribed and translated into a polypeptide in a cell *in vitro* or *in vivo* when placed under the control of suitable regulatory sequences. "Suitable regulatory sequences" refers to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include promoters, translation leader sequences, introns, polyadenylation recognition sequences, RNA processing sites, effector binding sites and stem-loop structures. The boundaries of the coding sequence are determined by a start codon at the 5' (amino) terminus and a translation stop codon at the 3' (carboxyl) terminus. A coding sequence can include, but is not limited to, prokaryotic sequences, cDNA from mRNA, genomic DNA sequences, and even synthetic DNA sequences. If the coding sequence is intended for expression in a eukaryotic cell, a polyadenylation signal and transcription termination sequence will usually be located 3' to the coding sequence.

"Open reading frame" is abbreviated ORF and refers to a length of nucleic acid sequence, either DNA, cDNA or RNA, that comprises a translation start signal or initiation codon, such as an ATG or AUG, and a termination codon and can be potentially translated into a polypeptide sequence.

The term "downstream" refers to a nucleotide sequence that is located 3' to a reference nucleotide sequence. In particular, downstream nucleotide sequences generally relate to sequences that follow the starting point of transcription. For example, the translation initiation codon of a gene is located downstream of the start site of transcription.

The term "upstream" refers to a nucleotide sequence that is located 5' to a reference nucleotide sequence. In particular, upstream nucleotide sequences generally relate to sequences that are located on the 5' side of a coding sequence or starting point of transcription. For example, most promoters are located upstream of the start site of transcription.

"Homologous recombination" refers to the insertion of a foreign DNA sequence into another DNA molecule, *e.g.,* insertion of a vector in a chromosome. Preferably, the vector targets a specific chromosomal site for homologous recombination. For specific homologous recombination, the vector will contain sufficiently long regions of homology to sequences of the chromosome to allow complementary binding and incorporation of the vector into the chromosome. Longer regions of homology, and greater degrees of sequence similarity, may increase the efficiency of homologous recombination.

A "vector" refers to any vehicle for the cloning of and/or transfer of a nucleic acid into a host cell. A vector may be a replicon to which another DNA segment may be attached so as to bring about the replication of the attached segment. A "replicon" refers to any genetic element (*e.g.,* plasmid, phage, cosmid, chromosome, virus) that functions as an autonomous unit of DNA replication *in vivo, i.e.,* capable of replication under its own control. The term "vector" includes both viral and nonviral vehicles for introducing the nucleic acid into a cell *in vitro, ex vivo* or *in vivo.* A large number of vectors known in the art may be used to manipulate nucleic acids, incorporate response elements and promoters into genes, *etc.* Possible vectors include, for example, plasmids or modified viruses including, for example adenovirus, retrovirus, adeno-associated virus, herpesvirus, or plasmids such as pBR322 or pUC plasmid derivatives, or the Bluescript vector. For example, the insertion of the DNA fragments corresponding to response elements and promoters into a suitable vector can be accomplished by ligating the appropriate DNA fragments into a chosen vector that has complementary cohesive termini. Alternatively, the ends of the DNA molecules may be enzymatically modified or any site may be produced by ligating nucleotide sequences (linkers) into the DNA termini. Such vectors may be engineered to contain selectable marker genes that provide for the selection of cells that have incorporated the marker into the cellular genome. Such markers allow identification and/or selection of host cells that incorporate and express the proteins encoded by the marker.

Viral vectors have been used in a wide variety of gene delivery applications in cells, as well as living animal subjects. Viral vectors that can be used include, but are not limited to, adenovirus, retrovirus, vaccinia virus, poxvirus, adeno-associated virus, herpes simplex virus, lentivirus, baculovirus, sendai virus, measles virus, simian virus 40 and Epstein-Barr virus vectors. Non-viral vectors include plasmids, lipoplexes (cationic liposome-DNA complex), polyplexes (cationinc polymer-DNA complex), and protein-DNA complexes. In addition to a nucleic acid, a vector may also comprise one or more regulatory regions, and/or selectable markers useful in selecting, measuring, and monitoring nucleic acid transfer results (transfer to which tissues, duration of expression, *etc*.).

The term "plasmid" refers to an extra-chromosomal element often carrying a gene that is not part of the central metabolism of the cell, and usually in the form of circular double-stranded DNA molecules. Such elements may be autonomously replicating sequences, genome integrating sequences, phage or nucleotide sequences, linear, circular, or supercoiled, of a single- or double-stranded DNA or RNA, derived from any source, in which a number of nucleotide sequences have been joined or recombined into a unique construction which is capable of introducing a promoter fragment and DNA sequence for a selected gene product along with appropriate 3' untranslated sequence into a cell.

A "cloning vector" refers to a "replicon," which is a unit length of a nucleic acid, preferably DNA, that replicates sequentially and which comprises an origin of replication, such as a plasmid, phage or cosmid, to which another nucleic acid segment may be attached so as to bring about the replication of the attached segment. Cloning vectors may be capable of replication in one cell type and expression in another ("shuttle vector"). Cloning vectors may comprise one or more sequences that can be used for selection of cells comprising the vector and/or one or more multiple cloning sites for insertion of sequences of interest.

The term "expression vector" refers to a vector, plasmid or vehicle designed to enable the expression of an inserted nucleic acid sequence following transformation into the host. The cloned gene, *i.e*., the inserted nucleic acid sequence, is usually placed under the control of control elements such as a promoter, a minimal promoter, an enhancer, or the like. Initiation control regions or promoters, which are useful to drive expression of a nucleic acid in the desired host cell are numerous and familiar to those skilled in the art. Virtually any promoter capable of driving expression of these genes can be used in an expression vector, including but not limited to, viral promoters, bacterial promoters, animal promoters, mammalian promoters, synthetic promoters, constitutive promoters, tissue specific promoters, pathogenesis or disease related promoters, developmental specific promoters, inducible promoters, light regulated promoters; including, but are not limited to, the SV40 early (SV40) promoter region, the promoter contained in the 3' long terminal repeat (LTR) of Rous sarcoma virus (RSV), the E1A or major late promoter (MLP) of adenoviruses (Ad), the human cytomegalovirus (HCMV) immediate early promoter, the herpes simplex virus (HSV) thymidine kinase (TK) promoter, the baculovirus IE1 promoter, the elongation factor 1 alpha (EF1) promoter, the glyceraldehyde-3-phosphate dehydrogenase (GAPDH) promoter, the phosphoglycerate kinase (PGK) promoter, the ubiquitin C (Ubc) promoter, the albumin promoter, the regulatory sequences of the mouse metallothionein-L promoter and transcriptional control regions, the ubiquitous promoters (HPRT, vimentin, β-actin, tubulin and the like), the promoters of the intermediate filaments (desmin, neurofilaments, keratin, GFAP, and the like), the promoters of therapeutic genes (of the MDR, CFTR or factor VIII type, and the like), pathogenesis or disease related-promoters, and promoters that exhibit tissue specificity and have been utilized in transgenic animals, such as the elastase I gene control region which is active in pancreatic acinar cells; insulin gene control region active in pancreatic beta cells, immunoglobulin gene control region active in lymphoid cells, mouse mammary tumor virus control region active in testicular, breast, lymphoid and mast cells; albumin gene, Apo AI and Apo AII control regions active in liver, alpha-fetoprotein gene control region active in liver, alpha 1-antitrypsin gene control region active in the liver, beta-globin gene control region active in myeloid cells, myelin basic protein gene control region active in oligodendrocyte cells in the brain, myosin light chain-2 gene control region active in skeletal muscle, and gonadotropic releasing hormone gene control region active in the hypothalamus, pyruvate kinase promoter, villin promoter, promoter of the fatty acid binding intestinal protein, promoter of the smooth muscle cell β-actin, and the like. In addition, these expression sequences may be modified by addition of enhancer or regulatory sequences and the like.

The expression vectors constructed above are then administered to a subject in the form of a pharmaceutical composition. Two or more isoforms of HGF may be administered separately, either sequentially or at the same time, *i.e.,* co-administered; separate plasmids for the two or more isoforms of HGF may be adminstered or co-administered or a single expression plasmid containing genes for two or more isoforms of HGF and capable of expressing the genes for the two or more isoforms of HGF may be administered. For example, the two isoforms flHGF and dHGF may be administered using two separate plasmids. Alternatively, the two separate plasmids containing genes for flHGF and dHGF may be used for co-administration. Finally, a single expression plasmid containing genes for both flHGF and dHGF may be administered. In certain aspects the flHGF and dHGF on a single expression plasmid is encoded by the same polynucleotide or by separate polynucleotides. There are a number of approaches to include more than one polynucleotide capable of expressing an HGF isoform on a single plasmid. These include, for example, the use of Internal Ribosome Entry Site (IRES) sequences, dual promoters/expression cassettes, and fusion proteins. The two or more isoforms expressed from the same plasmid or on two separate plasmids, as discussed above, are selected from the group consisting of flHGF, dHGF, NK1, and NK2, or selected from the group consisting of SEQ ID NOs: 2-5 and 11-12. The two or more isoforms can also include additional HGF isoforms known to one of ordinary skill in the art.

Vectors may be introduced into the desired host cells by methods known in the art, *e.g.*, injection, transfection, electroporation, microinjection, transduction, cell fusion, lipofection, use of a gene gun, or a DNA vector transporter (see, *e.g.*, Wu et al., J. Biol. Chem. 267:963 (1992); Wu et al., J. Biol. Chem. 263:14621 (1988); and Hartmut *et al.,* Canadian Patent Application No. 2,012,311).

A polynucleotide can also be introduced *in vivo* by lipofection. For the past decade, there has been increasing use of liposomes for encapsulation and transfection of nucleic acids *in vitro.* Synthetic cationic lipids designed to limit the difficulties and dangers encountered with liposome-mediated transfection can be used to prepare liposomes for *in vivo* transfection of a gene (Felgner et al., Proc. Natl. Acad. Sci. USA. 84:7413 (1987); Mackey et al., Proc. Natl. Acad. Sci. USA 85:8027 (1988); and Ulmer et al., Science 259:1745 (1993)). The use of cationic lipids may promote encapsulation of negatively charged nucleic acids, and also promote fusion with negatively charged cell membranes (Felgner et al., Science 337:387 (1989)). Particularly useful lipid compounds and compositions for transfer of nucleic acids are described in WO95/18863, WO96/17823 and U.S. 5,459,127. The use of lipofection to introduce exogenous genes into the specific organs *in vivo* has certain practical advantages. Molecular targeting of liposomes to specific cells represents one area of benefit. It is clear that directing transfection to particular cell types would be particularly preferred in a tissue with cellular heterogeneity, such as pancreas, liver, kidney, and the brain. Lipids may be chemically coupled to other molecules for the purpose of targeting (Mackey *et al.* 1988, supra). Targeted peptides, *e.g.,* hormones or neurotransmitters, and proteins such as antibodies, or non-peptide molecules could be coupled to liposomes chemically.

Other molecules are also useful for facilitating transfection of a nucleic acid *in vivo,* such as a cationic oligopeptide *(e.g.,* WO95/21931), peptides derived from DNA binding proteins *(e.g.,* WO96/25508), or a cationic polymer *(e.g.,* WO95/21931).

It is also possible to introduce a vector *in vivo* as a naked DNA plasmid (see U.S. Patent Nos. 5,693,622, 5,589,466 and 5,580,859). Receptor-mediated DNA delivery approaches can also be used (Curiel et al., Hum. Gene Ther. 3:147 (1992); and Wu et al., J. Biol. Chem. 262:4429 (1987)).

The term "transfection" refers to the uptake of exogenous or heterologous RNA or DNA by a cell. A cell has been "transfected" by exogenous or heterologous RNA or DNA when such RNA or DNA has been introduced inside the cell. A cell has been "transformed" by exogenous or heterologous RNA or DNA when the transfected RNA or DNA effects a phenotypic change. The transforming RNA or DNA can be integrated (covalently linked) into chromosomal DNA making up the genome of the cell.

"Transformation" refers to the transfer of a nucleic acid fragment into a host organism, resulting in genetically stable inheritance. Host organisms containing the transformed nucleic acid fragments are referred to as "transgenic" or "recombinant" or "transformed" organisms.

In addition, the recombinant vector comprising a polynucleotide may include one or more origins for replication in the cellular hosts in which their amplification or their expression is sought, markers or selectable markers.

The term "selectable marker" refers to an identifying factor, usually an antibiotic or chemical resistance gene, that is able to be selected for based upon the marker gene's effect, *i.e.,* resistance to an antibiotic, resistance to a herbicide, colorimetric markers, enzymes, fluorescent markers, and the like, wherein the effect is used to track the inheritance of a nucleic acid of interest and/or to identify a cell or organism that has inherited the nucleic acid of interest. Examples of selectable marker genes known and used in the art include: genes providing resistance to ampicillin, streptomycin, gentamycin, kanamycin, hygromycin, bialaphos herbicide, sulfonamide, and the like; and genes that are used as phenotypic markers, *i.e.,* anthocyanin regulatory genes, isopentanyl transferase gene, and the like.

The term "reporter gene" refers to a nucleic acid encoding an identifying factor that is able to be identified based upon the reporter gene's effect, wherein the effect is used to track the inheritance of a nucleic acid of interest, to identify a cell or organism that has inherited the nucleic acid of interest, and/or to measure gene expression induction or transcription. Examples of reporter genes known and used in the art include: luciferase (Luc), green fluorescent protein (GFP), chloramphenicol acetyltransferase (CAT), β-galactosidase (LacZ), β-glucuronidase (Gus), and the like. Selectable marker genes may also be considered reporter genes.

"Promoter and "promoter sequence" are used interchangeably and refer to a DNA sequence capable of controlling the expression of a coding sequence or functional RNA. In general, a coding sequence is located 3' to a promoter sequence. Promoters may be derived in their entirety from a native gene, or be composed of different elements derived from different promoters found in nature, or even comprise synthetic DNA segments. It is understood by those skilled in the art that different promoters may direct the expression of a gene in different tissues or cell types, or at different stages of development, or in response to different environmental or physiological conditions. Promoters that cause a gene to be expressed in most cell types at most times are commonly referred to as "constitutive promoters." Promoters that cause a gene to be expressed in a specific cell type are commonly referred to as "cell-specific promoters" or "tissue-specific promoters." Promoters that cause a gene to be expressed at a specific stage of development or cell differentiation are commonly referred to as "developmentally-specific promoters" or "cell differentiation-specific promoters." Promoters that are induced and cause a gene to be expressed following exposure or treatment of the cell with an agent, biological molecule, chemical, ligand, light, or the like that induces the promoter are commonly referred to as "inducible promoters" or "regulatable promoters." It is further recognized that since in most cases the exact boundaries of regulatory sequences have not been completely defined, DNA fragments of different lengths may have identical promoter activity.

The promoter sequence is typically bounded at its 3' terminus by the transcription initiation site and extends upstream (5' direction) to include the minimum number of bases or elements necessary to initiate transcription at levels detectable above background. Within the promoter sequence will be found a transcription initiation site (conveniently defined for example, by mapping with nuclease S1), as well as protein binding domains (consensus sequences) responsible for the binding of RNA polymerase.

A coding sequence is "under the control" of transcriptional and translational control sequences in a cell when RNA polymerase transcribes the coding sequence into mRNA, which is then trans-RNA spliced (if the coding sequence contains introns) and translated into the protein encoded by the coding sequence.

"Transcriptional and translational control sequences" refer to DNA regulatory sequences, such as promoters, enhancers, terminators, and the like, that provide for the expression of a coding sequence in a host cell. In eukaryotic cells, polyadenylation signals are control sequences.

The term "response element" refers to one or more cis-acting DNA elements which confer responsiveness on a promoter mediated through interaction with the DNA-binding domains of a transcription factor. This DNA element may be either palindromic (perfect or imperfect) in its sequence or composed of sequence motifs or half sites separated by a variable number of nucleotides. The half sites can be similar or identical and arranged as either direct or inverted repeats or as a single half site or multimers of adjacent half sites in tandem. The response element may comprise a minimal promoter isolated from different organisms depending upon the nature of the cell or organism into which the response element will be incorporated. The DNA binding domain of the transcription factor binds, in the presence or absence of a ligand, to the DNA sequence of a response element to initiate or suppress transcription of downstream gene(s) under the regulation of this response element.

The term "operably linked" refers to the association of nucleic acid sequences on a single nucleic acid fragment so that the function of one is affected by the other. For example, a promoter is operably linked with a coding sequence when it is capable of affecting the expression of that coding sequence *(i.e.,* that the coding sequence is under the transcriptional control of the promoter). Coding sequences can be operably linked to regulatory sequences in sense or antisense orientation.

The term "expression" as used herein refers to the transcription and stable accumulation of sense (mRNA) or antisense RNA derived from a nucleic acid or polynucleotide. Expression may also refer to translation of mRNA into a protein or polypeptide.

The terms "cassette," "expression cassette" and "gene expression cassette" refer to a segment of DNA that can be inserted into a nucleic acid or polynucleotide at specific restriction sites or by homologous recombination. The segment of DNA comprises a polynucleotide that encodes a polypeptide of interest, and the cassette and restriction sites are designed to ensure insertion of the cassette in the proper reading frame for transcription and translation. "Transformation cassette" refers to a specific vector comprising a polynucleotide that encodes a polypeptide of interest and having elements in addition to the polynucleotide that facilitate transformation of a particular host cell. Cassettes, expression cassettes, gene expression cassettes and transformation cassettes may also comprise elements that allow for enhanced expression of a polynucleotide encoding a polypeptide of interest in a host cell. These elements may include, but are not limited to: a promoter, a minimal promoter, an enhancer, a response element, a terminator sequence, a polyadenylation sequence, and the like.

The term "gene switch" refers to the combination of a response element associated with a promoter, and a ligand-dependent transcription factor-based system which, in the presence of one or more ligands, modulates the expression of a gene into which the response element and promoter are incorporated.

The terms "modulate" and "modulates" mean to induce, reduce or inhibit nucleic acid or gene expression, resulting in the respective induction, reduction or inhibition of protein or polypeptide production.

Enhancers that may be used in embodiments of the invention include but are not limited to: an SV40 enhancer, a cytomegalovirus (CMV) enhancer, an elongation factor 1 (EF1) enhancer, yeast enhancers, viral gene enhancers, and the like.

Termination control regions, *i.e.,* terminator or polyadenylation sequences, may also be derived from various genes native to the preferred hosts. Optionally, a termination site may be unnecessary, however, it is most preferred if included. In a one embodiment of the invention, the termination control region may be comprised or be derived from a synthetic sequence, synthetic polyadenylation signal, an SV40 late polyadenylation signal, an SV40 polyadenylation signal, a bovine growth hormone (BGH) polyadenylation signal, viral terminator sequences, or the like.

The terms "3' non-coding sequences" or "3' untranslated region (UTR)" refer to DNA sequences located downstream (3') of a coding sequence and may comprise polyadenylation [poly(A)] recognition sequences and other sequences encoding regulatory signals capable of affecting mRNA processing or gene expression. The polyadenylation signal is usually characterized by affecting the addition of polyadenylic acid tracts to the 3' end of the mRNA precursor.

"Regulatory region" refers to a nucleic acid sequence that regulates the expression of a second nucleic acid sequence. A regulatory region may include sequences which are naturally responsible for expressing a particular nucleic acid (a homologous region) or may include sequences of a different origin that are responsible for expressing different proteins or even synthetic proteins (a heterologous region). In particular, the sequences can be sequences of prokaryotic, eukaryotic, or viral genes or derived sequences that stimulate or repress transcription of a gene in a specific or non-specific manner and in an inducible or non-inducible manner. Regulatory regions include origins of replication, RNA splice sites, promoters, enhancers, transcriptional termination sequences, and signal sequences which direct the polypeptide into the secretory pathways of the target cell.

A regulatory region from a "heterologous source" refers to a regulatory region that is not naturally associated with the expressed nucleic acid. Included among the heterologous regulatory regions are regulatory regions from a different species, regulatory regions from a different gene, hybrid regulatory sequences, and regulatory sequences which do not occur in nature, but which are designed by one having ordinary skill in the art.

"RNA transcript" refers to the product resulting from RNA polymerase-catalyzed transcription of a DNA sequence. When the RNA transcript is a perfect complementary copy of the DNA sequence, it is referred to as the primary transcript or it may be a RNA sequence derived from post-transcriptional processing of the primary transcript and is referred to as the mature RNA. "Messenger RNA (mRNA)" refers to the RNA that is without introns and that can be translated into protein by the cell. "cDNA" refers to a double-stranded DNA that is complementary to and derived from mRNA. "Sense" RNA refers to RNA transcript that includes the mRNA and so can be translated into protein by the cell. "Antisense RNA" refers to a RNA transcript that is complementary to all or part of a target primary transcript or mRNA and that blocks the expression of a target gene. The complementarity of an antisense RNA may be with any part of the specific gene transcript, *i.e.,* at the 5' non-coding sequence, 3' non-coding sequence, or the coding sequence. "Functional RNA" refers to antisense RNA, ribozyme RNA, or other RNA that is not translated yet has an effect on cellular processes.

"Polypeptide," "peptide" and "protein" are used interchangeably and refer to a polymeric compound comprised of covalently linked amino acid residues.

An "isolated polypeptide," "isolated peptide" or "isolated protein" refer to a polypeptide or protein that is substantially free of those compounds that are normally associated therewith in its natural state (*e.g.*, other proteins or polypeptides, nucleic acids, carbohydrates, lipids). "Isolated" is not meant to exclude artificial or synthetic mixtures with other compounds, or the presence of impurities which do not interfere with biological activity, and which may be present, for example, due to incomplete purification, addition of stabilizers, or compounding into a pharmaceutically acceptable preparation.

A mutation may be made by any technique for mutagenesis known in the art, including but not limited to, *in vitro* site-directed mutagenesis (Hutchinson et al., J. Biol. Chem. 253:6551 (1978); Zoller et al., DNA 3:479 (1984); Oliphant et al., Gene 44:177 (1986); Hutchinson et al., Proc. Natl. Acad. Sci. USA 83:710 (1986)), use of TAB® linkers (Pharmacia), restriction endonuclease digestion/fragment deletion and substitution, PCR-mediated/oligonucleotide-directed mutagenesis, and the like. PCR-based techniques are preferred for site-directed mutagenesis (see Higuchi, 1989, "Using PCR to Engineer DNA", in PCR Technology: Principles and Applications for DNA Amplification, H. Erlich, ed., Stockton Press, Chapter 6, pp. 61-70).

A "variant" of a polypeptide or protein refers to any analogue, fragment, derivative, or mutant which is derived from a polypeptide or protein and which retains at least one biological property of the polypeptide or protein. Different variants of the polypeptide or protein may exist in nature. These variants may be allelic variations characterized by differences in the nucleotide sequences of the structural gene coding for the protein, or may involve differential splicing or post-translational modification. The skilled artisan can produce variants having single or multiple amino acid substitutions, deletions, additions, or replacements. These variants may include, inter alia: (a) variants in which one or more amino acid residues are substituted with conservative or non-conservative amino acids, (b) variants in which one or more amino acids are added to the polypeptide or protein, (c) variants in which one or more of the amino acids includes a substituent group, and (d) variants in which the polypeptide or protein is fused with another polypeptide such as serum albumin. The techniques for obtaining these variants, including genetic (suppressions, deletions, mutations, *etc.*), chemical, and enzymatic techniques, are known to persons having ordinary skill in the art.

The term "homology" refers to the percent of identity between two polynucleotide or two polypeptide moieties. The correspondence between the sequence from one moiety to another can be determined by techniques known to the art. For example, homology can be determined by a direct comparison of the sequence information between two polypeptide molecules by aligning the sequence information and using readily available computer programs. Alternatively, homology can be determined by hybridization of polynucleotides under conditions that form stable duplexes between homologous regions, followed by digestion with single-stranded-specific nuclease(s) and size determination of the digested fragments.

As used herein, the term "homologous" in all its grammatical forms and spelling variations refers to the relationship between proteins that possess a "common evolutionary origin," including proteins from superfamilies (e.g., the immunoglobulin superfamily) and homologous proteins from different species *(e.g.,* myosin light chain, etc.) (Reeck et al., Cell 50:667 (1987)). Such proteins (and their encoding genes) have sequence homology, as reflected by their high degree of sequence similarity. However, in common usage and in the present application, the term "homologous," when modified with an adverb such as "highly," may refer to sequence similarity and not a common evolutionary origin.

Accordingly, the term "sequence similarity" in all its grammatical forms refers to the degree of identity or correspondence between nucleic acid or amino acid sequences of proteins that may or may not share a common evolutionary origin (see Reeck et al., Cell 50:667 (1987)). Two DNA sequences may be "substantially homologous" or "substantially similar" when at least about 50% *(e.g.,* at least about 75%, 90%, or 95%) of the nucleotides match over the defined length of the DNA sequences. Sequences that are substantially homologous can be identified by comparing the sequences using standard software available in sequence data banks, or in a Southern hybridization experiment under, for example, stringent conditions as defined for that particular system. Defining appropriate hybridization conditions is within the skill of the art (see *e.g.,* Sambrook *et al.,* 1989, supra).

As used herein, "substantially similar" refers to nucleic acid fragments wherein changes in one or more nucleotide bases results in substitution of one or more amino acids, but do not affect the functional properties of the protein encoded by the DNA sequence. "Substantially similar" also refers to nucleic acid fragments wherein changes in one or more nucleotide bases do not affect the ability of the nucleic acid fragment to mediate alteration of gene expression by antisense or co-suppression technology. "Substantially similar" also refers to modifications of the nucleic acid fragments of the present invention such as deletion or insertion of one or more nucleotide bases that do not substantially affect the functional properties of the resulting transcript. Each of the proposed modifications is well within the routine skill in the art, as is determination of retention of biological activity of the encoded products.

Moreover, the skilled artisan recognizes that substantially similar sequences are also defined by their ability to hybridize, under stringent conditions (0.1X SSC, 0.1% SDS, 65°C and washed with 2X SSC, 0.1% SDS followed by 0.1X SSC, 0.1% SDS), with the sequences exemplified herein. Substantially similar nucleic acid fragments of the present invention are those nucleic acid fragments whose DNA sequences are at least about 70%, 80%, 90% or 95% identical to the DNA sequence of the nucleic acid fragments reported herein.

Two amino acid sequences are "substantially homologous" or "substantially similar" when greater than about 40% of the amino acids are identical, or greater than 60% are similar (functionally identical). Preferably, the similar or homologous sequences are identified by alignment using, for example, the GCG (Genetics Computer Group, Program Manual for the GCG Package, Version 7, Madison, Wisconsin) pileup program.

The term "corresponding to" is used herein to refer to similar or homologous sequences, whether the exact position is identical or different from the molecule to which the similarity or homology is measured. A nucleic acid or amino acid sequence alignment may include spaces. Thus, the term "corresponding to" refers to the sequence similarity, and not the numbering of the amino acid residues or nucleotide bases.

A "substantial portion" of an amino acid or nucleotide sequence comprises enough of the amino acid sequence of a polypeptide or the nucleotide sequence of a gene to putatively identify that polypeptide or gene, either by manual evaluation of the sequence by one skilled in the art, or by computer-automated sequence comparison and identification using algorithms such as BLAST (Basic Local Alignment Search Tool; Altschul et al., J. Mol. Biol. 215:403 (1993)); available at ncbi.nlm.nih.gov/BLAST/). In general, a sequence of ten or more contiguous amino acids or thirty or more nucleotides is necessary in order to putatively identify a polypeptide or nucleic acid sequence as homologous to a known protein or gene. Moreover, with respect to nucleotide sequences, gene specific oligonucleotide probes comprising 20 30 contiguous nucleotides may be used in sequence-dependent methods of gene identification (*e.g.*, Southern hybridization) and isolation (*e.g., in situ* hybridization of bacterial colonies or bacteriophage plaques). In addition, short oligonucleotides of 12-15 bases may be used as amplification primers in PCR in order to obtain a particular nucleic acid fragment comprising the primers. Accordingly, a "substantial portion" of a nucleotide sequence comprises enough of the sequence to specifically identify and/or isolate a nucleic acid fragment comprising the sequence.

The term "percent identity," as known in the art, is a relationship between two or more polypeptide sequences or two or more polynucleotide sequences, as determined by comparing the sequences. In the art, "identity" also means the degree of sequence relatedness between polypeptide or polynucleotide sequences, as the case may be, as determined by the match between strings of such sequences. "Identity" and "similarity" can be readily calculated by known methods, including but not limited to those described in: Computational Molecular Biology (Lesk, A. M., ed.) Oxford University Press, New York (1988); Biocomputing: Informatics and Genome Projects (Smith, D. W., ed.) Academic Press, New York (1993); Computer Analysis of Sequence Data, Part I (Griffin, A. M., and Griffin, H. G., eds.) Humana Press, New Jersey (1994); Sequence Analysis in Molecular Biology (von Heinje, G., ed.) Academic Press (1987); and Sequence Analysis Primer (Gribskov, M. and Devereux, J., eds.) Stockton Press, New York (1991). Preferred methods to determine identity are designed to give the best match between the sequences tested. Methods to determine identity and similarity are codified in publicly available computer programs. Sequence alignments and percent identity calculations may be performed using sequence analysis software such as the Megalign program of the LASERGENE bioinformatics computing suite (DNASTAR Inc., Madison, WI). Multiple alignment of the sequences may be performed using the Clustal method of alignment (Higgins et al., CABIOS. 5:151 (1989)) with the default parameters (GAP PENALTY=10, GAP LENGTH PENALTY=10). Default parameters for pairwise alignments using the Clustal method may be selected: KTUPLE 1, GAP PENALTY=3, WINDOW=5 and DIAGONALS SAVED=5.

The term "sequence analysis software" refers to any computer algorithm or software program that is useful for the analysis of nucleotide or amino acid sequences. "Sequence analysis software" may be commercially available or independently developed. Typical sequence analysis software includes, but is not limited to, the GCG suite of programs (Wisconsin Package Version 9.0, Genetics Computer Group (GCG), Madison, WI), BLASTP, BLASTN, BLASTX (Altschul et al., J. Mol. Biol. 215:403 (1990)), and DNASTAR (DNASTAR, Inc. 1228 S. Park St. Madison, WI 53715 USA). Within the context of this disclosure it will be understood that where sequence analysis software is used for analysis, that the results of the analysis will be based on the "default values" of the program referenced, unless otherwise specified. As used herein "default values" will mean any set of values or parameters which originally load with the software when first initialized.

"Chemically synthesized," as related to a sequence of DNA, means that the component nucleotides were assembled *in vitro*. Manual chemical synthesis of DNA may be accomplished using well-established procedures, or automated chemical synthesis can be performed using one of a number of commercially available machines. Accordingly, the genes can be tailored for optimal gene expression based on optimization of nucleotide sequence to reflect the codon bias of the host cell. The skilled artisan appreciates the likelihood of successful gene expression if codon usage is biased towards those codons favored by the host. Determination of preferred codons can be based on a survey of genes derived from the host cell where sequence information is available.

The term "exogenous gene" means a gene foreign to the subject, that is, a gene which is introduced into the subject through a transformation process, an unmutated version of an endogenous mutated gene or a mutated version of an endogenous unmutated gene. The method of transformation is not critical and may be any method suitable for the subject known to those in the art. Exogenous genes can be either natural or synthetic genes which are introduced into the subject in the form of DNA or RNA which may function through a DNA intermediate such as by reverse transcriptase. Such genes can be introduced into target cells, directly introduced into the subject, or indirectly introduced by the transfer of transformed cells into the subject.

The term "subject" means an intact animal, preferably a vertebrate, most preferably a mammal.

The term "isoform of HGF" refers to any HGF polypeptide having an amino acid sequence that is at least 80% identical (e.g., at least 90% or 95% identical) to a HGF amino acid sequence that is naturally produced in an animal, including all allelic variants. In one embodiment, the term refers to isoforms that are known to have cell proliferation activity. Isoforms of HGF include, without limitation, flHGF, dHGF, NK1, NK2, and NK4.

The term "flHGF" refers to the full length HGF protein of an animal, *e.g.,* a mammal, *e.g.,* amino acids 1-728 of human HGF.

The term "dHGF" refers to the deleted variant of HGF protein produced by alternative splicing of the HGF gene in an animal, *e.g.,* a mammal, *e.g.,* human HGF consisting of 723 amino acids with deletion of five amino acids in the 1st kringle domain of the alpha chain (F, L, P, S and S) from the full length HGF sequence.

The term "NK1" refers to an isoform of HGF from an animal, *e.g.,* a mammal, *e.g.,* a human, consisting of the N-terminal hairpin loop and kringle1 domains.

The term "NK2" refers to an isoform of HGF from an animal, *e.g.,* a mammal, *e.g.,* a human, consisting of the N-terminal hairpin loop, kringle1, and kringle2 domains.

The term "NK4" refers to an isoform of HGF from an animal, *e.g.,* a mammal, *e.g.,* a human, consisting of the N-terminal hairpin loop, kringle1, kringle2, kringle3, and kringle4 domains.

The composition of the present invention comprises two or more isoforms of HGF selected from flHGF, dHGF, NK1, NK2 and NK4. In one embodiment, the two or more isoforms of HGF are isoforms of a mammalian HGF, *e.g.,* human HGF. The amino acid sequence of HGF from various species is well known in the art and can be found in sequence databases such as GenBank (the amino acid sequence of human HGF having accession number BAA14348). In one embodiment, one of the isoforms of HGF is human flHGF (SEQ ID NO: 2). In another embodiment, one of the isoforms of HGF is human dHGF (SEQ ID NO: 3). In another embodiment, one of the isoforms of HGF is human NK1 (SEQ ID NO: 4). In another embodiment, one of the isoforms of HGF is human NK2 (SEQ ID NO: 5). In another embodiment, one of the isoforms of HGF is human NK4 (SEQ ID NO: 6). In a further embodiment, the two or more isoforms of HGF comprise flHGF and dHGF. In a different embodiment, the two or more isoforms of HGF consist of flHGF and dHGF.

In one embodiment, the isoforms of HGF are variants of human wild-type HGF isoforms. For example, the isoforms may be variants of the human flHGF, dHGF, NK1, NK2, or NK4 sequence having at least 80% sequence identity to the wild-type human flHGF (SEQ ID NO: 2), dHGF (SEQ ID NO: 3), NK1 (SEQ ID NO: 4), NK2 (SEQ ID NO: 5), or NK4 (SEQ ID NO: 6) sequence, *e.g.,* at least 85, 90, 95, 96, 97, 98, or 99% sequence identity. The variant may comprise additions, deletions, substitutions, or a combination thereof to the amino acid sequence of a wild-type human HGF isoform. Additions or substitutions also include the use of non-naturally occurring amino acids and may occur in any number internally, at the N-terminus and/or at the C-terminus.

Preferably, any substitutions are conservative amino acid substitutions. A "conservative amino acid substitution" is one in which the amino acid residue is replaced with an amino acid residue having a similar side chain. Families of amino acid residues having similar side chains have been defined within the art. These families include amino acids with basic side chains (*e.g.,* lysine, arginine, histidine), acidic side chains (*e.g.,* aspartic acid, glutamic acid), uncharged polar side chains (*e.g.,* glycine, asparagine, glutamine, serine, threonine, tyrosine, cysteine), nonpolar side chains (*e.g.,* alanine, valine, leucine, isoleucine, proline, phenylalanine, methionine, tryptophan), beta-branched side chains (*e.g.,* threonine, valine, isoleucine) and aromatic side chains (*e.g.,* tyrosine, phenylalanine, tryptophan, histidine).

Sequence identity is calculated by comparing two optimally aligned sequences over that region of comparison, determining the number of positions at which the identical amino acid residue occurs in both sequences to yield the number of matched positions, dividing the number of matched positions by the total number of positions in the region of comparison (*i.e.,* the window size), and multiplying the result by 100 to yield the percentage of sequence identity. In one aspect, percent identity is calculated as the percentage of amino acid residues in the smaller of two sequences which align with an identical amino acid residue in the sequence being compared, when four gaps in a length of 100 amino acids may be introduced to maximize alignment (Dayhoff, in Atlas of Protein Sequence and Structure, Vol. 5, p. 124, National Biochemical Research Foundation, Washington, D.C. (1972)). A determination of identity is typically made by a computer homology program known in the art. An exemplary program is the Gap program (Wisconsin Sequence Analysis Package, Version 8 for UNIX, Genetics Computer Group, University Research Park, Madison, WI) using the default settings, which uses the algorithm of Smith and Waterman (Adv. Appl. Math. 2:482 (1981)).

In one embodiment, the variant of an isoform of HGF retains substantially all of any one or more of the biological activities of the wild-type HGF isoform protein. The term "substantially all of the biological activity of the wild-type HGF," as used herein, refers to a variant of an HGF isoform which retains at least 70% of any one or more of the biological activities of the HGF isoform (*e.g.,* the ability to stimulate angiogenesis or promote cell proliferation). In some embodiments, at least 75, 80, 85, 90, or 95% of one or more of the biological activities of the HGF isoform is retained. HGF activity may be determined by *routine in vitro* and *in vivo* assays well known in the art (*e.g., in vivo* Matrigel plug and corneal neovascularization assays, *in vivo*/*in vitro* chick chorioallantoic membrane (CAM) assay, *in vitro* cellular (proliferation, migration, tube formation) and organotypic (aortic ring) assays).

The structure and function of HGF has been extensively studied and one of skill in the art is aware of the amino acids in the HGF sequence that are important for retaining substantially all of the biological activity of the protein and that are preferably not changed or only conservatively changed in any sequence variant of HGF. *See, e.g.,* Hartmann et al., Proc. Natl. Acad. Sci. USA 89:11574 (1992); Lokker et al., EMBO J. 11:2503 (1992), Zhou et al., Structure 6:109 (1998), Ultsch et al., Structure 6:1383 (1998), Shimizu et al., Biochem. Biophys. Res. Commun. 189:1329 (1992), Yoshiyama et al., Biochem. Biophys. Res. Commun. 175:660 (1991). For example, it appears that the N-terminal hairpin loop and kringle 1 domains are required for cell proliferation activity. Other amino acids that are not critical to biological activity may be deleted and/or substituted more freely. One of skill in the art can prepare variants of HGF isoforms using routine mutagenesis techniques, such as those described in the references cited above, and identify variants retaining substantially all of the biological activity of the HGF isoform.

In one embodiment of the invention, the two or more isoforms of HGF are present in the composition as one or more polynucleotides encoding the isoforms. In one embodiment, the one or more polynucleotides encode isoforms of mammalian HGF, *e.g.,* human HGF. The polynucleotide sequences of the HGF gene from various species are well known in the art and can be found in sequence databases such as GenBank (the polynucleotide sequence of the human HGF gene having accession number NM000601). In one embodiment, a polynucleotide encodes flHGF. In another embodiment, a polynucleotide encodes dHGF. In another embodiment, a polynucleotide encodes NK1. In another embodiment, a polynucleotide encodes NK2. In another embodiment, a polynucleotide encodes NK4. In a further embodiment, one or more polynucleotides encode both flHGF and dHGF. In a further embodiment, the one or more polynucleotides encode flHGF, dHGF, and NK1.

In one embodiment, the one or more polynucleotides encoding the two or more isoforms of HGF comprise a wild-type human HGF gene sequence. In another embodiment, the polynucleotides comprise a sequence variant of the wild-type HGF gene but still encode the wild-type amino acid sequence of the HGF isoform due to codon degeneracy. In a further embodiment, the one or more polynucleotides encode HGF isoform protein sequence variants as described above. In one embodiment, the polynucleotide sequence variants of the human HGF isoform gene sequence have at least 80% sequence identity to the wild-type human HGF isoform gene sequence, *e.g.,* at least 85, 90, 95, 96, 97, 98, or 99% identity.

In one embodiment of the invention, the two or more isoforms of HGF are encoded by a hybrid HGF gene that simultaneously expresses two or more of the isoforms, *e.g.,* flHGF and dHGF. The hybrid HGF gene described in US 2005/0079581 A1 comprises cDNA corresponding to exons 1 to 18 of HGF, and an inherent or foreign intron inserted between exons 4 and 5 of the cDNA, wherein the hybrid gene is devoid of other introns between exons other than the intron between exons 4 and 5. The intron comprises a fragment of the inherent intron or a recombinant sequence.

An embodiment of the hybrid HGF gene comprising the inherent intron is 7113 bp long and has the nucleotide sequence of SEQ ID NO: 7. The hybrid HGF gene simultaneously expresses both flHGF and dHGF, and has higher expression efficiency than flHGF cDNA.

Codon degeneracy enables the hybrid HGF gene to be modified or changed in the coding and/or non-coding region without altering the amino acid sequence of the protein and the expression of the gene. Accordingly, polynucleotides which are substantially identical to the hybrid HGF gene of SEQ ID NO: 7, and the fragments thereof fall within the scope of the invention. "Substantially identical" means that the sequence identity is not less than 80%, *e.g.,* not less than 90%, *e.g.,* not less than 95%.

A hybrid HGF gene may comprise a fragment of inherent intron optionally having a small recombinant sequence inserted therein between exons 4 and 5 of the HGF cDNA. Herein, such a hybrid HGF gene comprising a fragment of inherent intron is designated "HGF-X". Examples include HGF-X6, HGF-X7 and HGF-X8 having the nucleotide sequence of SEQ ID NOs: 8 to 10, respectively.

The two or more isoforms of HGF to be administered may be encoded by separate polynucleotides or a single polynucleotide. The polynucleotides may be operably linked to one or more regulatory sequences, *e.g.,* promoters or enhancers, controlling expression of the HGF isoforms. The promoter may be a constitutive promoter (*e.g.,* the human cytomegalovirus promoter) or an inducible promoter. The regulatory sequences may be part of a gene switch that regulates expression of the HGF isoforms by addition or removal of a specific ligand. Examples of ligand-dependent transcription factors that may be used in the gene switches of the invention include, without limitation, members of the nuclear receptor superfamily activated by their respective ligands (*e.g.,* glucocorticoid, estrogen, progestin, retinoid, ecdysone, and analogs and mimetics thereof) and rTTA activated by tetracycline. In a further embodiment, the promoter is a cardiomyocyte-specific promoter, (*e.g.,* cardiac ankyrin repeat protein, MYBPC3). When the two or more isoforms of HGF are encoded by separate polynucleotides, each polynucleotide may be operably linked to its own regulatory sequences. In another embodiment, the two or more polynucleotides may be under the control of a single regulatory sequence as part of a tandem cassette, optionally with sequences inserted between the two or more polynucleotides, *e.g.,* an internal ribosome binding site, to promote expression of all of the isoforms.

In one embodiment of the invention, the one or more polynucleotides encoding the two or more isoforms of HGF are part of a vector, *e.g.,* an expression vector. The vector may be, for example, a plasmid vector (*e.g.,* a pCK vector as disclosed in Lee et al., Biochem. Biophys. Res. Commun. 272:230 (2000); WO 2000/040737) or a single-or double-stranded RNA or DNA viral vector. Such vectors may be introduced into cells by well-known techniques for introducing DNA and RNA into cells. Viral vectors may be replication competent or replication defective. In the latter case, viral propagation generally will occur only in complementing host cells. As used herein, the term "host cell" or "host" is used to mean a cell of use in the present invention that is harboring one or more polynucleotides of the invention.

Thus, at a minimum, the vectors must include the polynucleotides of use in the invention. Other components of the vector may include, but are not limited to, selectable markers, chromatin modification domains, additional promoters driving expression of other polypeptides that may also be present on the vector (*e.g.,* a lethal polypeptide), genomic integration sites, recombination sites, and molecular insertion pivots. The vectors may comprise any number of these additional elements, either within or not within the polynucleotides, such that the vector can be tailored to the specific goals of the therapeutic methods desired.

The vectors that are introduced into the cells may further comprise a "selectable marker gene" which, when expressed, indicates that the vector has been introduced into the host cell. In this manner, the selector gene can be a positive marker for the presence of vector. While not critical to the present invention, the presence of a selectable marker gene allows the practitioner to select for a population of live cells where the vector construct has been introduced into the cells. Thus, cells where the vector has successfully been introduced may be selected. As used herein, the term "select" or variations thereof, when used in conjunction with cells, is intended to mean standard, well-known methods for choosing cells with a specific genetic make-up or phenotype. Typical methods include, but are not limited to, culturing cells in the presence of antibiotics, such as G418, puromycin and ampicillin. Other examples of selectable marker genes include, but are not limited to, genes that confer resistance to methotrexate, hygromycin, or mycophenolic acid. Cells comprising a vector construct comprising an antibiotic resistance gene or genes would then be capable of tolerating the antibiotic in culture. Likewise, cells not comprising a vector construct comprising an antibiotic resistance gene or genes would not be capable of tolerating the antibiotic in culture.

As used herein, a "chromatin modification domain" (CMD) refers to nucleotide sequences that interact with a variety of proteins associated with maintaining and/or altering chromatin structure, such as, but not limited to, DNA insulators. See Ciavatta et al., Proc. Natl. Acad. Sci. U.S.A. 103:9958 (2006). Examples of CMDs include, but are not limited to, the chicken β-globulin insulator and the chicken hypersensitive site 4 (cHS4). The use of different CMD sequences between one or more gene programs (*i.e.,* a promoter, coding sequence, and 3' regulatory region), for example, can facilitate the use of the differential CMD DNA sequences as "mini homology arms" in combination with various microorganism or *in vitro* recombineering technologies to "swap" gene programs between existing multigenic and monogenic shuttle vectors. Other examples of chromatin modification domains are known in the art or can be readily identified.

Particular vectors for use with the present invention are expression vectors that code for proteins or polynucleotides. Generally, such vectors comprise cis-acting control regions effective for expression in a host operatively linked to the polynucleotide to be expressed. Appropriate trans-acting factors are supplied by the host, supplied by a complementing vector or supplied by the vector itself upon introduction into the host.

A great variety of expression vectors can be used to express proteins or polynucleotides. Such vectors include chromosomal, episomal and virus-derived vectors, e.g., vectors derived from bacterial plasmids, from bacteriophage, from yeast episomes, from yeast chromosomal elements, from viruses such as adeno-associated viruses, lentiviruses, baculoviruses, papova viruses, SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. All may be used for expression in accordance with this aspect of the present invention. Generally, any vector suitable to maintain, propagate or express polynucleotides or proteins in a host may be used for expression in this regard.

The polynucleotide sequence in the expression vector is operatively linked to appropriate expression control sequence(s) including, for instance, a promoter to direct mRNA transcription. Representatives of additional promoters include, but are not limited to, constitutive promoters and tissue specific or inducible promoters. Examples of constitutive eukaryotic promoters include, but are not limited to, the promoter of the mouse metallothionein I gene (Hamer et al., J. Mol. Appl. Gen. 1:273 (1982)); the TK promoter of Herpes virus (McKnight, Cell 31:355 (1982)); the SV40 early promoter (Benoist et al., Nature 290:304 (1981)); and the vaccinia virus promoter. Additional examples of the promoters that could be used to drive expression of a protein or polynucleotide include, but are not limited to, tissue-specific promoters and other endogenous promoters for specific proteins, such as the albumin promoter (hepatocytes), a proinsulin promoter (pancreatic beta cells) and the like. In general, expression constructs will contain sites for transcription, initiation and termination and, in the transcribed region, a ribosome binding site for translation. The coding portion of the mature transcripts expressed by the constructs may include a translation initiating AUG at the beginning and a termination codon (UAA, UGA or UAG) appropriately positioned at the end of the polypeptide to be translated.

In addition, the constructs may contain control regions that regulate, as well as engender expression. Generally, such regions will operate by controlling transcription, such as repressor binding sites and enhancers, among others. Examples of eukaryotic vectors include, but are not limited to, pW-LNEO, pSV2CAT, pOG44, pXT1 and pSG available from Stratagene; pSVK3, pBPV, pMSG and pSVL available from Amersham Pharmacia Biotech; and pCMVDsRed2-express, pIRES2-DsRed2, pDsRed2-Mito, and pCMV-EGFP available from Clontech. Many other vectors are well-known and commercially available.

Selection of appropriate vectors and promoters for expression in a host cell is a well-known procedure, and the requisite techniques for vector construction and introduction into the host, as well as its expression in the host are routine skills in the art.

The introduction of the polynucleotides into the cells can be a transient transfection or stable transfection of the vector. Transient transfection of the vectors into the host cell can be effected by direct injection, calcium phosphate transfection, DEAE-dextran mediated transfection, cationic lipid-mediated transfection, electroporation, transduction, infection, or other methods. Such methods are described in many standard laboratory manuals, such as Davis et al., Basic Methods in Molecular Biology (1986); Keown et al., Meth. Enzymol. 185:527 (1990); Sambrook et al., 2001, Molecular Cloning, A Laboratory Manual, Third Edition, Cold Spring Harbor Laboratory Press, N.Y.

In one embodiment, the composition of the invention is formulated for injection.

The pharmaceutical composition of the invention may further comprise pharmaceutically acceptable carriers. Any of the conventional procedures in the pharmaceutical field may be used to prepare oral formulations such as tablets, capsules, pills, granules, suspensions and solutions; injection formulations such as solutions, suspensions, or dried powders that may be mixed with distilled water before injection; locally-applicable formulations such as ointments, creams and lotions; and other formulations.

Carriers generally used in the pharmaceutical field may be employed in the composition of the present invention. For example, orally-administered formulations may include binders, emulsifiers, disintegrating agents, excipients, solubilizing agents, dispersing agents, stabilizing agents, suspending agents, coloring agents or spicery. Injection formulations may comprise preservatives, unagonizing agents, solubilizing agents or stabilizing agents. Preparations for local administration may contain bases, excipients, lubricants or preservatives. Any of the suitable formulations known in the art (Remington's Pharmaceutical Science (18th edition), Mack Publishing Company, Eaton PA) may be used in the present invention.

The pharmaceutical composition of the invention can be clinically administered as various oral and parenteral formulations. A suitable formulation may be prepared using such excipients as additives, enhancers, binders, wetting agents, disintegrating agents and surfactants, or diluents. Solid formulations for oral administration include pills, tablets, dusting powder, granules and capsules. Those solid formulations may be prepared by mixing one or more excipients, e.g., starch, calcium carbonate, sucrose, lactose and gelatin with dibenzylbuthyllacton lignan derivatives. Also, lubricants such as magnesium stearate and talc may be included in the formulation. Liquid formulations for oral administration include suspension, solution, emulsion and syrup. Those formulations may contain wetting agents, sweeteners, aromatics and preservatives, in addition to general simple diluents such as water and liquid paraffin. Formulations for parenteral administration include sterilized aqueous solution, suspension, emulsion, freeze-dried alternative treatment and suppositories. Water-insoluble excipients and suspending agents comprise vegetable fats such as propylene glycol, polyethylene glycol and olive oil, and injectable esters such as ethyl oleate. Witepsol®, Macrogol®, Tween® 61, cacao fats, laurin fats and glycerogelatins may be used as bases of suppositories.

The following Examples are given for the purpose of illustration only, and are not intended to limit the scope of the invention.

### Example 1: Construction of plasmids

The pCK vector can drive gene expression from the human cytomegalovirus (HCMV) promoter, and has been described previously (Lee et al., Biochem. Biophys. Res. Commun. 272:230 (2000); WO 2000/040737).

pCK-VEGF165 was constructed by inserting VEGF165 cDNA into the pCK vector, and has been described previously (Lee et al., Biochem. Biophys. Res. Commun. 272:230 (2000); WO 2000/040737).

pCK-cHGF contains the cDNA encoding HGF₇₂₈ under the control of an HCMV promoter, and has been described previously (US 2005/0079581).

pCK-dHGF contains the cDNA encoding HGF₇₂₃ under the control of HCMV promoter, and has been described previously (PCT/KR03/00548).

pCK-HGF-X7 contains a hybrid HGF cDNA (SEQ ID NO: 9) that was designed to express 2 isoforms of HGF simultaneously inserted into the pCK vector, and has been described previously (US 2005/0079581).

### Example 2: Effect of HGF on cell migration and proliferation

The objective of this study was to evaluate the effect of HGF on cell migration and proliferation *in vitro.*

### 1. Materials and Methods

### (1) Preparation of HGF protein

pCK-HGF-X7 were transfected into 293T cells using FuGENE6™ (Roche Diagnostics, Germany). As controls, pCK, pCK-cHGF and pCK-dHGF were used. Two days after transfection, the culture supernatants containing HGF protein were obtained and the amount of HGF was measured by human HGF ELISA (R&D Systems, MN, USA), according to the manufacturer's recommendations.

### (2) Cell migration assay

The effect of HGF on migration of the human umbilical vein endothelial cell (HUVEC, Agiolab Co., Ltd., AL01-0122S), mouse skeletal myoblast cell (C2C12, ATCC No. CRL-1772) and rat cardiomyoblast cell (H9C2, ATCC No. CRL-1446) was evaluated in a modified Boyden chamber assay. The inserts of 24 transwell cell culture chamber (Corning, NY, US) having porous polycarbonate filters (8-µm pore size) were coated with 1% gelatin in PBS. HUVEC (n = 15) suspended in M199 medium supplemented with 1% of FBS, and C2C12 (n = 10) or H9C2 (n = 10) cells suspended in DMEM medium supplemented with 3% of FBS respectively, were added to the inserts at 1 x 10⁴ cell per well. Test substances (supernatants from the 293T cells transfected with pCK, pCK-cHGF, pCK-dHGF or pCK-HGF-X7) were diluted to the final HGF concentration of 50 ng/ml in M199 or DMEM supplemented with 1% or 3% of FBS respectively, and 600 µl of the diluted test substances was placed in the lower chamber. Cells were allowed to migrate for 3 h at 37° C in a CO₂ incubator, then the inserts were raised, rinsed with PBS, fixed with 4% formaldehyde for 10 min, and stained with 0.2% crystal violet. Cell migration was quantified by counting cells located on the opposite side of the inserts. Cells were counted from 5 high-power fields (x 200) in each insert. The image was analyzed using Image-Pro® plus (Media Cybernetics, US).

### (3) Cell proliferation assay

The effect of HGF on proliferation of the HUVEC cells was evaluated using [³H]thymidine incorporation assay. HUVEC cells (n = 10) suspended in M199 medium supplemented with 1% of FBS were plated to a 96 well plate at 5 x 10³ cells per well. Ten nanograms of HGF protein (supernatants from the 293T cells transfected with pCK, pCK-cHGF, pCK-dHGF or pCK-HGF-X7) were added to the cells. Cells were allowed to proliferate for 48 hours at 37° C in a CO₂ incubator. Afterwards, 1 µCi of [³H]thymidine was added to each well and cells were incubated for another 16 hours at 37° C. The cells were harvested and the [³H]thymidine incorporation was measured using a liquid scintillation counter (Wallac, Turku, Finland).

### 2. Results and Discussion

To explore the biological outcomes of two HGF protein isoforms, their effect on cell migration and proliferation was examined. These assays were performed using the supernatants from 293T cells transfected with each expression vector as described in Materials and Methods. In all experiments, the identical amount of HGF protein was used.

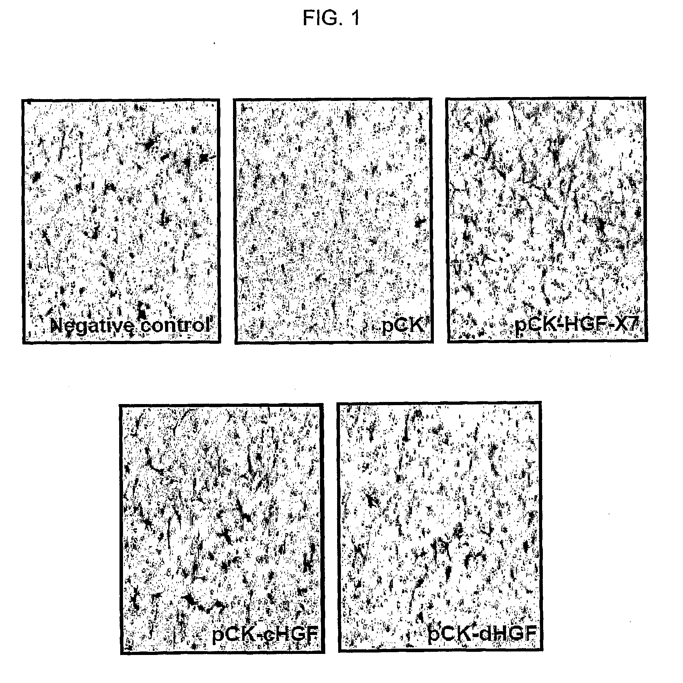

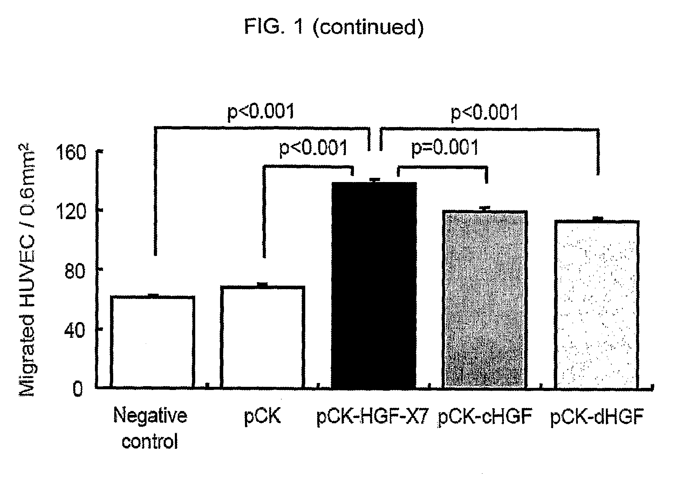

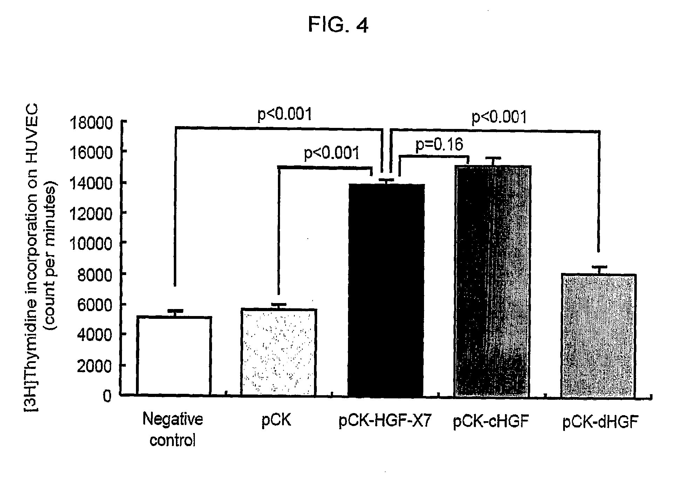

As shown in Figures 1, 2 and 3, the presence of two HGF isoforms produced from pCK-HGF-X7 more efficiently induced migration of HUVEC, C2C12 and H9C2 cells, respectively, as compared to when only one isoform was present. The presence of two HGF isoforms also promoted effective proliferation of HUVEC cells (Figure 4). The supernatant produced from pCK-HGF-X7 transfected cells more efficiently induced HUVEC cell proliferation than the supernatant produced from pCK-dHGF transfected cells. These results show that the combined effects of two isoforms of HGF produced more potent migration and proliferation activities in endothelial cells, and more potent migration activities in skeletal and cardiac myoblast cells.

Vascular endothelial cell migration is strongly associated with angiogenesis, and migration of myocardial and skeletal muscle precursor cells is a crucial step in muscle development and post-lesional muscle regeneration. Thus, these results show that administration of both isoforms of HGF may provide a more effective way to induce neovascularization and regeneration of ischemic tissues. Vascular endothelial cell migration and proliferation are also the natural process associated with vessel wall formation. Therefore, these results also show that administration of both isoforms of HGF can promote re-enothelialization of blood vessel walls.

### Example 3: Efficacy evaluation of HGF in rat ischemic heart disease model

The objective of this study was to evaluate the cardio-protective effect of intramyocardial injection of HGF in a rat ischemic heart disease model. The experimental procedure is given in Figure 5.

### 1. Materials and Methods

### (1) Animals

Thirty eight Sprague-Dawley rats (male, 12 weeks of age, 350 to 400 g, SLC) were provided with food and water ad libitum upon arrival, and provided 7 days of rest before being subjected to the surgery.

### (2) Myocardial infarction model

For the analysis of pharmacological efficacy of HGF in the present study, a rat ischemic heart disease model, one of the widely used pathologic models for CAD, was employed. The rats were anesthetized with an intramuscular injection of Xylazine (5 mg/kg) followed by Ketamine (50 mg/kg). After sterilization with 95% alcohol and iodine, the chest was covered with a sterile surgical cloth and just the incision area was exposed, thereby providing a completely sterile condition for the surgery. Endotracheal intubation was performed via the orotracheal route. During the operation, positive pressure ventilation was maintained. Electrocardiograms and oxygen saturation were monitored continuously. Mid thoracotomy was performed. After opening the pericardium followed by exploration of the anterial wall of the left ventricle, the proximal one-third of the left anterior descending coronary artery (LAD) was ligated using 6-0 polypropylene sutures buttressed with a small piece of Nelaton (5Fr). ST-segment elevation on the monitored electrocardiogram was confirmed. After LAD ligation for 60 minutes, the rat ischemic myocardium was reperfused. The pericardium and thoracotomy wounds were closed. A single chest tube connected to mid wall suction was removed after enough spontaneous respiration returned and the endotracheal tube was removed. Then the incision was checked for the presence of hemorrhage. After the bleeding was controlled, the incised muscle, fascia, and skin were sutured. After the surgery, gentamicin (3 mg/kg/day) was intramuscularly administered for 3 days to prevent infection. Transthoracic echocardiogram was performed 28 days after the surgery to confirm the induction of myocardial infarction in rats.

### (3) Efficacy evaluation study design

The efficacy of HGF was tested by directly injecting the plasmids containing the HGF gene into the ischemic heart muscle and observing the cardio-protective effect physiologically and anatomically. The plasmids containing the HGF gene were immediately administered after the induction of ischemia (Day 0). Each animal was intramyocardially injected with a total dose of 250 µg pCK-cHGF (n=12) or pCK-HGF-X7 (n=10). For the negative and positive controls, the same amount of pCK vector lacking the HGF coding sequence (n=7), and pCK-VEGF165 (n=9) were injected. The improvements in the physiological function of heart were assessed by transthoracic echocardiography on Day 1, 14, 28 and 56 after the DNA injection. The level of angiogenesis and anti-fibrosis were measured after the autopsy.

### (4) Cardio-physiological analysis

On Day 1, left ventricular ejection fraction and systolic interventricular septum were measured using transthoracic echocardiography. The values obtained at Day 1 were set as baseline values. On Days 14, 28 and 56, echocardiography was performed again. The values obtained at Days 1, 14, 28 and 56 were compared between the pCK, pCK-HGF-X7, pCK-cHGF, and pCK-VEGF165 groups. In addition, tissue slices were taken from ischemic hearts to analyze changes in the capillary density and fibrosis in the left ventricle.

### (5) Capillary density analysis

On Day 56, myocardial tissues were obtained from the ischemic heart and fixed in 10% formalin solution for 2 days, then embedded in paraffin. A few serial sections were prepared from each specimen. On the tissue sections, endothelial cells of the capillaries were identified by staining with CD31 antibody. Capillary density was quantitatively analyzed under a microscope at 400x magnification and presented as the number of capillaries per 0.15 mm² (Image-Pro® plus, Version 4.1, Media Cybernetics, Bethesda, Maryland, USA). The value was then compared between the treatment groups.

### (6) Anti-fibrosis analysis

On Day 56, myocardium tissues were obtained from the ischemic heart and fixed in 10% formalin solution for 2 days and then embedded in paraffin. A few serial sections were prepared from each specimen and stained with Trichrom to assess the collagen content. Fibrotic area in the left ventricle was quantitatively analyzed under a microscope at 8x magnification. The value was then compared between the treatment groups.

### (7) Statistics

Results were presented as the mean ± SEM and analyzed using SPSS (version 10.0, SPSS. Inc, Chicago, IL, USA). The statistical analysis of all data was performed using one-way ANOVA and subsequent LSD's test or Tukey's test to determine the significance of differences in multiple comparisons. P values less than 0.05 were considered significant.

### 2. Results

### (1) Induction of the left ventricular myocardial infarction in rat

Transthoracic echocardiogram was performed 28 days after the induction of the myocardial infarction to confirm the animal disease model. It was observed that the physiological function of the left ventricle significantly decreased after the surgical induction of myocardial ischemia. Also, myocardial fibrosis was observed in the anterolateral wall of the left ventricle.

### (2) The effect of HGF on the function of the left ventricle

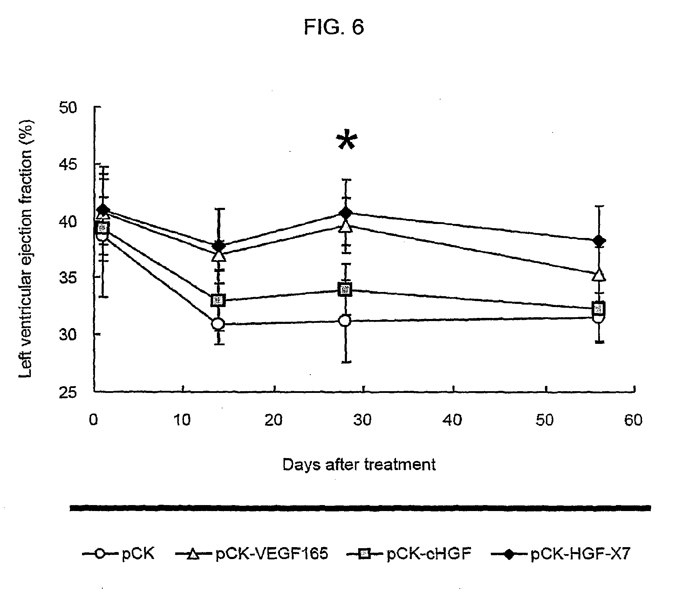

The changes in left ventricular ejection fraction (LVEF) after the DNA injection were compared between treatment groups. On Days 1 and 14, there was no statistically significant difference in the LVEF between groups. However, by 28 days after the intra-myocardial DNA treatment, the value of the LVEF was statistically significantly higher in the pCK-HGF-X7 treatment group (40.77 ± 2.92%) as compared with the pCK group (31.24 ± 3.58%, *p*=0.028) or the pCK-cHGF group (33.99 ± 2.26%, *p* = 0.069). The value of the LVEF in the pCK-VEGF165 group (39.63 ± 2.44%) seemed to be higher than the pCK group (p = 0.056) or the pCK-cHGF (p = 0.138) group, but the difference was not statistically significant. A similar pattern was observed on Day 56 after the treatment (Figure 6).

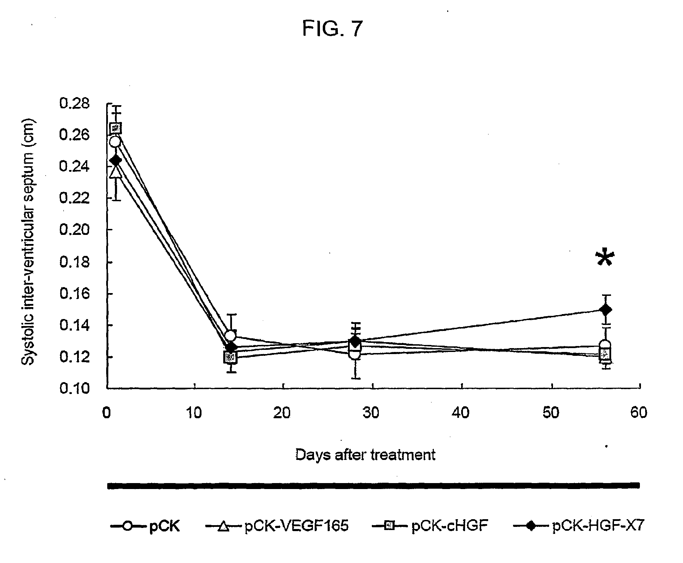

The changes in the systolic inter-ventricular septum (IVS) after the treatment of DNA were also compared. Systolic IVS was increased significantly only in the pCK-HGF-X7-treated group; on Day 56, the IVS of the pCK-HGF-X7 treated group was considerably higher than the pCK (p = 0.061), pCK-VEGF165 (*p* = 0.012) or the pCK-cHGF (p = 0.011)-treated group (Figure 7).

### (3) The effect of HGF on capillary density

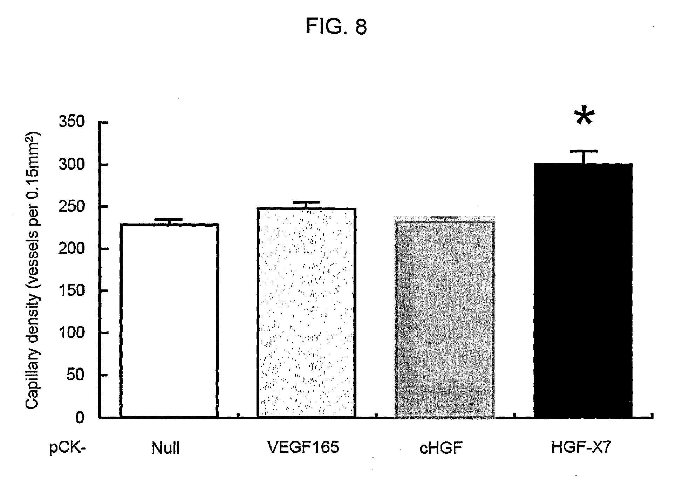

On Day 56, capillary density in the myocardium tissues of the ischemic border area was 300.00 ± 14.71 per 0.15 mm² for the pCK-HGF-X7 treatment group. This capillary density was significantly higher than 227.54 ± 6.16 per 0.15 mm² of the pCK group (p < 0.001), 247.38 ± 7.52 per 0.15 mm² of the pCK-VEGF165 group (p = 0.001) or 231.35 ± 5.55 per 0.15 mm² of the pCK-cHGF group (p < 0.001) (Figure 8).

### (4) The effect of HGF on myocardial fibrosis

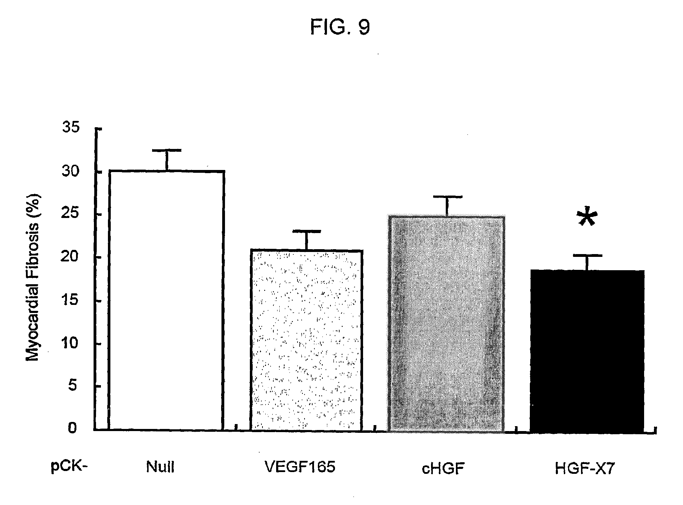

On Day 56, the extent of fibrosis in the left ventricle was 18.88 ± 1.81% for the pCK-HGF-X7 treatment group. This percent fibrosis was significantly lower than the 30.20 ± 2.35% of the pCK group (p = 0.009). The extent of fibrosis in the pCK-VEGF165 group (20.96 ± 2.25%) was also lower than that of the pCK group (p = 0.049), though the difference was statistically less significant as compared with the PCK-HGF-X7 group. But the extent of fibrosis in the pCK-cHGF group (25.02 ± 2.49%) was not considerably lower than that of the pCK treatment group (p = 0.411) (Figure 9).

### 3. Discussion

The therapeutic potential of HGF was evaluated in the rat ischemic heart disease model, which is a well known animal model for CAD. On day 0, heart ischemia was generated by surgical procedure and a total of 250 µg of plasmids containing the HGF gene or control DNAs was injected into the ischemic myocardium. The effects of HGF were evaluated by echocardiographical and/or histological analysis. The function of the ischemic heart was significantly improved in the pCK-HGF-X7 treatment group. The pCK-cHGF group did not show any significant improvement in heart function, even though pCK-cHGF expresses one isoform of HGF protein.

### Example 4: Efficacy evaluation of pCK-HGF-X7 in human clinical trial

The efficacy of pCK-HGF-X7 was evaluated in a human clinical trial. Two subjects undergoing coronary artery bypass graft (CABG) were injected with 0.5 mg of pCK-HGF-X7.

### 1. Methods

### (1) Subjects

The subjects were included in the trial when they met the following criteria; 1) aged 19 to 75 years, 2) having reversible perfusion defect (more than 7 percent of difference between rest and stress perfusion) by the assessment of MIBI-SPECT, 3) to be estimated as still having incomplete revascularization area after CABG or to be estimated as having myocardial perfusion territory which is not suitable for CABG.

The subjects were excluded if they had a previous history or current evidence of 1) malignancy, 2) uncontrolled ventricular arrhythmia, 3) advanced heart failure or evidence of left ventricular dysfunction above Killip class II and having left ventricular ejection fraction <25% by transthoracic 2D echocardiography, 4) severe infectious disease, 5) uncontrolled blood disorder, 6) valvular heart disease and needing debulking surgery of left ventricular, 6) proliferative retinopathy, 7) stroke, 8) uncontrolled essential hypertension by the assessment of JNC II, 9) severe liver and kidney disease, or 10) CABG previously.

The protocol was approved by the Institutional Review Board of Seoul National University Hospital, as well as by the Korean Food and Drug Administration.

### (2) MIBI-SPECT myocardial perfusion study

^{99m}Tc-MIBI gated SPECT (Vertex EPIC, ADAC Labs, CA., USA) at rest and after pharmacological stress with adenosine was performed in all patients before pCK-HGF-X7 treatment and 3 and 6 months after pCK-HGF-X7 treatment. The SPECT images were constructed by electrocardiography gating, and analyzed with a semi-quantitative 20-segment model using an auto-quantitation program (AutoQUANT, ADAC Labs, CA., USA).

Seven percent of difference between rest and stress perfusion scores under SPECT is the bottom line of reversible perfusion defect in myocardial ischemia. Thus, ≥ 7% of difference between rest and stress perfusion indicates that a myocardium has reversible perfusion defect and < 7% of difference indicates that the myocardium has normal perfusion.

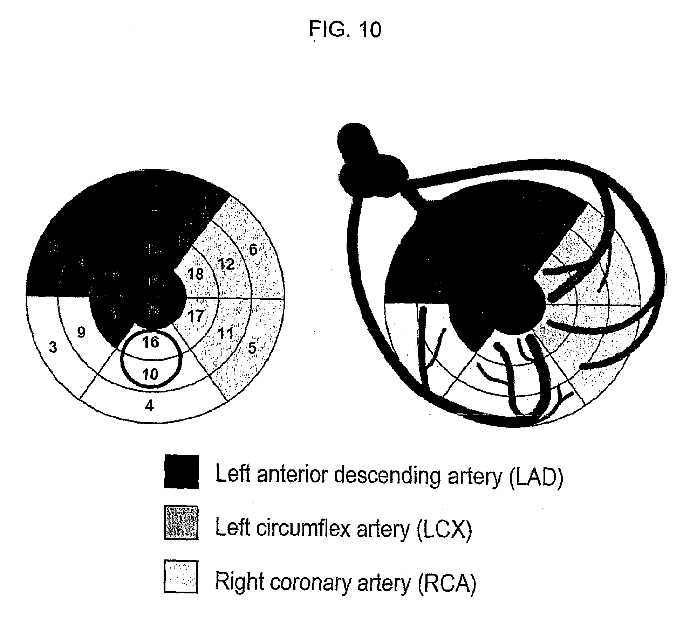

The scores of the rest and stress perfusion were obtained from segment number 10 and 16 of the SPECT bull's-eye image, and the difference of scores was assessed on screening period, 3 and 6 months after the pCK-HGF-X7 injection (Figure 10).

### (3) Intramyocardial pCK-HGF-X7 injection under CABG

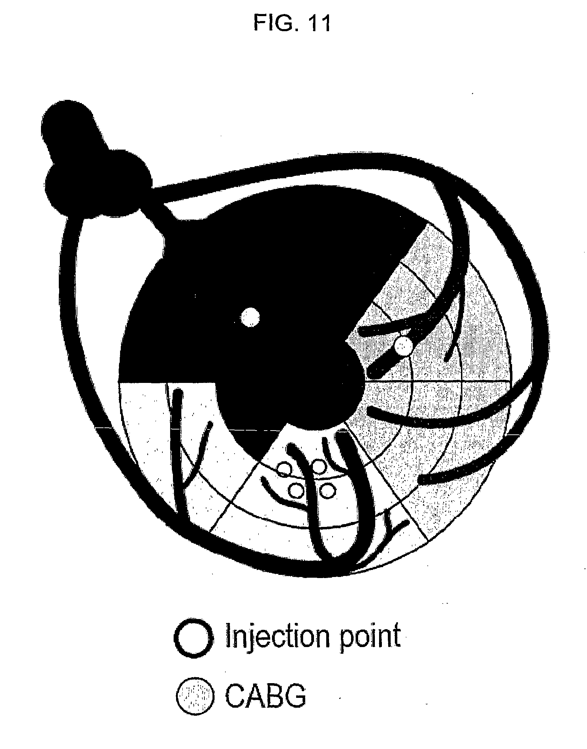

Coronary artery bypass grafting of the left anterior descending coronary artery and circumflex coronary artery was completed through a standard median sternotomy. 0.5 mg of pCK-HGF-X7 (0.125 mg/0.25 mL/injection; 4 sites/patient) was administered by intramyocardial injection into both sides of the posterior descending artery which was not suitable for CABG when assessed by MIBI-SPECT, although having decreased perfusion. The segment numbers administered with pCK-HGF-X7 were 10 and 16 of the bull's eye image obtained from MIBI-SPECT (Figure 11).

### 2. Results and Discussion

### (1) The effect of pCK-HGF-X7 on the myocardial perfusion under MIBI-SPECT

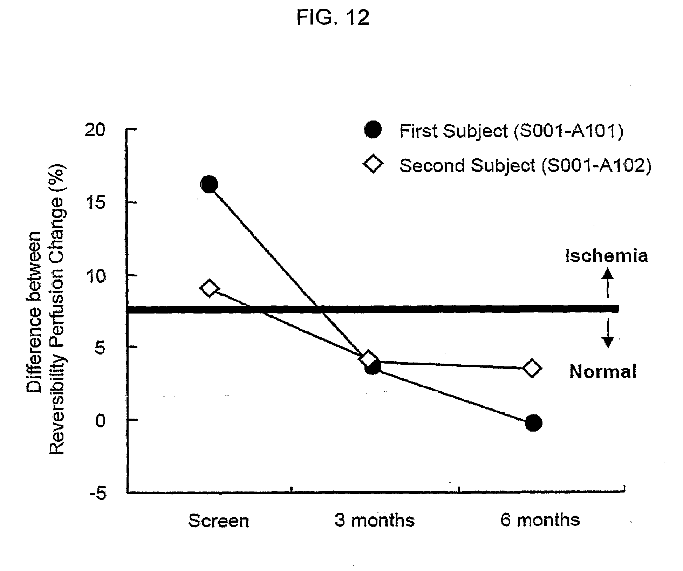

The difference between the rest and stress perfusion scores under SPECT before and after the pCK-HGF-X7 injection was compared. In the first subject, the average difference between the rest and stress perfusion scores before the pCK-HGF-X7 injection was 16%. At 3 and 6 months after pCK-HGF-X7 injection, the average difference between the rest and stress perfusion scores in the injected area (segment number 10 and 16) was 3.5% and 0.5% which was significantly different from the baseline value. In the second subject, the average difference between the rest and stress perfusion scores before the pCK-HGF-X7 injection was 9%. At 3 and 6 months after pCK-HGF-X7 injection, the average difference between the rest and stress perfusion scores in the injected area (segment number 10 and 16) was 4% and 3.5% which was significantly different from the baseline value (Figure 12).

In conclusion, the inhamyocardial injection of pCK-HGF-X7 to affected subjects can change the status of myocardial perfusion from reversible defect to normal. These results indicate that the administration of pCK-HGF-X7 can significantly improve myocardial perfusion.

### Example 5: Efficacy evaluation of pCK-HGF-X7 in porcine ameroid ischemia model

The objective of this study was to evaluate the cardio-protective effect of percutaneous endocardial injection of pCK-HGF-X7 using a catheter under the guidance of cardiac imaging system in a porcine ameroid ischemia model.

### 1. Materials and Methods

### (1) Animal

Neutered Yorkshire domestic pigs (n = 9, male, 20 to 40 Kg) were provided with food and water ad libitum upon arrival, and provided 7 days rest before being subjected to surgery.

### (2) Porcine ameroid ischemia model

The porcine model of chronic myocardial ischemia via ameroid constriction is a well-established, clinically-relevant and accepted preclinical model of chronic myocardial ischemia in the testing of novel angiogenic therapies. This model simulates both the human coronary anatomy, as well as the extent of vessel formation in response to ischemia in humans. In addition, this porcine model is well established in the testing of cardiovascular medical devices, due to similarities in size and anatomy to the human cardiovascular system.

The pigs were subjected to a surgical implantation of an ameroid constrictor to the proximal left circumflex to create chronic ischemia. Pigs were sedated via an intramuscular injection of Telazol® 4-6mg/kg and Atropine Sulfate 0.02 to 0.05 mg/kg. Then Isoflurane was administered via facemask to induce general anesthesia for surgical interventions. The pigs were intubated and appropriately prepped for surgery. The pigs were placed in right lateral recumbency. An intravenous Plasmalyte and Lidocaine drip maintained hydration throughout surgery and control any arrhythmias. Scrub solution was applied to sterilely prep the surgical site, the chest was covered with sterile cloth drapes and the entire animal was covered with a body drape. Pancuronium bromide was administered intravenously for muscle relaxation after a deep plane of anesthesia had been established. A 20-cm incision was made in the left chest at the 5th intercostal space. The ribs were retracted followed by the lungs, and wrapped in gauze sponges soaked with saline. The pericardium was opened just distal to the phrenic nerve by a horizontal incision and the heart suspended in a pericardial cradle. The circumflex coronary artery (LCX) was dissected for a distance of about 0.5 cm just proximal to the 1st marginal branch and an ameroid constrictor of the appropriate size for the artery is placed around it. All collateral materials that would impact the LCX bed were permanently ligated. Lidocaine bolus was administered if necessary for control of arrhythmias. The pericardium was not routinely sutured closed, but was used as an aid for securing the ameroid in place on the artery. Chromic gut (PDS II) suture in a continuous pattern was used to close the muscle layers. Braided Dexon™ suture was used subcutaneously. Surgical staples were used to close the skin. Then all pigs received Enrofloxacin (5.0mg/kg/day) intramuscularly for 3 days to prevent infection.

### (3) Efficacy evaluation study design

Four weeks after ameroid implantation, the pigs were randomized to receive 1 mg of pCK-HGF-X7 [low dose group: 1 mg/2 ml (n=3)], 4 mg of pCK-HGF-X7 [high dose group: 4 mg/8 ml (n=3)] or control article consisting of the same excipient buffers used with pCK-HGF-X7 [vehicle control group: 8 ml (n=3)]. The articles were administered using the NOGA® MyoStar delivery catheter (Biosense Webster, USA) into transendocardial route. Each animal received eight (low dose group: 0.125 mg/0.25 ml/injection site) or sixteen (high dose group: 0.25 mg/0.5 ml/injection site) injection of pCK-HGF-X7 or control article (vehicle control group: 0.5 ml/injection site) into the border zone of viable and ischemic myocardium on lateral and posterior wall. In order to evaluate functional outcome of pCK-HGF-X7 treatment, the myocardial perfusion (κ) at peak stress before and after the DNA treatment was determined as measured by myocardial contrast stress echocardiography.

### 2. Results

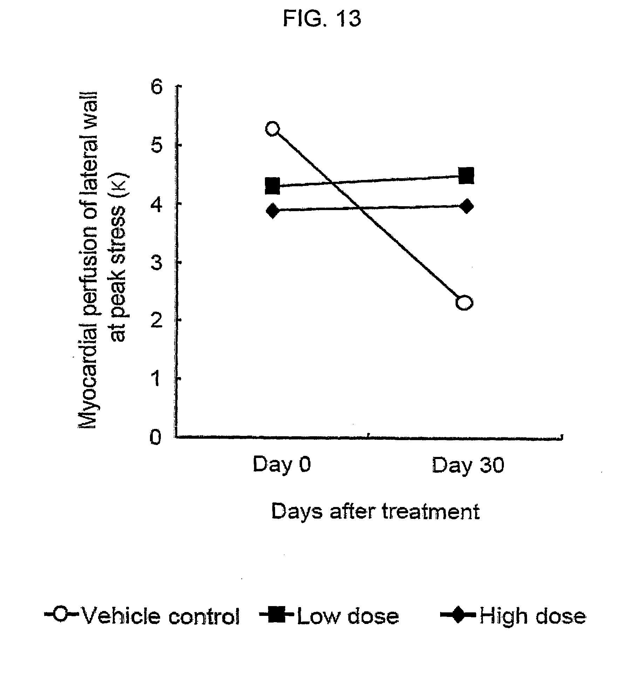

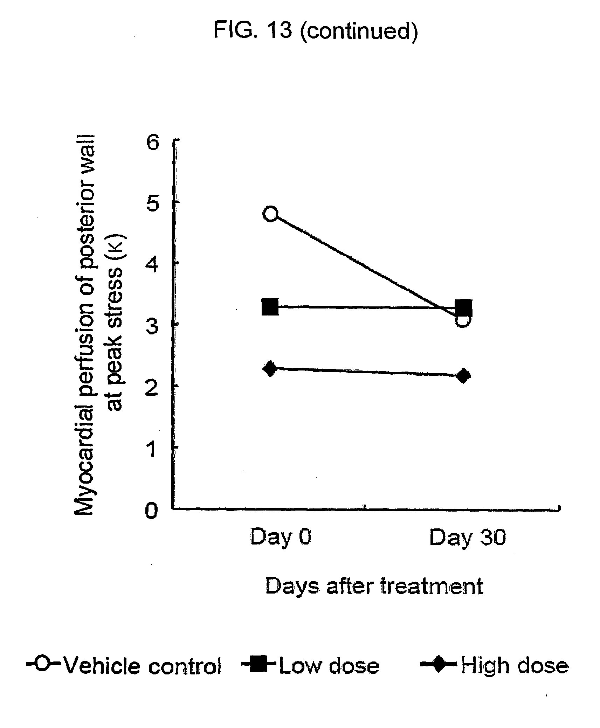

The changes in the myocardial perfusion at peak stress before and after the plasmid DNA injection were compared between treatment groups. In the vehicle control group, Day 30 showed a tendency to decreased perfusion as compare to that on Day 0. But two pCK-HGF-X7 groups on Day 30 showed a tendency to preservation of perfusions as compare to that at Day 0 (Figure 13). These results suggested that transendocardial transfer of pCK-HGF-X7 could protect against the decrease of myocardial perfusion induced by myocardial ischemia.

These results show that HGF can be delivered using a catheter. The percutaneous transendocardial injection catheter has been used for endocardial injection of various drugs, e.g., stem cells, adenovirus and naked DNA under guidance of the electromechanical cardiac navigation system in clinical trials. These results indicate that pCK-HGF-X7 plasmid DNA can be safely administered into the myocardium having perfusion decrease as a percutaneous transendocardial injection using a catheter under the electromechanical guidance. Thus, catheter injection systems to transfer pCK-HGF-X7 to the heart can be used in patients having ischemic cardiac tissue such as stable, unstable angina pectoris or acute, chronic myocardial infarction.

### Example 6: Delivery of HGF expressing cells

HGF can be delivered in the form of cells comprising the pCK-HGF-X7. The mesenchymal stem cells are collected from a subject. The source of mesenchymal stem cells can be marrow aspirates or mobilized peripheral blood. The harvested mesenchymal stem cells are cultured and transfected with pCK-HGF-X7 using liposome. Cells are then harvested, washed with saline, re-suspended in the infusion solution, and infused into the subject. The mesenchymal stem cells transfected with pCK-HGF-X7 can be administered into the ischemic and infracted cardiac tissue i) as a intramyocardial injection using a syringe, or ii) as a percutaneous transendocardial injection using a catheter under the electromechanical guidance.

### Example 7: pCK-HGF-X7-eluting stent

This example demonstrates the production of plasmid-eluting stents.

### A. Production of plasmid-eluting stainless steel stent

### 1. Materials and Methods

### (1) Stainless steel stent

Stainless steel stents (SS stent, Liberté®, 3.0 mm x 20 mm) were purchased from Boston Scientific (USA).

### (2) Production of pCK-HGF-X7-eluting SS stent

### a. Production of non-polymer based, pCK-HGF-X7-eluting SS stent

To wash the surface of SS stent struts, the stents were sonicated three times in ethanol for 3 minutes (Vibra-Cell™, Sonics & Materials INC., Switzland) and dried for 30 minutes at 37° C. Then the stents were immersed one time in 5 mg/ml of pCK-HGF-X7 for 5 minutes and dried for 30 minutes at 37° C.

### b. Production of polymer based, pCK-HGF-X7-eluting SS stent

To make the polymer based SS stents, the stents were immersed one time in 5 mg/ml of phosphorylcholine (PC) polymer (CM5208, Vertellus Specilities INC., UK) in ethanol. Then, the PC polymer based-SS stents were immersed one time in 5 mg/ml of pCK-HGF-X7 for 5 minutes and dried for 20 minutes at 37° C.

### (3) Quantitation of pCK-HGF-X7 eluted from SS stent

In order to analyze the quantity of pCK-HGF-X7 that had been loaded onto the SS stents, the large volume of solution removed and replenished (0.8 ml out of 1 ml) was chosen to quantify pCK-HGF-X7 elution in a quick and efficient fashion.

pCK-HGF-X7 coated SS stents were individually immersed in cryotubes containing 1 ml of normal saline. The tubes containing stents were placed onto the roller mixer (HIP-RMF40, Hyunil LAB-MATE, Korea) for 60 minutes under the condition of 40 rotations a minutes. pCK-HGF-X7 eluates were collected at 1, 5, 10, 20 and 40 minutes. At each time point, 0.8 ml of the solution was withdrawn for UV analysis and 0.8 ml of fresh normal saline was added back to the cryotube to maintain the total volume of the solution. The cryotube was then placed onto the roller mixer to continue the experiment. Concentration of the withdrawn pCK-HGF-X7 eluates obtained from each time point was measured at 260 nm using Ultraspec 3000 (Amersham Pharmacia Biotech., Sweden). As a negative control, the SS stent without pCK-HGF-X7 was also tested using the above quantitation method.

### 2. Results

The results are given in Tables 1 and 2. Almost 78 µg of pCK-HGF-X7 was eluted from the non-polymer based SS stent at one (1) minute and 115 µg of it was eluted at 40 minutes. Also, almost 43 µg of pCK-HGF-X7 was eluted from the polymer based SS stent at 60 minutes. These results indicate that the plasmid can be coated on both non-polymer and polymer based SS stents, and that the coated plasmid can be eluted from SS stent. Therefore, it was concluded that the plasmid-eluting SS stent was successfully produced.

**Table 1: Quantitation of pCK-HGF-X7 eluted from the non-polymer based SS stent**

| Extraction Time (Min) | Cumulative pCK-HGF-X7 extracted | | | |
|---|---|---|---|---|
| | Negative Control | | Non-polymer based SS stent | |
| | µg of pCK-HGF-X7 | Relative Recovery (%) | µg of pCK-HGF-X7 | Relative Recovery (%) |
| 1 | 0.53 ± 0.14 | 0 | 77.98 ± 29.29 | 67.88 |
| 5 | 0.87 ± 0.03 | 0 | 108.97 ± 13.20 | 94.86 |
| 10 | 1.41 ± 0.18 | 0 | 112.48 ± 14.23 | 97.92 |
| 20 | 1.55 ± 0.11 | 0 | 114.15 ± 13.76 | 99.38 |
| 40 | 1.83 ± 0.24 | 0 | 114.87 ± 13.84 | 100.00 |

**Table 2: Quantitation of pCK-HGF-X7 eluted from the polymer based SS stent**

| Extraction Time (Min) | Cumulative pCK-HGF-X7 extracted | | | |
|---|---|---|---|---|
| | Negative Control | | Polymer based SS stent | |
| | µg of pCK-HGF-X7 | Relative Recovery (%) | µg of pCK-HGF-X7 | Relative Recovery (%) |
| 10 | 1.85 ± 0.07 | 0 | 39.74 ± 9.27 | 84.45 |
| 20 | 1.37 ± 0.03 | 0 | 41.64 ± 9.14 | 88.49 |
| 30 | 1.35 ± 0.00 | 0 | 43.35 ± 9.12 | 92.12 |
| 40 | 1.41 ± 0.07 | 0 | 44.55 ± 9.03 | 94.69 |
| 50 | 1.00 ± 0.08 | 0 | 45.82 ± 8.89 | 97.37 |
| 60 | 0.96 ± 0.05 | 0 | 47.05 ± 9.02 | 100.00 |

### B. Production of plasmid-eluting cobalt chromium stent

### 1. Materials and Methods

### (1) Cobalt Chromium Stent

Cobalt Chromium stents (Co-Cr stent, ARTHOSPico, 2.75 mm x 12 mm) were purchased from AMG international (Germany).

### (2) Production of pCK-HGF-X7-eluting Co-Cr stent

### a. Production of non-polymer based, pCK-HGF-X7-eluting Co-Cr stent

To wash the surface of SS stent struts, the stents were sonicated three times in ethanol for 3 minutes and dried for 30 minutes at 37° C. Then the stents were immersed one time in 5 mg/ml of pCK-HGF-X7 for 5 minutes and dried for 30 minutes at 37° C.

### b. Production of polymer based, pCK-HGF-X7-eluting Co-Cr stent

To make the polymer based Co-Cr stents, the stents were immersed one time in 5 mg/ml of phosphorylcholine (PC) polymer (CM5208, Vertellus Specilities INC., UK) in ethanol. Then, the PC polymer based Co-Cr stents were immersed three times in 5 mg/ml of pCK-HGF-X7 for 5 minutes and dried for 10 minutes at 37° C.

### (3) Quantitation of pCK-HGF-X7 eluted from Co-Cr stent

In order to analyze the quantity of pCK-HGF-X7 that had been loaded onto the Co-Cr stents, the large volume of solution removed and replenished (0.8 ml out of 1 ml) was chosen to quantify pCK-HGF-X7 elution in a quick and efficient fashion.

pCK-HGF-X7 coated CO-Cr stents were added individually to cryotubes containing 1 ml of normal saline. The tubes were placed onto the roller mixer (HIP-RMF40, Hyunil LAB-MATE, Korea) for 60 minutes under the condition of 40 rotations a minutes. pCK-HGF-X7 eluates were collected at 10, 20, 30, 40, 50 and 60 minutes. At each time point, 0.8 ml of the solution was withdrawn for UV analysis and 0.8 ml of fresh normal saline was added back to the cryotube to maintain the total volume of the solution. The cryotube was then placed onto the roller mixer to continue the experiment. Concentration of the withdrawn pCK-HGF-X7 eluates obtained from each time point was measured at 260 nm using Ultraspec 3000. As a negative control, the Co-Cr stent without pCK-HGF-X7 was also tested using the above quantitation method.

### 2. Results and Discussion

The results are shown in Tables 3 and 4. Almost 60 µg and 85 µg of pCK-HGF-X7 were eluted at one (1) minute and 20 minutes from the non-polymer based Co-Cr stent, respectively. Also, almost 43 µg of pCk-HGF-X7 was eluted from the polymer based Co-Cr stent at 60 minutes. These results indicate that the plasmid can be coated on both non-polymer and polymer based Co-Cr stents, and that the coated plasmid can be eluted from the Co-Cr stent although the amount of plasmid eluted from the Co-Cr stent is less than that from SS stent. Therefore, it was concluded that the plasmid-eluting Co-Cr stent was successfully produced.

**Table 3: Quantitation of pCK-HGF-X7 eluted from the non-polymer based Co-Cr stent**

| Extraction Time (Min) | Cumulative pCK-HGF-X7 extracted | | | |
|---|---|---|---|---|
| | Negative Control | | Non-polymer based Co-Cr stent | |
| | µg of pCK-HGF-X7 | Relative Recovery (%) | µg of pCK-HGF-X7 | Relative Recovery (%) |
| 1 | 0.53 ± 0.14 | 0 | 60.20 ± 11.18 | 70.48 |
| 5 | 0.87 ± 0.03 | 0 | 80.52 ± 4.13 | 94.28 |
| 10 | 1.41 ± 0.18 | 0 | 83.52 ± 4.10 | 97.79 |
| 20 | 1.55 ± 0.11 | 0 | 84.77 ± 3.99 | 99.25 |
| 40 | 1.83 ± 0.24 | 0 | 85.41 ± 4.11 | 100.00 |

**Table 4: Quantitation of pCK-HGF-X7 eluted from the polymer based Co-Cr stent**

| Extraction Time (Min) | Cumulative pCK-HGF-X7 extracted |
|---|---|
| | Polymer based CO-Cr stent |
| | µg of pCK-HGF-X7 |
| 60 | 21.9 ± 0.00 |

### Example 8: Efficacy evaluation of HGF-X7-eluting stent in rabbit balloon denudation model

The objective of this study was to evaluate the acceleration of re-endothelialization by HGF-X7-eluting stent in a rabbit balloon denudation model.

### 1. Materials and Methods

### (1) Animals

Ten New Zealand white rabbits (male, 3.5 to 4.0 Kg, Doo-Yeol Biotech, Korea) were provided with food and water ad libitum upon arrival, and provided 7 days of rest before being subjected to the stent implantation.

### (2) Rabbit balloon denudation model and stent implantation

The rabbits were anesthetized with an intramuscular injection of Xylazine (5 mg/kg) followed by Ketamine (50 mg/kg). After sterilization with 95% alcohol and iodine, the neck was covered with a sterile surgical cloth and only the incision area was exposed, thereby providing a completely sterile condition for the surgery. After surgical exposure of the external carotid artery, a 5F introducer sheath (Cordis, USA) was advanced into the external carotid artery. A 2.8F micro-catheter was advanced to the proximal portion of the external iliac artery after insertion of a 1.4F guide-wire (Terumo, Japan) into the femoral artery using standard fluoroscopy methods. 1000 U heparins and 0.1 mg of nitroglycerin were administered.

Balloon denudation of the external iliac artery was performed as follows: A 2.5 x 8 mm balloon catheter (GoodMan, Japan) was inserted into the external iliac artery through the guide-wire followed by removal of the micro-catheter from the rabbit. After inflation of the balloon catheter (10 atm), the endothelium of the external iliac artery was denudated at a distance of approximately 1.0 cm by sequential 10 times withdrawal. The balloon catheter for iliac denudation was removed from the rabbit and a new balloon catheter mounted with a pCK-HGF-X7-eluting SS stent (PES) or a bare-metal stent (BMS) was advanced into the external iliac artery which was denudated. Stent implantation was performed for 15 seconds at 12 atm of balloon inflation. PES (n=10) and BMS (n=10) were implanted bilaterally.

### (3) Optical coherent tomography (OCT) analysis

For the optical coherent tomography (OCT) analysis, the Helios occlusion balloon catheter (LightLab, USA) was advanced into the proximal portion of the external iliac artery through the guide-wire, which was then removed from the rabbit. The OCT image wire (LightLab, USA) was then placed 1.5 cm distance from the distal edge of the implanted stent. OCT images were acquired using 10 ml of normal saline flush. After removal of all devices from the rabbit, the external carotid artery was ligated with 3-0 silk suture. The incision was then checked for the presence of a hemorrhage. After the bleeding was controlled, the incised muscle, fascia, and skin were sutured. Gentamicin (3 mg/kg/day) was intramuscularly administered for three days to prevent infection. Also, 32.5 mg of clopidogrel (Sanofi-Aventis, France) and 25 mg of aspirin (Bayer, Germany) were adminstered every day.

### (4) Scanning electron microscopy (SEM)

At Day 14 and 28 after the stent implantation, the animals were sacrificed and the vessels were harvested and fixed with 2.5% glutaldehyde solution for 2 hours. The fixed vessels were washed three times with phosphate buffered saline (PBS) and the post-fixation was performed with 1% OsO₄ solution. The post-fixed vessels were washed three times with PBS and the dehydration procedures were sequentially performed with 60 to 95% ethyl alcohol. Finally, gold coating was performed on the dehydrated vessels.

### (5) Statistics

Results were presented as the mean ± SEM and analyzed using SPSS (version 10.0, SPSS Inc., Chicago, IL, USA). The statistical analysis of all data was performed using Student's t-test. P value less than 0.05 were considered significant.

### 2. Results and Discussion

To evaluate whether the pCK-HGF-X7-eluting stent could accelerate the re-endothelialization process, the changes of intimal dimension and the nature of cells covering the stent were examined by OCT and SEM, respectively.

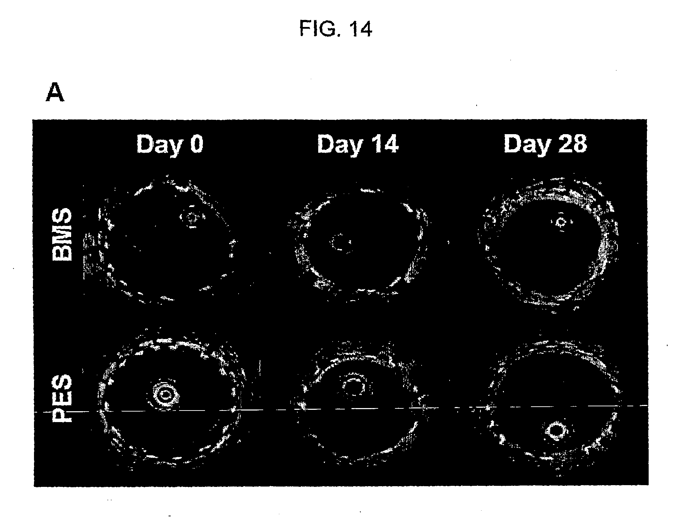

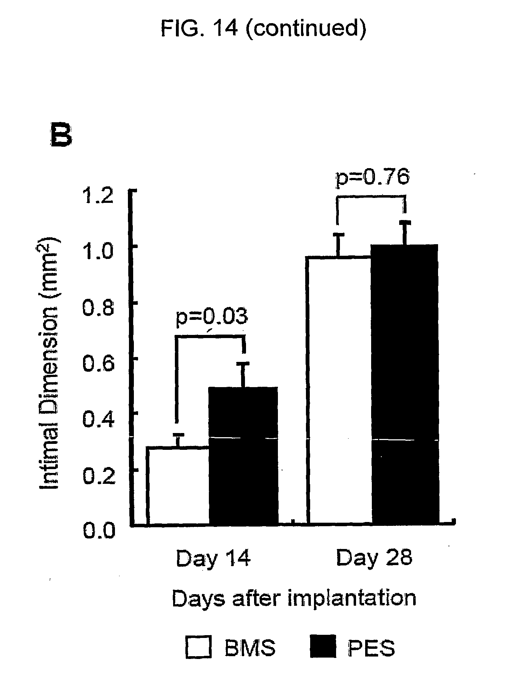

OCT images were obtained at Day 0, 14, and 28 after stent implantation. Nine cross-section images were obtained from each stent. Baseline and follow-up data of OCT are shown in Figure 14. Post-intervention results were similar in both groups (Figure 14A; Day 0). However, the cross-section area of intimal demension (ID-CSA, mm²) of the PES group at Day 14 after stent implantation was significantly extended compared to that of the BMS group (Figure 14B; PES vs BMS, 0.23 ± 0.05 mm² vs 0.48 ± 0.09 mm², *p*=0.03). At Day 28 after stent implantation, the ID-CSAs between the two groups were similar (Figure 14B; PES vs BMS, 0.91 ± 0.08 mm² vs 0.98 ± 0.09 mm², *p*=0.76). These results show that the pCK-HGF-X7-eluting stent enhanced the growth of the cells on the surface of the stent compared with a bare-metal stent.

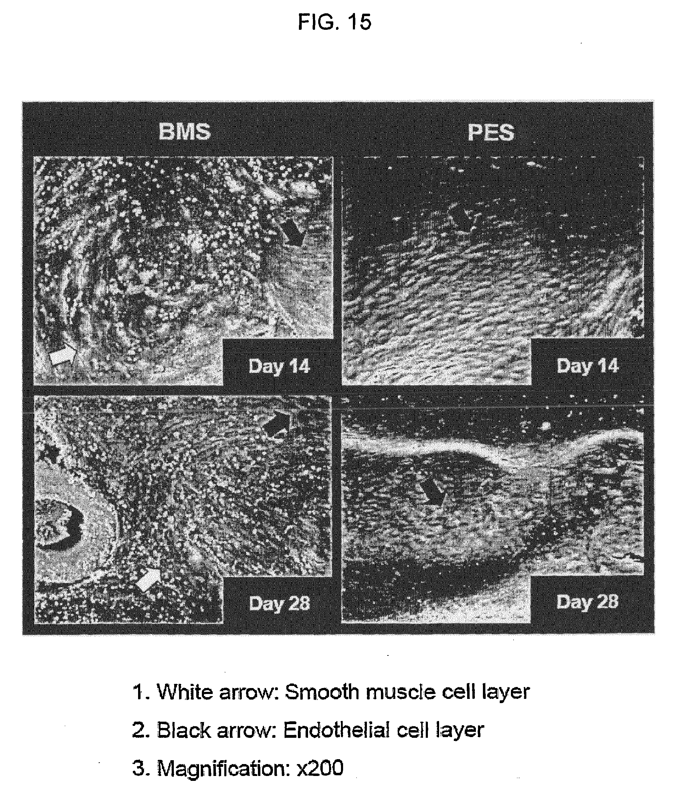

Next, the kind of cells that proliferated on the stent were determined by SEM analysis. SEM was performed at Days 14 and 28. Figure 15 shows that endothelial cells (see black arrow) evenly covered the surface of the stent in the PES group while a mixed neointima composed of smooth muscle cells (see white arrow) and endothelial cells were observed in the BMS group.

These results demonstrate that the pCK-HGF-X7-eluting stent can accelerate re-endothelialization and thus be a useful tool to treat an obscured blood vessel.

The results above show that the presence of the 2 isoforms of HGF (HGF and dHGF) can more effectively induce the growth and migration of endothelial cells *in vitro* than that of HGF or dHGF alone and that the transfer of nucleotide sequences expressing both of the 2 isoforms of HGF (HGF and dHGF) *in vivo* can accelerate the re-endothelialization process of a blood vessel. These results indicate that 2 isoforms of HGF can attenuate restenosis more effectively through rapid re-endothelialization activity than that of a single isoform of HGF.

## Claims

1. A composition for use in treating incomplete revascularisation of the myocardium in a subject who has had a coronary artery bypass graft but there has been incomplete revascularisation of the myocardium, the composition comprising two or more isoforms of HGF or one or more polynucleotides encoding the isoforms as active ingredients, wherein said two or more isoforms of HGF are selected from the group consisting of full length HGF (flHGF), deleted variant HGF (dHGF), NK1, NK2 and NK4.

2. The composition for use of claim 1, wherein the subject is in need of treatment of restenosis.

3. The composition for use of any one of claims 1-2, wherein said two or more isoforms of HGF are administered as polynucleotides encoding the isoforms.

4. The composition for use of any one of claims 1-3, wherein said composition is administered by injection.

5. The composition for use of any one of claims 1-4, wherein said composition is administered by using a delivery device, and preferably wherein said delivery device is a stent.

6. The composition for use of claim 5, wherein said stent is selected from the group consisting of a non-polymer-based stainless steel stent, a polymer-based stainless steel stent, a non-polymer-based cobalt chromium stent, and a polymer-based cobalt chromium stent.

7. The composition for use of claim 5, wherein said composition is eluted from said stent.

8. The composition for use of claim 1, wherein said two or more isoforms of HGF comprise full length HGF (flHGF) and deleted variant HGF (dHGF).

9. The composition for use of claim 8, wherein said two or more isoforms of HGF further comprise NK1.

10. The composition for use of claim 8, wherein said two or more isoforms of HGF consist of flHGF and dHGF.

11. The composition for use of claim 8, wherein said flHGF and dHGF are each at least 80%, preferably at least 90%, or preferably at least 95% identical to the wild-type sequence of human flHGF and human dHGF.

12. The composition for use of claim 11, wherein said flHGF and dHGF are human flHGF and human dHGF.

13. The composition for use of claim 8, wherein said flHGF and said dHGF are encoded by separate polynucleotides or are encoded by the same polynucleotide.

14. The composition for use of claim 1, wherein said one or more polynucleotides are operably linked to a promoter, and preferably a constitutive promoter.

15. The composition for use of claim 1, wherein said one or more polynucleotides are on different vectors.

16. The composition for use of claim 1, wherein said one or more polynucleotides are on the same vector.

17. The composition for use of claim 16, wherein said vector is a viral vector or a plasmid vector, preferably a pCK vector.

18. The composition for use of claim 8, wherein said flHGF and said dHGF are encoded by a hybrid HGF construct comprising HGF exons 1-18 or degenerates thereof which do not alter the encoded amino acid sequence and further comprising an intron between exons 4 and 5, wherein said construct is devoid of other introns between exons other than said intron between exons 4 and 5.

19. The composition for use of claim 18, wherein said intron is an inherent intron, and preferably wherein said hybrid HGF construct comprises the nucleotide sequence of SEQ ID NO: 7.

20. The composition for use of claim 18, wherein said intron is a fragment of an inherent intron, and preferably wherein said hybrid HGF construct comprises the nucleotide sequence of SEQ ID NO: 8, 9 or 10.

21. The composition for use of claim 1, wherein said subject is a human and said isoforms of HGF are administered at a dose of about 1 µg to about 100 mg each.

22. The composition for use of claim 1, wherein said subject is a human and said polynucleotides are administered at a dose of about 1 µg to about 10 mg.

23. A use of a composition comprising two or more isoforms of HGF or one or more polynucleotides encoding the isoforms for the manufacture of a medicament for treating incomplete revascularisation of the myocardium in a subject who has had a coronary artery bypass graft, but there has been incomplete revascularisation of the myocardium, wherein said two or more isoforms are selected from the group consisting of full length HGF (flHGF), deleted variant HGF (dHGF), NK1, NK2 and NK4.

## Patentansprüche

1. Zusammensetzung zur Verwendung bei der Behandlung einer unvollständigen Revaskularisation des Myokards bei einem Patienten, bei dem eine koronare Bypass-Operation durchgeführt wurde, die Revaskularisation des Myokards aber unvollständig war, wobei die Zusammensetzung zwei oder mehr Isoformen von HGF oder ein oder mehr Polynucleotide, die für die Isoformen kodieren, als Wirkstoffe beinhaltet, wobei die zwei oder mehr Isoformen von HGF aus der Gruppe ausgewählt sind, die aus vollständigem HGF (flHGF), deletierter HGF-Variante (dHGF), NK1, NK2 und NK4 besteht.

2. Zusammensetzung zur Verwendung gemäß Anspruch 1, wobei der Patient eine Restenosebehandlung benötigt.

3. Zusammensetzung zur Verwendung gemäß einem der Ansprüche 1-2, wobei die zwei oder mehr Isoformen von HGF als Polynucleotide, die für die Isoformen kodieren, verabreicht werden.

4. Zusammensetzung zur Verwendung gemäß einem der Ansprüche 1-3, wobei die Zusammensetzung mittels Injektion verabreicht wird.

5. Zusammensetzung zur Verwendung gemäß einem der Ansprüche 1-4, wobei die Zusammensetzung unter Verwendung einer Zuführvorrichtung verabreicht wird und wobei die Zuführvorrichtung vorzugsweise ein Stent ist.

6. Zusammensetzung zur Verwendung gemäß Anspruch 5, wobei der Stent aus der Gruppe ausgewählt ist, die aus einem nicht polymerbasierten Edelstahl-Stent, einem polymerbasierten Edelstahl-Stent, einem nicht polymerbasierten Kobalt-Chrom-Stent und einem polymerbasierten Kobalt-Chrom-Stent besteht.

7. Zusammensetzung zur Verwendung gemäß Anspruch 5, wobei die Zusammensetzung aus dem Stent freigesetzt wird.

8. Zusammensetzung zur Verwendung gemäß Anspruch 1, wobei die zwei oder mehr Isoformen von HGF vollständigen HGF (flHGF) und deletierte HGF-Variante (dHGF) beinhalten.

9. Zusammensetzung zur Verwendung gemäß Anspruch 8, wobei die zwei oder mehr Isoformen von HGF ferner NK1 beinhalten.

10. Zusammensetzung zur Verwendung gemäß Anspruch 8, wobei die zwei oder mehr Isoformen von HGF aus flHGF und dHGF bestehen.

11. Zusammensetzung zur Verwendung gemäß Anspruch 8, wobei flHGF und dHGF jeweils zu mindestens 80%, vorzugsweise zu mindestens 90%, oder vorzugsweise zu mindestens 95% identisch mit der Wildtyp-Sequenz von menschlichem flHGF und menschlicher dHGF sind.

12. Zusammensetzung zur Verwendung gemäß Anspruch 11, wobei der flHGF und die dHGF menschlicher flHGF und menschliche dHGF sind.

13. Zusammensetzung zur Verwendung gemäß Anspruch 8, wobei für flHGF und dHGF separate Polynucleotiden kodieren oder die gleichen Polynucleotide kodieren.

14. Zusammensetzung zur Verwendung gemäß Anspruch 1, wobei das eine oder die mehreren Polynucleotide wirkverbunden mit einem Promoter, und vorzugsweise mit einem konstitutiven Promoter, verknüpft sind.

15. Zusammensetzung zur Verwendung gemäß Anspruch 1, wobei sich das eine oder die mehreren Polynucleotide auf verschiedenen Vektoren befinden.

16. Zusammensetzung zur Verwendung gemäß Anspruch 1, wobei sich das eine oder die mehreren Polynucleotide auf dem gleichen Vektor befinden.

17. Zusammensetzung zur Verwendung gemäß Anspruch 16, wobei der Vektor ein viraler Vektor oder ein Plasmidvektor, vorzugsweise ein pCK-Vektor, ist.

18. Zusammensetzung zur Verwendung gemäß Anspruch 8, wobei für flHGF und dHGF ein Hybrid-HGF-Konstrukt kodiert, das HGF-Exons 1-18 oder degenerierte Formen davon beinhaltet, welche die kodierte Aminosäuresequenz nicht verändern, und das ferner ein Intron zwischen Exons 4 und 5 beinhaltet, wobei dem Konstrukt andere Introns zwischen Exons mit Ausnahme des Introns zwischen Exons 4 und 5 fehlen.

19. Zusammensetzung zur Verwendung gemäß Anspruch 18, wobei das Intron ein inhärentes Intron ist und wobei das Hybrid-HGF-Konstrukt vorzugsweise die Nucleotidsequenz von SEQ ID NO: 7 beinhaltet.

20. Zusammensetzung zur Verwendung gemäß Anspruch 18, wobei das Intron ein Fragment eines inhärenten Introns ist und wobei das Hybrid-HGF-Konstrukt vorzugsweise die Nucleotidsequenz von SEQ ID NO: 8, 9 oder 10 beinhaltet.

21. Zusammensetzung zur Verwendung gemäß Anspruch 1, wobei der Patient ein Mensch ist und die Isoformen von HGF jeweils in einer Dosis von etwa 1 µg bis etwa 100 mg verabreicht werden.

22. Zusammensetzung zur Verwendung gemäß Anspruch 1, wobei der Patient ein Mensch ist und die Polynucleotide jeweils in einer Dosis von etwa 1 µg bis etwa 10 mg verabreicht werden.

23. Verwendung einer Zusammensetzung, die zwei oder mehr Isoformen von HGF oder ein oder mehr Polynucleotide, die für die Isoformen kodieren, beinhaltet, zur Herstellung eines Medikaments zur Behandlung einer unvollständigen Revaskularisation des Myokards bei einem Patienten, bei dem eine koronare Bypass-Operation durchgeführt wurde, die Revaskularisation des Myokards aber unvollständig war, wobei die zwei oder mehr Isoformen von HGF aus der Gruppe ausgewählt sind, die aus vollständigem HGF (fIHGF), deletierter HGF-Variante (dHGF), NK1, NK2 und NK4 besteht.

## Revendications

1. Composition pour utilisation dans le traitement d'une revascularisation incomplète du myocarde chez un sujet qui a subi un pontage coronarien par greffe, mais présente une revascularisation incomplète du myocarde, la composition comprenant deux ou plusieurs isoformes de HGF ou un ou plusieurs polynucléotides codant pour les isoformes comme ingrédients actifs, dans laquelle lesdites deux ou plusieurs isoformes de HGF sont choisies dans le groupe constitué de HGF de pleine longueur (flHGF), d'une variante de HGF délétée (dHGF), de NK1, de NK2 et de NK4.

2. Composition pour usage selon la revendication 1, dans laquelle le sujet a besoin d'un traitement de la resténose.

3. Composition pour usage selon l'une quelconque des revendications 1 et 2, dans laquelle lesdites deux ou plusieurs isoformes de HGF sont administrées sous la forme de polynucléotides codant pour les isoformes.

4. Composition pour usage selon l'une quelconque des revendications 1 à 3, dans laquelle ladite composition est administrée par injection.

5. Composition pour usage selon l'une quelconque des revendications 1 à 4, dans laquelle ladite composition est administrée en utilisant un dispositif de délivrance et, de préférence, dans laquelle ledit dispositif de délivrance est un stent.

6. Composition pour usage selon la revendication 5, dans laquelle ledit stent est choisi dans le groupe constitué d'un stent d'acier inoxydable à base non polymère, d'un stent d'acier inoxydable à base polymère, d'un stent de cobalt et de chrome à base non polymère et d'un stent de cobalt et de chrome à base polymère.

7. Composition pour usage selon la revendication 5, dans laquelle ladite composition est éluée par ledit stent.

8. Composition pour usage selon la revendication 1, dans laquelle lesdites une ou plusieurs isoformes de HGF comprennent un HGF de pleine longueur (flHGF) et une variante de HGF délétée (dHGF).

9. Composition pour usage selon la revendication 8, dans laquelle lesdites deux ou plusieurs isoformes de HGF comprennent en outre du NK1.

10. Composition pour usage selon la revendication 8, dans laquelle lesdites deux ou plusieurs isoformes de HGF sont constituées de flHGF et de dHGF.

11. Composition pour usage selon la revendication 8, dans laquelle lesdits flHGF et dHGF sont chacun identiques au moins à 80 %, de préférence au moins à 90 %, mieux encore au moins à 95 %, à la séquence de type sauvage du flHGF humain et du dHGF humain.

12. Composition pour usage selon la revendication 11, dans laquelle ledit flHGF et ledit dHGF sont du flHGF humain et du dHGF humain.

13. Composition pour usage selon la revendication 8, dans laquelle ledit flHGF et ledit dHGF sont codés par des polynucléotides séparés ou sont codés par le même polynucléotide.

14. Composition pour usage selon la revendication 1, dans laquelle lesdits un ou plusieurs polynucléotides sont liés en service à un promoteur, de préférence à un promoteur constitutif.

15. Composition pour usage selon la revendication 1, dans laquelle lesdits un ou plusieurs polynucléotides se trouvent sur différents vecteurs.

16. Composition pour usage selon la revendication 1, dans laquelle lesdits un ou plusieurs polynucléotides se trouvent sur le même vecteur.

17. Composition pour usage selon la revendication 16, dans laquelle ledit vecteur est un vecteur viral ou un vecteur plasmidique, de préférence un vecteur de pCK.

18. Composition pour usage selon la revendication 8, dans laquelle ledit flHGF et ledit dHGF sont codés par une construction de HGF hybride comprenant les exons de HGF 1-18 ou leurs dégénérés qui ne modifient pas la séquence d'acides aminés codée et comprenant en outre un intron entre les exons 4 et 5, dans laquelle ladite construction est dénuée d'autres introns entre les exons autres que ledit intron entre les exons 4 et 5.

19. Composition pour usage selon la revendication 18, dans laquelle ledit intron est un intron inhérent, de préférence, dans laquelle ladite construction de HGF hybride comprend la séquence nucléotidique de SEQ ID n° 7.

20. Composition pour usage selon la revendication 18, dans laquelle ledit intron est un fragment d'un intron inhérent et, de préférence, dans laquelle ladite construction de HGF hybride comprend la séquence nucléotidique de la SEQ ID n° 8, 9 ou 10.

21. Composition pour usage selon la revendication 1, dans laquelle ledit sujet est un être humain et lesdites isoformes de HGF sont administrées en dose d'environ 1 µg à environ 100 mg chacune.

22. Composition pour usage selon la revendication 1, dans laquelle ledit sujet est un être humain et lesdits polynucléotides sont administrés en dose d'environ 1 µg à environ 10 mg.

23. Usage d'une composition comprenant deux ou plusieurs isoformes de HGF ou un ou plusieurs polynucléotides codant pour les isoformes pour la fabrication d'un médicament permettant de traiter une revascularisation incomplète du myocarde chez un sujet, qui a subi un pontage coronaire par greffe, mais qui présente une revascularisation incomplète du myocarde, dans lequel lesdites deux ou plusieurs isoformes sont choisies dans le groupe constitué de HGF de pleine longueur (flHGF), d'une variante de HGF délétée (dHGF), de NK1, de NK2 et de NK4.


### SEQUENCE LISTING

<110> viromed Co., Ltd.
<120> TREATMENT AND PREVENTION OF CARDIAC CONDITIONS USING TWO OR MORE ISOFORMS OF HEPATOCYTE GROWTH FACTOR
<130> 463.106370
<140> PCT/KR2009/000406
   <141> 2009-01-28
<150> US 61/023,756
   <151> 2008-01-25
<160> 12
<170> PatentIn 3.3
<210> 1
   <211> 2187
   <212> DNA
   <213> Hepatocyte Growth Factor
<400> 1
   atgtgggtga ccaaactcct gccagccctg ctgctgcagc atgtcctcct gcatctcctc     60
   ctgctcccca tcgccatccc ctatgcagag ggacaaagga aaagaagaaa tacaattcat    120
   gaattcaaaa aatcagcaaa gactacccta atcaaaatag atccagcact gaagataaaa    180
   accaaaaaag tgaatactgc agaccaatgt gctaatagat gtactaggaa taaaggactt    240
   ccattcactt gcaaggcttt tgtttttgat aaagcaagaa aacaatgcct ctggttcccc    300
   ttcaatagca tgtcaagtgg agtgaaaaaa gaatttggcc atgaatttga cctctatgaa    360
   aacaaagact acattagaaa ctgcatcatt ggtaaaggac gcagctacaa gggaacagta    420
   tctatcacta agagtggcat caaatgtcag ccctggagtt ccatgatacc acacgaacac    480
   agctttttgc cttcgagcta tcggggtaaa gacctacagg aaaactactg tcgaaatcct    540
   cgaggggaag aagggggacc ctggtgtttc acaagcaatc cagaggtacg ctacgaagtc    600
   tgtgacattc ctcagtgttc agaagttgaa tgcatgacct gcaatgggga gagttatcga    660
   ggtctcatgg atcatacaga atcaggcaag atttgtcagc gctgggatca tcagacacca    720
   caccggcaca aattcttgcc tgaaagatat cccgacaagg gctttgatga taattattgc    780
   cgcaatcccg atggccagcc gaggccatgg tgctatactc ttgaccctca cacccgctgg    840
   gagtactgtg caattaaaac atgcgctgac aatactatga atgacactga tgttcctttg    900
   gaaacaactg aatgcatcca aggtcaagga gaaggctaca ggggcactgt caataccatt    960
   tggaatggaa ttccatgtca gcgttgggat tctcagtatc ctcacgagca tgacatgact   1020
   cctgaaaatt tcaagtgcaa ggacctacga gaaaattact gccgaaatcc agatgggtct   1080
   gaatcaccct ggtgttttac cactgatcca aacatccgag ttggctactg ctcccaaatt   1140
   ccaaactgtg atatgtcaca tggacaagat tgttatcgtg ggaatggcaa aaattatatg   1200
   ggcaacttat cccaaacaag atctggacta acatgttcaa tgtgggacaa gaacatggaa   1260
   gacttacatc gtcatatctt ctgggaacca gatgcaagta agctgaatga gaattactgc   1320
   cgaaatccag atgatgatgc tcatggaccc tggtgctaca cgggaaatcc actcattcct    1380
   tgggattatt gccctatttc tcgttgtgaa ggtgatacca cacctacaat agtcaattta    1440
   gaccatcccg taatatcttg tgccaaaacg aaacaattgc gagttgtaaa tgggattcca    1500
   acacgaacaa acataggatg gatggttagt ttgagataca gaaataaaca tatctgcgga    1560
   ggatcattga taaaggagag ttgggttctt actgcacgac agtgtttccc ttctcgagac    1620
   ttgaaagatt atgaagcttg gcttggaatt catgatgtcc acggaagagg agatgagaaa    1680
   tgcaaacagg ttctcaatgt ttcccagctg gtatatggcc ctgaaggatc agatctggtt    1740
   ttaatgaagc ttgccaggcc tgctgtcctg gatgattttg ttagtacgat tgatttacct    1800
   aattatggat gcacaattcc tgaaaagacc agttgcagtg tttatggctg gggctacact     1860
   ggattgatca actatgatgg cctattacga gtggcacatc tctatataat gggaaatgag    1920
   aaatgcagcc agcatcatcg agggaaggtg actctgaatg agtctgaaat atgtgctggg    1980
   gctgaaaaga ttggatcagg accatgtgag ggggattatg gtggcccact tgtttgtgag    2040
   caacataaaa tgagaatggt tcttggtgtc attgttcctg gtcgtggatg tgccattcca    2100
   aatcgtcctg gtatttttgt ccgagtagca tattatgcaa aatggataca caaaattatt    2160
   ttaacatata aggtaccaca gtcatag                                         2187
<210> 2
   <211> 728
   <212> PRT
   <213> Homo sapiens
<400> 2
   Met Trp Val Thr Lys Leu Leu Pro Ala Leu Leu Leu Gln His Val Leu
   1               5                   10                  15
   Leu His Leu Leu Leu Leu Pro Ile Ala Ile Pro Tyr Ala Glu Gly Gln
               20                  25                  30
   Arg Lys Arg Arg Asn Thr Ile His Glu Phe Lys Lys Ser Ala Lys Thr
           35                  40                  45
   Thr Leu Ile Lys Ile Asp Pro Ala Leu Lys Ile Lys Thr Lys Lys Val
       50                  55                  60
   Asn Thr Ala Asp Gln Cys Ala Asn Arg Cys Thr Arg Asn Lys Gly Leu
   65                  70                  75                  80
   Pro Phe Thr Cys Lys Ala Phe Val Phe Asp Lys Ala Arg Lys Gln Cys
                   85                  90                  95
   Leu Trp Phe Pro Phe Asn Ser Met Ser Ser Gly Val Lys Lys Glu Phe
               100                 105                 110
   Gly His Glu Phe Asp Leu Tyr Glu Asn Lys Asp Tyr Ile Arg Asn Cys
   115 120 125
   Ile Ile Gly Lys Gly Arg Ser Tyr Lys Gly Thr Val Ser Ile Thr Lys
   130 135 140
   Ser Gly Ile Lys Cys Gln Pro Trp Ser Ser Met Ile Pro His Glu His
   145 150 155 160
   Ser Phe Leu Pro Ser Ser Tyr Arg Gly Lys Asp Leu Gln Glu Asn Tyr
   165 170 175
   Cys Arg Asn Pro Arg Gly Glu Glu Gly Gly Pro Trp Cys Phe Thr Ser
   180 185 190
   Asn Pro Glu Val Arg Tyr Glu Val Cys Asp Ile Pro Gln Cys Ser Glu
   195 200 205
   Val Glu Cys Met Thr Cys Asn Gly Glu Ser Tyr Arg Gly Leu Met Asp
   210 215 220
   His Thr Glu Ser Gly Lys Ile Cys Gln Arg Trp Asp His Gln Thr Pro
   225 230 235 240
   His Arg His Lys Phe Leu Pro Glu Arg Tyr Pro Asp Lys Gly Phe Asp
   245 250 255
   Asp Asn Tyr Cys Arg Asn Pro Asp Gly Gln Pro Arg Pro Trp Cys Tyr
   260 265 270
   Thr Leu Asp Pro His Thr Arg Trp Glu Tyr Cys Ala Ile Lys Thr Cys
   275 280 285
   Ala Asp Asn Thr Met Asn Asp Thr Asp Val Pro Leu Glu Thr Thr Glu
   290 295 300
   Cys Ile Gln Gly Gln Gly Glu Gly Tyr Arg Gly Thr Val Asn Thr Ile
   305 310 315 320
   Trp Asn Gly Ile Pro Cys Gln Arg Trp Asp Ser Gln Tyr Pro His Glu
   325 330 335
   His Asp Met Thr Pro Glu Asn Phe Lys Cys Lys Asp Leu Arg Glu Asn
   340 345 350
   Tyr Cys Arg Asn Pro Asp Gly Ser Glu Ser Pro Trp Cys Phe Thr Thr
   355 360 365
   Asp Pro Asn Ile Arg Val Gly Tyr Cys Ser Gln Ile Pro Asn Cys Asp
   370 375 380
   Met Ser His Gly Gln Asp Cys Tyr Arg Gly Asn Gly Lys Asn Tyr Met
   385 390 395 400
   Gly Asn Leu Ser Gln Thr Arg Ser Gly Leu Thr Cys Ser Met Trp Asp
   405 410 415
   Lys Asn Met Glu Asp Leu His Arg His Ile Phe Trp Glu Pro Asp Ala
   420 425 430
   Ser Lys Leu Asn Glu Asn Tyr Cys Arg Asn Pro Asp Asp Asp Ala His
   435 440 445
   Gly Pro Trp Cys Tyr Thr Gly Asn Pro Leu Ile Pro Trp Asp Tyr Cys
   450 455 460
   Pro Ile Ser Arg Cys Glu Gly Asp Thr Thr Pro Thr Ile Val Asn Leu
   465 470 475 480
   Asp His Pro Val Ile Ser Cys Ala Lys Thr Lys Gln Leu Arg Val Val
   485 490 495
   Asn Gly Ile Pro Thr Arg Thr Asn Ile Gly Trp Met Val Ser Leu Arg
   500 505 510
   Tyr Arg Asn Lys His Ile Cys Gly Gly Ser Leu Ile Lys Glu Ser Trp
   515 520 525
   Val Leu Thr Ala Arg Gln Cys Phe Pro Ser Arg Asp Leu Lys Asp Tyr
   530 535 540
   Glu Ala Trp Leu Gly Ile His Asp Val His Gly Arg Gly Asp Glu Lys
   545 550 555 560
   Cys Lys Gln Val Leu Asn Val Ser Gln Leu Val Tyr Gly Pro Glu Gly
   565 570 575
   Ser Asp Leu Val Leu Met Lys Leu Ala Arg Pro Ala Val Leu Asp Asp
   580 585 590
   Phe Val Ser Thr Ile Asp Leu Pro Asn Tyr Gly Cys Thr Ile Pro Glu
   595 600 605
   Lys Thr Ser Cys Ser Val Tyr Gly Trp Gly Tyr Thr Gly Leu Ile Asn
   610 615 620
   Tyr Asp Gly Leu Leu Arg Val Ala His Leu Tyr Ile Met Gly Asn Glu
   625 630 635 640
   Lys Cys Ser Gln His His Arg Gly Lys Val Thr Leu Asn Glu Ser Glu
   645 650 655
   Ile Cys Ala Gly Ala Glu Lys Ile Gly Ser Gly Pro Cys Glu Gly Asp
                   660                 665                 670
   Tyr Gly Gly Pro Leu Val Cys Glu Gln His Lys Met Arg Met Val Leu
           675                 680                 685
   Gly Val Ile Val Pro Gly Arg Gly Cys Ala Ile Pro Asn Arg Pro Gly
       690                 695                 700
   Ile Phe Val Arg Val Ala Tyr Tyr Ala Lys Trp Ile His Lys Ile Ile
   705                 710                 715                 720
   Leu Thr Tyr Lys Val Pro Gln Ser
                   725
<210> 3
   <211> 723
   <212> PRT
   <213> Homo sapiens
<400> 3
   Met Trp Val Thr Lys Leu Leu Pro Ala Leu Leu Leu Gln His Val Leu
   1               5                   10                  15
   Leu His Leu Leu Leu Leu Pro Ile Ala Ile Pro Tyr Ala Glu Gly Gln
               20                  25                  30
   Arg Lys Arg Arg Asn Thr Ile His Glu Phe Lys Lys Ser Ala Lys Thr
           35                  40                  45
   Thr Leu Ile Lys Ile Asp Pro Ala Leu Lys Ile Lys Thr Lys Lys Val
       50                  55                  60
   Asn Thr Ala Asp Gln Cys Ala Asn Arg Cys Thr Arg Asn Lys Gly Leu
   65                  70                  75                  80
   Pro Phe Thr Cys Lys Ala Phe Val Phe Asp Lys Ala Arg Lys Gln Cys
                   85                  90                  95
   Leu Trp Phe Pro Phe Asn Ser Met Ser Ser Gly Val Lys Lys Glu Phe
               100                 105                 110
   Gly His Glu Phe Asp Leu Tyr Glu Asn Lys Asp Tyr Ile Arg Asn Cys
           115                 120                 125
   Ile Ile Gly Lys Gly Arg Ser Tyr Lys Gly Thr Val Ser Ile Thr Lys
       130                 135                 140
   Ser Gly Ile Lys Cys Gln Pro Trp Ser Ser Met Ile Pro His Glu His
   145                 150                 155                 160
   Ser Tyr Arg Gly Lys Asp Leu Gln Glu Asn Tyr Cys Arg Asn Pro Arg
                   165                 170                 175
   Gly Glu Glu Gly Gly Pro Trp Cys Phe Thr Ser Asn Pro Glu Val Arg
               180                 185                 190
   Tyr Glu Val Cys Asp Ile Pro Gln Cys Ser Glu Val Glu Cys Met Thr
           195                 200                 205
   Cys Asn Gly Glu Ser Tyr Arg Gly Leu Met Asp His Thr Glu Ser Gly
       210                 215                 220
   Lys Ile Cys Gln Arg Trp Asp His Gln Thr Pro His Arg His Lys Phe
   225                 230                 235                 240
   Leu Pro Glu Arg Tyr Pro Asp Lys Gly Phe Asp Asp Asn Tyr Cys Arg
                   245                 250                 255
   Asn Pro Asp Gly Gln Pro Arg Pro Trp Cys Tyr Thr Leu Asp Pro His
               260                 265                 270
   Thr Arg Trp Glu Tyr Cys Ala Ile Lys Thr Cys Ala Asp Asn Thr Met
           275                 280                 285
   Asn Asp Thr Asp Val Pro Leu Glu Thr Thr Glu Cys Ile Gln Gly Gln
       290                 295                 300
   Gly Glu Gly Tyr Arg Gly Thr Val Asn Thr Ile Trp Asn Gly Ile Pro
   305                 310                 315                 320
   Cys Gln Arg Trp Asp Ser Gln Tyr Pro His Glu His Asp Met Thr Pro
                   325                 330                 335
   Glu Asn Phe Lys Cys Lys Asp Leu Arg Glu Asn Tyr Cys Arg Asn Pro
               340                 345                 350
   Asp Gly Ser Glu Ser Pro Trp Cys Phe Thr Thr Asp Pro Asn Ile Arg
           355                 360                 365
   Val Gly Tyr Cys Ser Gln Ile Pro Asn Cys Asp Met Ser His Gly Gln
       370                 375                 380
   Asp Cys Tyr Arg Gly Asn Gly Lys Asn Tyr Met Gly Asn Leu Ser Gln
   385                 390                 395                 400
   Thr Arg Ser Gly Leu Thr Cys Ser Met Trp Asp Lys Asn Met Glu Asp
                   405                 410                 415
   Leu His Arg His Ile Phe Trp Glu Pro Asp Ala Ser Lys Leu Asn Glu
               420                 425                 430
   Asn Tyr Cys Arg Asn Pro Asp Asp Asp Ala His Gly Pro Trp Cys Tyr
           435                 440                 445
   Thr Gly Asn Pro Leu Ile Pro Trp Asp Tyr Cys Pro Ile Ser Arg Cys
   450 455 460
   Glu Gly Asp Thr Thr Pro Thr Ile Val Asn Leu Asp His Pro Val Ile
   465 470 475 480
   Ser Cys Ala Lys Thr Lys Gln Leu Arg Val Val Asn Gly Ile Pro Thr
   485 490 495
   Arg Thr Asn Ile Gly Trp Met Val Ser Leu Arg Tyr Arg Asn Lys His
   500 505 510
   Ile Cys Gly Gly Ser Leu Ile Lys Glu Ser Trp Val Leu Thr Ala Arg
   515 520 525
   Gln Cys Phe Pro Ser Arg Asp Leu Lys Asp Tyr Glu Ala Trp Leu Gly
   530 535 540
   Ile His Asp Val His Gly Arg Gly Asp Glu Lys Cys Lys Gln Val Leu
   545 550 555 560
   Asn Val Ser Gln Leu Val Tyr Gly Pro Glu Gly Ser Asp Leu Val Leu
   565 570 575
   Met Lys Leu Ala Arg Pro Ala Val Leu Asp Asp Phe Val Ser Thr Ile
   580 585 590
   Asp Leu Pro Asn Tyr Gly Cys Thr Ile Pro Glu Lys Thr Ser Cys Ser
   595 600 605
   Val Tyr Gly Trp Gly Tyr Thr Gly Leu Ile Asn Tyr Asp Gly Leu Leu
   610 615 620
   Arg Val Ala His Leu Tyr Ile Met Gly Asn Glu Lys Cys Ser Gln His
   625 630 635 640
   His Arg Gly Lys Val Thr Leu Asn Glu Ser Glu Ile Cys Ala Gly Ala
   645 650 655
   Glu Lys Ile Gly Ser Gly Pro Cys Glu Gly Asp Tyr Gly Gly Pro Leu
   660 665 670
   Val Cys Glu Gln His Lys Met Arg Met Val Leu Gly Val Ile Val Pro
   675 680 685
   Gly Arg Gly Cys Ala Ile Pro Asn Arg Pro Gly Ile Phe Val Arg Val
   690 695 700
   Ala Tyr Tyr Ala Lys Trp Ile His Lys Ile Ile Leu Thr Tyr Lys Val
   705 710 715 720
   Pro Gln Ser
<210> 4
   <211> 207
   <212> PRT
   <213> Homo sapiens
<400> 4
   Met Trp Val Thr Lys Leu Leu Pro Ala Leu Leu Leu Gln His Val Leu
   1               5                   10                  15
   Leu His Leu Leu Leu Leu Pro Ile Ala Ile Pro Tyr Ala Glu Gly Gln
               20                  25                  30
   Arg Lys Arg Arg Asn Thr Ile His Glu Phe Lys Lys Ser Ala Lys Thr
           35                  40                  45
   Thr Leu Ile Lys Ile Asp Pro Ala Leu Lys Ile Lys Thr Lys Lys Val
       50                  55                  60
   Asn Thr Ala Asp Gln Cys Ala Asn Arg Cys Thr Arg Asn Lys Gly Leu
   65                  70                  75                  80
   Pro Phe Thr Cys Lys Ala Phe Val Phe Asp Lys Ala Arg Lys Gln Cys
                   85                  90                  95
   Leu Trp Phe Pro Phe Asn Ser Met Ser Ser Gly Val Lys Lys Glu Phe
               100                 105                 110
   Gly His Glu Phe Asp Leu Tyr Glu Asn Lys Asp Tyr Ile Arg Asn Cys
           115                 120                 125
   Ile Ile Gly Lys Gly Arg Ser Tyr Lys Gly Thr Val Ser Ile Thr Lys
       130                 135                 140
   Ser Gly Ile Lys Cys Gln Pro Trp Ser Ser Met Ile Pro His Glu His
   145                 150                 155                 160
   Ser Phe Leu Pro Ser Ser Tyr Arg Gly Lys Asp Leu Gln Glu Asn Tyr
                   165                 170                 175
   Cys Arg Asn Pro Arg Gly Glu Glu Gly Gly Pro Trp Cys Phe Thr Ser
               180                 185                 190
   Asn Pro Glu Val Arg Tyr Glu Val Cys Asp Ile Pro Gln Cys Ser
           195                 200                 205
<210> 5
   <211> 290
   <212> PRT
   <213> Homo sapiens
<400> 5
   Met Trp Val Thr Lys Leu Leu Pro Ala Leu Leu Leu Gln His Val Leu
   1               5                   10                  15
   Leu His Leu Leu Leu Leu Pro Ile Ala Ile Pro Tyr Ala Glu Gly Gln
               20                  25                  30
   Arg Lys Arg Arg Asn Thr Ile His Glu Phe Lys Lys Ser Ala Lys Thr
           35                  40                  45
   Thr Leu Ile Lys Ile Asp Pro Ala Leu Lys Ile Lys Thr Lys Lys Val
       50                  55                  60
   Asn Thr Ala Asp Gln Cys Ala Asn Arg Cys Thr Arg Asn Lys Gly Leu
   65                  70                  75                  80
   Pro Phe Thr Cys Lys Ala Phe Val Phe Asp Lys Ala Arg Lys Gln Cys
                   85                  90                  95
   Leu Trp Phe Pro Phe Asn Ser Met Ser Ser Gly Val Lys Lys Glu Phe
               100                 105                 110
   Gly His Glu Phe Asp Leu Tyr Glu Asn Lys Asp Tyr Ile Arg Asn Cys
           115                 120                 125
   Ile Ile Gly Lys Gly Arg Ser Tyr Lys Gly Thr Val Ser Ile Thr Lys
       130                 135                 140
   Ser Gly Ile Lys Cys Gln Pro Trp Ser Ser Met Ile Pro His Glu His
   145                 150                 155                 160
   Ser Phe Leu Pro Ser Ser Tyr Arg Gly Lys Asp Leu Gln Glu Asn Tyr
                   165                 170                 175
   Cys Arg Asn Pro Arg Gly Glu Glu Gly Gly Pro Trp Cys Phe Thr Ser
               180                 185                 190
   Asn Pro Glu Val Arg Tyr Glu Val Cys Asp Ile Pro Gln Cys Ser Glu
           195                 200                 205
   Val Glu Cys Met Thr Cys Asn Gly Glu Ser Tyr Arg Gly Leu Met Asp
       210                 215                 220
   His Thr Glu Ser Gly Lys Ile Cys Gln Arg Trp Asp His Gln Thr Pro
   225                 230                 235                 240
   His Arg His Lys Phe Leu Pro Glu Arg Tyr Pro Asp Lys Gly Phe Asp
                   245                 250                 255
   Asp Asn Tyr Cys Arg Asn Pro Asp Gly Gln Pro Arg Pro Trp Cys Tyr
   260 265 270
   Thr Leu Asp Pro His Thr Arg Trp Glu Tyr Cys Ala Ile Lys Thr Cys
   275 280 285
   Glu Thr
   290
<210> 6
   <211> 470
   <212> PRT
   <213> Homo sapiens
<400> 6
   Met Trp Val Thr Lys Leu Leu Pro Ala Leu Leu Leu Gln His Val Leu
   1 5 10 15
   Leu His Leu Leu Leu Leu Pro Ile Ala Ile Pro Tyr Ala Glu Gly Gln
   20 25 30
   Arg Lys Arg Arg Asn Thr Ile His Glu Phe Lys Lys Ser Ala Lys Thr
   35 40 45
   Thr Leu Ile Lys Ile Asp Pro Ala Leu Lys Ile Lys Thr Lys Lys Val
   50 55 60
   Asn Thr Ala Asp Gln Cys Ala Asn Arg Cys Thr Arg Asn Lys Gly Leu
   65 70 75 80
   Pro Phe Thr Cys Lys Ala Phe Val Phe Asp Lys Ala Arg Lys Gln Cys
   85 90 95
   Leu Trp Phe Pro Phe Asn Ser Met Ser Ser Gly Val Lys Lys Glu Phe
   100 105 110
   Gly His Glu Phe Asp Leu Tyr Glu Asn Lys Asp Tyr Ile Arg Asn Cys
   115 120 125
   Ile Ile Gly Lys Gly Arg Ser Tyr Lys Gly Thr Val Ser Ile Thr Lys
   130 135 140
   Ser Gly Ile Lys Cys Gln Pro Trp Ser Ser Met Ile Pro His Glu His
   145 150 155 160
   Ser Phe Leu Pro Ser Ser Tyr Arg Gly Lys Asp Leu Gln Glu Asn Tyr
   165 170 175
   Cys Arg Asn Pro Arg Gly Glu Glu Gly Gly Pro Trp Cys Phe Thr Ser
   180 185 190
   Asn Pro Glu Val Arg Tyr Glu Val Cys Asp Ile Pro Gln Cys Ser Glu
   195 200 205
   Val Glu Cys Met Thr Cys Asn Gly Glu Ser Tyr Arg Gly Leu Met Asp
       210                 215                 220
   His Thr Glu Ser Gly Lys Ile Cys Gln Arg Trp Asp His Gln Thr Pro
   225                 230                 235                 240
   His Arg His Lys Phe Leu Pro Glu Arg Tyr Pro Asp Lys Gly Phe Asp
                   245                 250                 255
   Asp Asn Tyr Cys Arg Asn Pro Asp Gly Gln Pro Arg Pro Trp Cys Tyr
               260                 265                 270
   Thr Leu Asp Pro His Thr Arg Trp Glu Tyr Cys Ala Ile Lys Thr Cys
           275                 280                 285
   Ala Asp Asn Thr Met Asn Asp Thr Asp Val Pro Leu Glu Thr Thr Glu
       290                 295                 300
   Cys Ile Gln Gly Gln Gly Glu Gly Tyr Arg Gly Thr Val Asn Thr Ile
   305                 310                 315                 320
   Trp Asn Gly Ile Pro Cys Gln Arg Trp Asp Ser Gln Tyr Pro His Glu
                   325                 330                 335
   His Asp Met Thr Pro Glu Asn Phe Lys Cys Lys Asp Leu Arg Glu Asn
               340                 345                 350
   Tyr Cys Arg Asn Pro Asp Gly Ser Glu Ser Pro Trp Cys Phe Thr Thr
           355                 360                 365
   Asp Pro Asn Ile Arg Val Gly Tyr Cys Ser Gln Ile Pro Asn Cys Asp
       370                 375                 380
   Met Ser His Gly Gln Asp Cys Tyr Arg Gly Asn Gly Lys Asn Tyr Met
   385                 390                 395                 400
   Gly Asn Leu Ser Gln Thr Arg Ser Gly Leu Thr Cys Ser Met Trp Asp
                   405                 410                 415
   Lys Asn Met Glu Asp Leu His Arg His Ile Phe Trp Glu Pro Asp Ala
               420                 425                 430
   Ser Lys Leu Asn Glu Asn Tyr Cys Arg Asn Pro Asp Asp Asp Ala His
           435                 440                 445
   Gly Pro Trp Cys Tyr Thr Gly Asn Pro Leu Ile Pro Trp Asp Tyr Cys
       450                 455                 460
   Pro Ile Ser Arg Cys Glu
   465                 470
<210> 7
   <211> 7113
   <212> DNA
   <213> hepatocyte growth factor hybrid
<400> 7
   atgtgggtga ccaaactcct gccagccctg ctgctgcagc atgtcctcct gcatctcctc      60
   ctgctcccca tcgccatccc ctatgcagag ggacaaagga aaagaagaaa tacaattcat     120
   gaattcaaaa aatcagcaaa gactacccta atcaaaatag atccagcact gaagataaaa     180
   accaaaaaag tgaatactgc agaccaatgt gctaatagat gtactaggaa taaaggactt     240
   ccattcactt gcaaggcttt tgtttttgat aaagcaagaa aacaatgcct ctggttcccc     300
   ttcaatagca tgtcaagtgg agtgaaaaaa gaatttggcc atgaatttga cctctatgaa     360
   aacaaagact acattagaaa ctgcatcatt ggtaaaggac gcagctacaa gggaacagta     420
   tctatcacta agagtggcat caaatgtcag ccctggagtt ccatgatacc acacgaacac     480
   aggtaagaac agtatgaaga aaagagatga agcctctgtc ttttttacat gttaacagtc      540
   tcatattagt ccttcagaat aattctacaa tcctaaaata acttagccaa cttgctgaat     600
   tgtattacgg caaggtttat atgaattcat gactgatatt tagcaaatga ttaattaata     660
   tgttaataaa atgtagccaa aacaatatct taccttaatg cctcaatttg tagatctcgg     720
   tatttgtgaa ataataacgt aaacttcgtt taaaaggatt cttcttcctg tctttgagaa     780
   agtacggcac tgtgcagggg gagaggttga ttgtgaaaaa tcagaggtag atgagaatct     840
   tactgagggc tgagggttct ttaaccttgg tggatctcaa cattggttgc acattaaaat     900
   cacctgctgc aagcccttga cgaatcttac ttagaagatg acaacacaga acaattaaat     960
   cagaatctct ggggagaata gggcaccagt attttttgag ctcccaccat gattccaaag     1020
   tgcagccaaa tttgagaacc actgctaaaa gctcaagctt cagattgacc agcttttcca    1080
   tctcacctat cgcctaaaga ccaaattgga taaatgtgtt cattacgaca gatgggtact    1140
   atttaaagat gagtaaacac aatatactta ggctcgtcag actgagagtt ttaatcatca    1200
   ctgaggaaaa acatagatat ctaatactga ctggagtatt agtcaaggct tatttcacac    1260
   acaattttat cagaaaccaa agtagtttaa aacagctctc cccttattag taatgcattg    1320
   gagggtttac tttaccatgt accttgctga gcactgtacc ttgttaatct catttacttg    1380
   taatgagaac cacacagcgg gtagttttat tggttctatt ttacctacat gacaaaactg    1440
   aagcataaaa acacttagta agttttcagt gtcatgcaca actaggaagt gacatggcca    1500
   gaatataagc ccagtcacca tcactctata acctgcgctt ttaacaactt cagggcatga    1560
   cacatttggc cggtcagtag aacccatgct gtgatttgtt tttgcagtgg tggtgatgac    1620
   tgccttgttg aatccacttt ttattctatt ccattttggg gacacaattc tgcaagatga    1680
   ttcttcatta ggaaacagag atgagttatt gaccaacaca gaaagaaaaa gagtttgttg    1740
   ctccacactg ggattaaacc tatgatcttg gcctaattaa cactagctag taagtgtcca    1800
   agctgatcat ctctacaaca tttcaataac agaaaacaac aattttcaaa attagttact    1860
   tacaattatg tagaaatgcc tctaaaacac agtattttcc ttatattaca aaaacaaaaa 1920
   ttataattgg ttttgtcctc ttttgagagt ttgcatggtg ttactccctg catagtgaag 1980
   aaaacatttt atttaagtag atggatctaa gtttttcatg aacaaaggaa tgacatttga 2040
   aatcaatcct accctagtcc aggagaatgc attagattaa cctagtagag gtcttatttc 2100
   accctgagtt ttctatgatc gtgattctct gctggaggag taattgtgaa atagatctct 2160
   ctgggaactg gcttcctagt ccaatcagct cttttaccaa tgaacacttc cttgtgatat 2220
   agatgtttat ggccgagagg atccagtata ttaataaaat ccctttttgt attcaatgag 2280
   ggaaacacat aattttcatc aattagcagc ttattggaat atctgcatga tggtttaaca 2340
   cttttaagtg ttgactaaag attaatttta cagaaaatag aaaaagaaat atgtttctgt 2400
   ctggaggaat gatttattgt tgacccctaa attgaaatat tttactagtg gcttaatgga 2460
   aagatgatga aagatgatga aattaatgta gaagcttaac tagaaaatca ggtgacctga 2520
   tatctacatc tgtatccttc attggccacc cagcattcat taatgaatca gatgatggaa 2580
   tagatcaagt ttcctaggaa cacagtgaat attaaaagaa aacaaaggga gcctagcacc 2640
   tagaagacct agtttatatt tcaaagtata tttggatgta acccaatttt aaacatttcc 2700
   tcacttgtct ctcttaaagc cttgccaaca gcaaggacag agaaccaaaa atagtgtata 2760
   tatgaataaa tgcttattac agaatctgct gactggcaca tgctttgtgt gtaatgggtt 2820
   ctcataaaca cttgttgaat gaacacacat aagtgaaaga gcatggctag gcttcatccc 2880
   ttggtcaaat atggggtgct aaagaaaagc aggggaaata cattgggaca ctaacaaaaa 2940
   aaaacagtta atttaggtaa aagataaaat acaccacaga atgaagaaaa gagatgaccc 3000
   agactgctct ttaaccttca tgtcctagag aggtttttga tatgaattgc attcagaatt 3060
   gtggaaagga gcccatcttt tctcttcatt ttgattttat taactccaat gggggaattt 3120
   tattcgtgtt ttggccatat ctacttttga tttctacatt attctctctt cctttctacc 3180
   tgtatttgtc ctaataaatt gttgacttat taattcacta cttcctcaca gctttttttt 3240
   ggctttacaa atccactgga aaggtatatg ggtgtatcac tttgtgtatt tcggtgtgca 3300
   tgtgtagagg ggacaaaaat cctctctcaa actataaata ttgagtattt gtgtattgaa 3360
   catttgctat aactactagg tttcttaaat aatcttaata tataaaatga tatagaaaaa 3420
   gggaaattat agttcgtatt attcatctaa gtgaagagat taaaacccag ggagtaaata 3480
   aattgtctaa ggactaaggt tgtatactat ttaggtgata gatatggggc aaccgtatgg 3540
   gttttatgat taacaaataa acttctcacc actctaccat atcaactttt ccataaaaga 3600
   gagctatagt attctttgct taaataaatt tgattagtgc atgacttctt gaaaacatat 3660
   aaagcaaaag tcacatttga ttctatcaga aaagtgagta agccatggcc caaacaaaag 3720
   atgcattaaa atattctgga atgatggagc taaaagtaag aaaaatgact ttttaaaaaa 3780
   gtttactgtt aggaattgtg aaattatgct gaattttagt tgcattataa tttttgtcag 3840
   tcatacggtc tgacaacctg tcttatttct atttccccat atgaggaatg ctagttaagt 3900
   atggatatta actattacta cttagatgca ttgaagttgc ataatatgga taatacttca 3960
   ctggttccct gaaaatgttt agttagtaat aagtctctta cactatttgt tttgtccaat 4020
   aatttatatt ttctgaagac ttaactctag aatacactca tgtcaaaatg aaagaatttc 4080
   attgcaaaat attgcttggt acatgacgca tacctgtatt tgttttgtgt cacaacatga 4140
   aaaatgatgg tttattagaa gtttcattgg gtaggaaaca catttgaatg gtatttacta 4200
   agatactaaa atccttggac ttcactctaa ttttagtgcc atttagaact caaggtctca 4260
   gtaaaagtag aaataaagcc tgttaacaaa acacaagctg aatattaaaa atgtaactgg 4320
   attttcaaag aaatgtttac tggtattacc tgtagatgta tattctttat tatgatcttt 4380
   tgtgtaaagt ctggcagaca aatgcaatat ctaattgttg agtccaatat cacaagcagt 4440
   acaaaagtat aaaaaagact tggccttttc taatgtgtta aaatacttta tgctggtaat 4500
   aacactaaga gtagggcact agaaatttta agtgaagata atgtgttgca gttactgcac 4560
   tcaatggctt actattataa accaaaactg ggatcactaa gctccagtca gtcaaaatga 4620
   tcaaaattat tgaagagaat aagcaattct gttctttatt aggacacagt agatacagac 4680
   tacaaagtgg agtgtgctta ataagaggta gcatttgtta agtgtcaatt actctattat 4740
   cccttggagc ttctcaaaat aaccatataa ggtgtaagat gttaaaggtt atggttacac 4800
   tcagtgcaca ggtaagctaa taggctgaga gaagctaaat tacttactgg ggtctcacag 4860
   taagaaagtg agctgaagtt tcagcccaga tttaactgga ttctgggctc tttattcatg 4920
   ttacttcatg aatctgtttc tcaattgtgc agaaaaaagg gggctattta taagaaaagc 4980
   aataaacaaa caagtaatga tctcaaataa gtaatgcaag aaatagtgag atttcaaaat 5040
   cagtggcagc gatttctcag ttctgtccta agtggccttg ctcaatcacc tgctatcttt 5100
   tagtggagct ttgaaattat gtttcagaca acttcgattc agttctagaa tgtttgactc 5160
   agcaaattca caggctcatc tttctaactt gatggtgaat atggaaattc agctaaatgg 5220
   atgttaataa aattcaaacg ttttaaggac agatgaaaat gacagaattt taaggtaaaa 5280
   tatatgaagg aatataagat aaaggatttt tctaccttca gcaaaaacat acccactaat 5340
   tagtaaaatt aataggcaaa aaaaagttgc atgctcttat actgtaatga ttatcatttt 5400
   aaaactagct ttttgccttc gagctatcgg ggtaaagacc tacaggaaaa ctactgtcga 5460
   aatcctcgag gggaagaagg gggaccctgg tgtttcacaa gcaatccaga ggtacgctac 5520
   gaagtctgtg acattcctca gtgttcagaa gttgaatgca tgacctgcaa tggggagagt 5580
   tatcgaggtc tcatggatca tacagaatca ggcaagattt gtcagcgctg ggatcatcag 5640
   acaccacacc ggcacaaatt cttgcctgaa agatatcccg acaagggctt tgatgataat 5700
   tattgccgca atcccgatgg ccagccgagg ccatggtgct atactcttga ccctcacacc 5760
   cgctgggagt actgtgcaat taaaacatgc gctgacaata ctatgaatga cactgatgtt 5820
   cctttggaaa caactgaatg catccaaggt caaggagaag gctacagggg cactgtcaat 5880
   accatttgga atggaattcc atgtcagcgt tgggattctc agtatcctca cgagcatgac 5940
   atgactcctg aaaatttcaa gtgcaaggac ctacgagaaa attactgccg aaatccagat 6000
   gggtctgaat caccctggtg ttttaccact gatccaaaca tccgagttgg ctactgctcc 6060
   caaattccaa actgtgatat gtcacatgga caagattgtt atcgtgggaa tggcaaaaat 6120
   tatatgggca acttatccca aacaagatct ggactaacat gttcaatgtg ggacaagaac 6180
   atggaagact tacatcgtca tatcttctgg gaaccagatg caagtaagct gaatgagaat 6240
   tactgccgaa atccagatga tgatgctcat ggaccctggt gctacacggg aaatccactc 6300
   attccttggg attattgccc tatttctcgt tgtgaaggtg ataccacacc tacaatagtc 6360
   aatttagacc atcccgtaat atcttgtgcc aaaacgaaac aattgcgagt tgtaaatggg 6420
   attccaacac gaacaaacat aggatggatg gttagtttga gatacagaaa taaacatatc 6480
   tgcggaggat cattgataaa ggagagttgg gttcttactg cacgacagtg tttcccttct 6540
   cgagacttga aagattatga agcttggctt ggaattcatg atgtccacgg aagaggagat 6600
   gagaaatgca aacaggttct caatgtttcc cagctggtat atggccctga aggatcagat 6660
   ctggttttaa tgaagcttgc caggcctgct gtcctggatg attttgttag tacgattgat 6720
   ttacctaatt atggatgcac aattcctgaa aagaccagtt gcagtgttta tggctggggc 6780
   tacactggat tgatcaacta tgatggccta ttacgagtgg cacatctcta tataatggga 6840
   aatgagaaat gcagccagca tcatcgaggg aaggtgactc tgaatgagtc tgaaatatgt 6900
   gctggggctg aaaagattgg atcaggacca tgtgaggggg attatggtgg cccacttgtt 6960
   tgtgagcaac ataaaatgag aatggttctt ggtgtcattg ttcctggtcg tggatgtgcc 7020
   attccaaatc gtcctggtat ttttgtccga gtagcatatt atgcaaaatg gatacacaaa 7080
   attattttaa catataaggt accacagtca tag 7113
<210> 8
   <211> 4679
   <212> DNA
   <213> Artificial sequence
<220>
   <223> HGF-X6 gene
<400> 8
   atgtgggtga ccaaactcct gccagccctg ctgctgcagc atgtcctcct gcatctcctc 60
   ctgctcccca tcgccatccc ctatgcagag ggacaaagga aaagaagaaa tacaattcat 120
   gaattcaaaa aatcagcaaa gactacccta atcaaaatag atccagcact gaagataaaa 180
   accaaaaaag tgaatactgc agaccaatgt gctaatagat gtactaggaa taaaggactt 240
   ccattcactt gcaaggcttt tgtttttgat aaagcaagaa aacaatgcct ctggttcccc 300
   ttcaatagca tgtcaagtgg agtgaaaaaa gaatttggcc atgaatttga cctctatgaa 360
   aacaaagact acattagaaa ctgcatcatc ggtaaaggac gcagctacaa gggaacagta 420
   tctatcacta agagtggcat caaatgtcag ccctggagtt ccatgatacc acacgaacac 480
   aggtaagaac agtatgaaga aaagagatga agcctctgtc ttttttacat gttaacagtc 540
   tcatattagt ccttcagaat aattctacaa tcctaaaata acttagccaa cttgctgaat 600
   tgtattacgg caaggtttat atgaattcat gactgatatt tagcaaatga ttaattaata 660
   tgttaataaa atgtagccaa aacaatatct taccttaatg cctcaatttg tagatctcgg 720
   tatttgtgga tcccttcctt tctacctgta tttgtcctaa taaattgttg acttattaat 780
   tcactacttc ctcacagctt ttttttggct ttacaaatcc actggaaagg tatatgggtg 840
   tatcactttg tgtatttcgg tgtgcatgtg tagaggggac aaaaatcctc tctcaaacta 900
   taaatattga gtatttgtgt attgaacatt tgctataact actaggtttc ttaaataatc 960
   ttaatatata aaatgatata gaaaaaggga aattatagtt cgtattattc atctaagtga 1020
   agagattaaa acccagggag taaataaatt gtctaaggac taaggttgta tactatttag 1080
   gtgatagata tggggcaacc gtatgggttt tatgattaac aaataaactt ctcaccactc 1140
   taccatatca acttttccat aaaagagagc tatagtattc tttgcttaaa taaatttgat 1200
   tagtgcatga cttcttgaaa acatataaag caaaagtcac atttgattct atcagaaaag 1260
   tgagtaagcc atggcccaaa caaaagatgc attaaaatat tctggaatga tggagctaaa 1320
   agtaagaaaa atgacttttt aaaaaagttt actgttagga attgtgaaat tatgctgaat 1380
   tttagttgca ttataatttt tgtcagtcat acggtctgac aacctgtctt atttctattt 1440
   ccccatatga ggaatgctag ttaagtatgg atattaacta ttactactta gatgcattga 1500
   agttgcataa tatggataat acttcactgg ttccctgaaa atgtttagtt agtaataagt 1560
   ctcttacact atttgttttg tccaataatt tatattttct gaagacttaa ctctagaata 1620
   cactcatgtc aaaatgaaag aatttcattg caaaatattg cttggtacat gacgcatacc 1680
   tgtatttgtt ttgtgtcaca acatgaaaaa tgatggttta ttagaagttt cattgggtag 1740
   gaaacacatt tgaatggtat ttactaagat actaaaatcc ttggacttca ctctaatttt 1800
   agtgccattt agaactcaag gtctcagtaa aagtagaaat aaagcctgtt aacaaaacac 1860
   aagctgaata ttaaaaatgt aactggattt tcaaagaaat gtttactggt attacctgta 1920
   gatgtatatt ctttattatg atcttttgtg taaagtctgg cagacaaatg caatatctaa 1980
   ttgttgagtc caatatcaca agcagtacaa aagtataaaa aagacttggc cttttctaat 2040
   gtgttaaaat actttatgct ggtaataaca ctaagagtag ggcactagaa attttaagtg 2100
   aagataatgt gttgcagtta ctgcactcaa tggcttacta ttataaacca aaactgggat 2160
   cactaagctc cagtcagtca aaatgatcaa aattattgaa gagaataagc aattctgttc 2220
   tttattagga cacagtagat acagactaca aagtggagtg tgcttaataa gaggtagcat 2280
   ttgttaagtg tcaattactc tattatccct tggagcttct caaaataacc atataaggtg 2340
   taagatgtta aaggttatgg ttacactcag tgcacaggta agctaatagg ctgagagaag 2400
   ctaaattact tactggggtc tcacagtaag aaagtgagct gaagtttcag cccagattta 2460
   actggattct gggctcttta ttcatgttac ttcatgaatc tgtttctcaa ttgtgcagaa 2520
   aaaagggggc tatttataag aaaagcaata aacaaacaag taatgatctc aaataagtaa 2580
   tgcaagaaat agtgagattt caaaatcagt ggcagcgatt tctcagttct gtcctaagtg 2640
   gccttgctca atcacctgct atcttttagt ggagctttga aattatgttt cagacaactt 2700
   cgattcagtt ctagaatgtt tgactcagca aattcacagg ctcatctttc taacttgatg 2760
   gtgaatatgg aaattcagct aaatggatgt taataaaatt caaacgtttt aaggacagat 2820
   gaaaatgaca gaattttaag gtaaaatata tgaaggaata taagataaag gatttttcta 2880
   ccttcagcaa aaacataccc actaattagt aaaattaata ggcaaaaaaa agttgcatgc 2940
   tcttatactg taatgattat cattttaaaa ctagcttttt gccttcgagc tatcggggta 3000
   aagacctaca ggaaaactac tgtcgaaatc ctcgagggga agaaggggga ccctggtgtt 3060
   tcacaagcaa tccagaggta cgctacgaag tctgtgacat tcctcagtgt tcagaagttg 3120
   aatgcatgac ctgcaatggg gagagttatc gaggtctcat ggatcataca gaatcaggca 3180
   agatttgtca gcgctgggat catcagacac cacaccggca caaattcttg cctgaaagat 3240
   atcccgacaa gggctttgat gataattatt gccgcaatcc cgatggccag ccgaggccat 3300
   ggtgctatac tcttgaccct cacacccgct gggagtactg tgcaattaaa acatgcgctg 3360
   acaatactat gaatgacact gatgttcctt tggaaacaac tgaatgcatc caaggtcaag 3420
   gagaaggcta caggggcact gtcaatacca tttggaatgg aattccatgt cagcgttggg 3480
   attctcagta tcctcacgag catgacatga ctcctgaaaa tttcaagtgc aaggacctac 3540
   gagaaaatta ctgccgaaat ccagatgggt ctgaatcacc ctggtgtttt accactgatc 3600
   caaacatccg agttggctac tgctcccaaa ttccaaactg tgatatgtca catggacaag 3660
   attgttatcg tgggaatggc aaaaattata tgggcaactt atcccaaaca agatctggac 3720
   taacatgttc aatgtgggac aagaacatgg aagacttaca tcgtcatatc ttctgggaac 3780
   cagatgcaag taagctgaat gagaattact gccgaaatcc agatgatgat gctcatggac 3840
   cctggtgcta cacgggaaat ccactcattc cttgggatta ttgccctatt tctcgttgtg 3900
   aaggtgatac cacacctaca atagtcaatt tagaccatcc cgtaatatct tgtgccaaaa 3960
   cgaaacaatt gcgagttgta aatgggattc caacacgaac aaacatagga tggatggtta 4020
   gtttgagata cagaaataaa catatctgcg gaggatcatt gataaaggag agttgggttc 4080
   ttactgcacg acagtgtttc ccttctcgag acttgaaaga ttatgaagct tggcttggaa 4140
   ttcatgatgt ccacggaaga ggagatgaga aatgcaaaca ggttctcaat gtttcccagc 4200
   tggtatatgg ccctgaagga tcagatctgg ttttaatgaa gcttgccagg cctgctgtcc 4260
   tggatgattt tgttagtacg attgatttac ctaattatgg atgcacaatt cctgaaaaga 4320
   ccagttgcag tgtttatggc tggggctaca ctggattgat caactatgat ggcctattac 4380
   gagtggcaca tctctatata atgggaaatg agaaatgcag ccagcatcat cgagggaagg 4440
   tgactctgaa tgagtctgaa atatgtgctg gggctgaaaa gattggatca ggaccatgtg 4500
   agggggatta tggtggccca cttgtttgtg agcaacataa aatgagaatg gttcttggtg 4560
   tcattgttcc tggtcgtgga tgtgccattc caaatcgtcc tggtattttt gtccgagtag 4620
   catattatgc aaaatggata cacaaaatta ttttaacata taaggtacca cagtcatag 4679
<210> 9
   <211> 3679
   <212> DNA
   <213> Artificial Sequence
<220>
   <223> HGF-X7 gene
<400> 9
   atgtgggtga ccaaactcct gccagccctg ctgctgcagc atgtcctcct gcatctcctc 60
   ctgctcccca tcgccatccc ctatgcagag ggacaaagga aaagaagaaa tacaattcat 120
   gaattcaaaa aatcagcaaa gactacccta atcaaaatag atccagcact gaagataaaa 180
   accaaaaaag tgaatactgc agaccaatgt gctaatagat gtactaggaa taaaggactt 240
   ccattcactt gcaaggcttt tgtttttgat aaagcaagaa aacaatgcct ctggttcccc 300
   ttcaatagca tgtcaagtgg agtgaaaaaa gaatttggcc atgaatttga cctctatgaa 360
   aacaaagact acattagaaa ctgcatcatc ggtaaaggac gcagctacaa gggaacagta 420
   tctatcacta agagtggcat caaatgtcag ccctggagtt ccatgatacc acacgaacac 480
   aggtaagaac agtatgaaga aaagagatga agcctctgtc ttttttacat gttaacagtc 540
   tcatattagt ccttcagaat aattctacaa tcctaaaata acttagccaa cttgctgaat 600
   tgtattacgg caaggtttat atgaattcat gactgatatt tagcaaatga ttaattaata 660
   tgttaataaa atgtagccaa aacaatatct taccttaatg cctcaatttg tagatctcgg 720
   tatttgtgga tcctgggtag gaaacacatt tgaatggtat ttactaagat actaaaatcc 780
   ttggacttca ctctaatttt agtgccattt agaactcaag gtctcagtaa aagtagaaat 840
   aaagcctgtt aacaaaacac aagctgaata ttaaaaatgt aactggattt tcaaagaaat 900
   gtttactggt attacctgta gatgtatatt ctttattatg atcttttgtg taaagtctgg 960
   cagacaaatg caatatctaa ttgttgagtc caatatcaca agcagtacaa aagtataaaa 1020
   aagacttggc cttttctaat gtgttaaaat actttatgct ggtaataaca ctaagagtag 1080
   ggcactagaa attttaagtg aagataatgt gttgcagtta ctgcactcaa tggcttacta 1140
   ttataaacca aaactgggat cactaagctc cagtcagtca aaatgatcaa aattattgaa 1200
   gagaataagc aattctgttc tttattagga cacagtagat acagactaca aagtggagtg 1260
   tgcttaataa gaggtagcat ttgttaagtg tcaattactc tattatccct tggagcttct 1320
   caaaataacc atataaggtg taagatgtta aaggttatgg ttacactcag tgcacaggta 1380
   agctaatagg ctgagagaag ctaaattact tactggggtc tcacagtaag aaagtgagct 1440
   gaagtttcag cccagattta actggattct gggctcttta ttcatgttac ttcatgaatc 1500
   tgtttctcaa ttgtgcagaa aaaagggggc tatttataag aaaagcaata aacaaacaag 1560
   taatgatctc aaataagtaa tgcaagaaat agtgagattt caaaatcagt ggcagcgatt 1620
   tctcagttct gtcctaagtg gccttgctca atcacctgct atcttttagt ggagctttga 1680
   aattatgttt cagacaactt cgattcagtt ctagaatgtt tgactcagca aattcacagg 1740
   ctcatctttc taacttgatg gtgaatatgg aaattcagct aaatggatgt taataaaatt 1800
   caaacgtttt aaggacagat gaaaatgaca gaattttaag gtaaaatata tgaaggaata 1860
   taagataaag gatttttcta ccttcagcaa aaacataccc actaattagt aaaattaata 1920
   ggcaaaaaaa agttgcatgc tcttatactg taatgattat cattttaaaa ctagcttttt 1980
   gccttcgagc tatcggggta aagacctaca ggaaaactac tgtcgaaatc ctcgagggga 2040
   agaaggggga ccctggtgtt tcacaagcaa tccagaggta cgctacgaag tctgtgacat 2100
   tcctcagtgt tcagaagttg aatgcatgac ctgcaatggg gagagttatc gaggtctcat 2160
   ggatcataca gaatcaggca agatttgtca gcgctgggat catcagacac cacaccggca 2220
   caaattcttg cctgaaagat atcccgacaa gggctttgat gataattatt gccgcaatcc 2280
   cgatggccag ccgaggccat ggtgctatac tcttgaccct cacacccgct gggagtactg 2340
   tgcaattaaa acatgcgctg acaatactat gaatgacact gatgttcctt tggaaacaac 2400
   tgaatgcatc caaggtcaag gagaaggcta caggggcact gtcaatacca tttggaatgg 2460
   aattccatgt cagcgttggg attctcagta tcctcacgag catgacatga ctcctgaaaa 2520
   tttcaagtgc aaggacctac gagaaaatta ctgccgaaat ccagatgggt ctgaatcacc 2580
   ctggtgtttt accactgatc caaacatccg agttggctac tgctcccaaa ttccaaactg 2640
   tgatatgtca catggacaag attgttatcg tgggaatggc aaaaattata tgggcaactt 2700
   atcccaaaca agatctggac taacatgttc aatgtgggac aagaacatgg aagacttaca 2760
   tcgtcatatc ttctgggaac cagatgcaag taagctgaat gagaattact gccgaaatcc 2820
   agatgatgat gctcatggac cctggtgcta cacgggaaat ccactcattc cttgggatta 2880
   ttgccctatt tctcgttgtg aaggtgatac cacacctaca atagtcaatt tagaccatcc 2940
   cgtaatatct tgtgccaaaa cgaaacaatt gcgagttgta aatgggattc caacacgaac 3000
   aaacatagga tggatggtta gtttgagata cagaaataaa catatctgcg gaggatcatt 3060
   gataaaggag agttgggttc ttactgcacg acagtgtttc ccttctcgag acttgaaaga 3120
   ttatgaagct tggcttggaa ttcatgatgt ccacggaaga ggagatgaga aatgcaaaca 3180
   ggttctcaat gtttcccagc tggtatatgg ccctgaagga tcagatctgg ttttaatgaa 3240
   gcttgccagg cctgctgtcc tggatgattt tgttagtacg attgatttac ctaattatgg 3300
   atgcacaatt cctgaaaaga ccagttgcag tgtttatggc tggggctaca ctggattgat 3360
   caactatgat ggcctattac gagtggcaca tctctatata atgggaaatg agaaatgcag 3420
   ccagcatcat cgagggaagg tgactctgaa tgagtctgaa atatgtgctg gggctgaaaa 3480
   gattggatca ggaccatgtg agggggatta tggtggccca cttgtttgtg agcaacataa 3540
   aatgagaatg gttcttggtg tcattgttcc tggtcgtgga tgtgccattc caaatcgtcc 3600
   tggtattttt gtccgagtag catattatgc aaaatggata cacaaaatta ttttaacata 3660
   taaggtacca cagtcatag 3679
<210> 10
   <211> 2729
   <212> DNA
   <213> Artificial Sequence
<220>
   <223> HGF-X8 gene
<400> 10
   atgtgggtga ccaaactcct gccagccctg ctgctgcagc atgtcctcct gcatctcctc 60
   ctgctcccca tcgccatccc ctatgcagag ggacaaagga aaagaagaaa tacaattcat 120
   gaattcaaaa aatcagcaaa gactacccta atcaaaatag atccagcact gaagataaaa 180
   accaaaaaag tgaatactgc agaccaatgt gctaatagat gtactaggaa taaaggactt 240
   ccattcactt gcaaggcttt tgtttttgat aaagcaagaa aacaatgcct ctggttcccc 300
   ttcaatagca tgtcaagtgg agtgaaaaaa gaatttggcc atgaatttga cctctatgaa 360
   aacaaagact acattagaaa ctgcatcatc ggtaaaggac gcagctacaa gggaacagta 420
   tctatcacta agagtggcat caaatgtcag ccctggagtt ccatgatacc acacgaacac 480
   aggtaagaac agtatgaaga aaagagatga agcctctgtc ttttttacat gttaacagtc 540
   tcatattagt ccttcagaat aattctacaa tcctaaaata acttagccaa cttgctgaat 600
   tgtattacgg caaggtttat atgaattcat gactgatatt tagcaaatga ttaattaata 660
   tgttaataaa atgtagccaa aacaatatct taccttaatg cctcaatttg tagatctcgg 720
   tatttgtgga tccttatgtt tcagacaact tcgattcagt tctagaatgt ttgactcagc 780
   aaattcacag gctcatcttt ctaacttgat ggtgaatatg gaaattcagc taaatggatg 840
   ttaataaaat tcaaacgttt taaggacaga tgaaaatgac agaattttaa ggtaaaatat 900
   atgaaggaat ataagataaa ggatttttct accttcagca aaaacatacc cactaattag 960
   taaaattaat aggcaaaaaa aagttgcatg ctcttatact gtaatgatta tcattttaaa 1020
   actagctttt tgccttcgag ctatcggggt aaagacctac aggaaaacta ctgtcgaaat 1080
   cctcgagggg aagaaggggg accctggtgt ttcacaagca atccagaggt acgctacgaa 1140
   gtctgtgaca ttcctcagtg ttcagaagtt gaatgcatga cctgcaatgg ggagagttat 1200
   cgaggtctca tggatcatac agaatcaggc aagatttgtc agcgctggga tcatcagaca 1260
   ccacaccggc acaaattctt gcctgaaaga tatcccgaca agggctttga tgataattat 1320
   tgccgcaatc ccgatggcca gccgaggcca tggtgctata ctcttgaccc tcacacccgc 1380
   tgggagtact gtgcaattaa aacatgcgct gacaatacta tgaatgacac tgatgttcct 1440
   ttggaaacaa ctgaatgcat ccaaggtcaa ggagaaggct acaggggcac tgtcaatacc 1500
   atttggaatg gaattccatg tcagcgttgg gattctcagt atcctcacga gcatgacatg 1560
   actcctgaaa atttcaagtg caaggaccta cgagaaaatt actgccgaaa tccagatggt 1620
   ctgaatcacc ctggtgtttt accactgatc caaacatccg agttggctac tgctcccaaa    1680
   ttccaaactg tgatatgtca catggacaag attgttatcg tgggaatggc aaaaattata    1740
   tgggcaactt atcccaaaca agatctggac taacatgttc aatgtgggac aagaacatgg    1800
   aagacttaca tcgtcatatc ttctgggaac cagatgcaag taagctgaat gagaattact    1860
   gccgaaatcc agatgatgat gctcatggac cctggtgcta cacgggaaat ccactcattc    1920
   cttgggatta ttgccctatt tctcgttgtg aaggtgatac cacacctaca atagtcaatt    1980
   tagaccatcc cgtaatatct tgtgccaaaa cgaaacaatt gcgagttgta aatgggattc    2040
   caacacgaac aaacatagga tggatggtta gtttgagata cagaaataaa catatctgcg    2100
   gaggatcatt gataaaggag agttgggttc ttactgcacg acagtgtttc ccttctcgag    2160
   acttgaaaga ttatgaagct tggcttggaa ttcatgatgt ccacggaaga ggagatgaga    2220
   aatgcaaaca ggttctcaat gtttcccagc tggtatatgg ccctgaagga tcagatctgg    2280
   ttttaatgaa gcttgccagg cctgctgtcc tggatgattt tgttagtacg attgatttac    2340
   ctaattatgg atgcacaatt cctgaaaaga ccagttgcag tgtttatggc tggggctaca    2400
   ctggattgat caactatgat ggcctattac gagtggcaca tctctatata atgggaaatg    2460
   agaaatgcag ccagcatcat cgagggaagg tgactctgaa tgagtctgaa atatgtgctg    2520
   gggctgaaaa gattggatca ggaccatgtg agggggatta tggtggccca cttgtttgtg    2580
   agcaacataa aatgagaatg gttcttggtg tcattgttcc tggtcgtgga tgtgccattc    2640
   caaatcgtcc tggtattttt gtccgagtag catattatgc aaaatggata cacaaaatta    2700
   ttttaacata taaggtacca cagtcatag                                      2729
<210> 11
   <211> 285
   <212> PRT
   <213> Homo sapiens
<400> 11
   Met Trp Val Thr Lys Leu Leu Pro Ala Leu Leu Leu Gln His Val Leu
   1               5                   10                  15
   Leu His Leu Leu Leu Leu Pro Ile Ala Ile Pro Tyr Ala Glu Gly Gln
               20                  25                  30
   Arg Lys Arg Arg Asn Thr Ile His Glu Phe Lys Lys Ser Ala Lys Thr
           35                  40                  45
   Thr Leu Ile Lys Ile Asp Pro Ala Leu Lys Ile Lys Thr Lys Lys Val
       50                  55                  60
   Asn Thr Ala Asp Gln Cys Ala Asn Arg Cys Thr Arg Asn Lys Gly Leu
   65                  70                  75                  80
   Pro Phe Thr Cys Lys Ala Phe Val Phe Asp Lys Ala Arg Lys Gln Cys
                   85                  90                  95
   Leu Trp Phe Pro Phe Asn Ser Met Ser Ser Gly Val Lys Lys Glu Phe
   100 105 110
   Gly His Glu Phe Asp Leu Tyr Glu Asn Lys Asp Tyr Ile Arg Asn Cys
   115 120 125
   Ile Ile Gly Lys Gly Arg Ser Tyr Lys Gly Thr Val Ser Ile Thr Lys
   130 135 140
   Ser Gly Ile Lys Cys Gln Pro Trp Ser Ser Met Ile Pro His Glu His
   145 150 155 160
   Ser Tyr Arg Gly Lys Asp Leu Gln Glu Asn Tyr Cys Arg Asn Pro Arg
   165 170 175
   Gly Glu Glu Gly Gly Pro Trp Cys Phe Thr Ser Asn Pro Glu Val Arg
   180 185 190
   Tyr Glu Val Cys Asp Ile Pro Gln Cys Ser Glu Val Glu Cys Met Thr
   195 200 205
   Cys Asn Gly Glu Ser Tyr Arg Gly Leu Met Asp His Thr Glu Ser Gly
   210 215 220
   Lys Ile Cys Gln Arg Trp Asp His Gln Thr Pro His Arg His Lys Phe
   225 230 235 240
   Leu Pro Glu Arg Tyr Pro Asp Lys Gly Phe Asp Asp Asn Tyr Cys Arg
   245 250 255
   Asn Pro Asp Gly Gln Pro Arg Pro Trp Cys Tyr Thr Leu Asp Pro His
   260 265 270
   Thr Arg Trp Glu Tyr Cys Ala Ile Lys Thr Cys Glu Thr
   275 280 285
<210> 12
   <211> 296
   <212> PRT
   <213> Homo sapiens
<400> 12
   Met Trp Val Thr Lys Leu Leu Pro Ala Leu Leu Leu Gln His Val Leu
   1 5 10 15
   Leu His Leu Leu Leu Leu Pro Ile Ala Ile Pro Tyr Ala Glu Gly Gln
   20 25 30
   Arg Lys Arg Arg Asn Thr Ile His Glu Phe Lys Lys Ser Ala Lys Thr
   35 40 45
   Thr Leu Ile Lys Ile Asp Pro Ala Leu Lys Ile Lys Thr Lys Lys Val
       50                  55                  60
   Asn Thr Ala Asp Gln Cys Ala Asn Arg Cys Thr Arg Asn Lys Gly Leu
   65                  70                  75                  80
   Pro Phe Thr Cys Lys Ala Phe Val Phe Asp Lys Ala Arg Lys Gln Cys
                   85                  90                  95
   Leu Trp Phe Pro Phe Asn Ser Met Ser Ser Gly Val Lys Lys Glu Phe
               100                 105                 110
   Gly His Glu Phe Asp Leu Tyr Glu Asn Lys Asp Tyr Ile Arg Asn Cys
           115                 120                 125
   Ile Ile Gly Lys Gly Arg Ser Tyr Lys Gly Thr Val Ser Ile Thr Lys
       130                 135                 140
   Ser Gly Ile Lys Cys Gln Pro Trp Ser Ser Met Ile Pro His Glu His
   145                 150                 155                 160
   Ser Phe Leu Pro Ser Ser Tyr Arg Gly Lys Asp Leu Gln Glu Asn Tyr
                   165                 170                 175
   Cys Arg Asn Pro Arg Gly Glu Glu Gly Gly Pro Trp Cys Phe Thr Ser
               180                 185                 190
   Asn Pro Glu Val Arg Tyr Glu Val Cys Asp Ile Pro Gln Cys Ser Glu
           195                 200                 205
   Val Glu Cys Met Thr Cys Asn Gly Glu Ser Tyr Arg Gly Leu Met Asp
       210                 215                 220
   His Thr Glu Ser Gly Lys Ile Cys Gln Arg Trp Asp His Gln Thr Pro
   225                 230                 235                 240
   His Arg His Lys Phe Leu Pro Glu Arg Tyr Pro Asp Lys Gly Phe Asp
                   245                 250                 255
   Asp Asn Tyr Cys Arg Asn Pro Asp Gly Gln Pro Arg Pro Trp Cys Tyr
               260                 265                 270
   Thr Leu Asp Pro His Thr Arg Trp Glu Tyr Cys Ala Ile Lys Asn Met
           275                 280                 285
   Arg Asp Ile Thr Trp Ala Leu Asn
       290                 295